(19) Europäisches Patentamt
European Patent Office
Office européen des brevets

(11) EP 3 639 802 B1

(12) # EUROPEAN PATENT SPECIFICATION

(45) Date of publication and mention of the grant of the patent:
**06.12.2023 Bulletin 2023/49**

(21) Application number: **18817925.3**

(22) Date of filing: **14.06.2018**

(51) International Patent Classification (IPC):
***A61F 13/511*** (2006.01) ***A61F 13/15*** (2006.01)
***A61F 13/513*** (2006.01) ***D04H 1/4291*** (2012.01)
***D04H 3/16*** (2006.01) ***D04H 5/06*** (2006.01)
***D06M 15/643*** (2006.01) ***D06M 15/647*** (2006.01)

(52) Cooperative Patent Classification (CPC):
**A61F 13/51104; A61F 13/51113; A61F 13/5116; A61F 13/51305; D04H 1/4291; D04H 3/16; D04H 5/06; D06M 15/643**

(86) International application number:
**PCT/JP2018/022798**

(87) International publication number:
**WO 2018/230666 (20.12.2018 Gazette 2018/51)**

(54) **ABSORBENT ARTICLE**

ABSORBIERENDER GEGENSTAND

ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2017 JP 2017119148**

(43) Date of publication of application:
**22.04.2020 Bulletin 2020/17**

(73) Proprietor: **Kao Corporation**
**Chuo-ku**
**Tokyo 103-8210 (JP)**

(72) Inventors:
- **SUZUKI, Hana**
**Haga-gun**
**Tochigi 321-3497 (JP)**
- **KABAYA, Yoshiaki**
**Haga-gun**
**Tochigi 321-3497 (JP)**
- **SANGAWA, Yuta**
**Haga-gun**
**Tochigi 321-3497 (JP)**

(74) Representative: **Vossius & Partner**
**Patentanwälte Rechtsanwälte mbB**
**Siebertstrasse 3**
**81675 München (DE)**

(56) References cited:

| | |
|---|---|
| WO-A1-2014/171388 | WO-A1-2016/051833 |
| WO-A1-2016/098796 | WO-A1-2017/033867 |
| JP-A- 2004 000 465 | JP-A- 2014 502 928 |
| JP-A- 2015 132 038 | JP-A- 2016 060 994 |
| JP-A- 2016 060 995 | JP-A- 2016 117 981 |
| JP-A- 2017 039 111 | US-A- 5 258 129 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 639 802 B1

## Description

FIELD OF THE INVENTION

The present invention relates to an absorbent article such as a sanitary towel.

BACKGROUND OF THE INVENTION

In recent years, proposals have been made for improving wear feel, such as dryness, of a nonwoven fabric used in a topsheet or the like in contact with the skin in an absorbent article or the like. For example, Patent Literature 1 describes that a liquid film formed in narrow interfibre spaces of the nonwoven fabric can be cleaved by a liquid film cleavage agent to reduce residual liquid in the nonwoven fabric. Thus, dryness of the nonwoven fabric can be remarkably improved.

CITATION LIST

PATENT LITERATURES

Patent Literature 1: JP-A-2016-117981 ("JP-A" means unexamined published Japanese patent application)

SUMMARY OF THE INVENTION

The present invention according to claim provides an absorbent article, containing a topsheet, a backsheet, and an absorbent body interposed between the topsheet and the backsheet, in which the topsheet has an arrangement portion of one or more of compounds selected from the following compound C1 and the following compound C2 in a thickness intermediate position or on an absorbent body side, wherein

compound C1 having a spreading coefficient of 15 mN/m or more to a liquid having surface tension of 50 mN/m, and a water solubility of 0 g or more and 0.025 g or less, and compound C2 is a compound having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m.

Further, the present invention provides a method for producing a nonwoven fabrich according to claim 9, containing a step of providing one or more of compounds selected from the following compound C1 and the following compound C2 on one surface of a first fibre layer having thermally fusible fibres, a step of laminating a second fibre layer having thermally fusible fibres on the surface of the first fibre layer, on which one or more of compounds selected from the compound C1 and the compound C2 is provided, and a step of bonding the first fibre layer and the second fibre layer by thermal fusion by heat treatment at a temperature higher than or equal to a melting point of the thermally fusible fibres, wherein compound C1 having a spreading coefficient of 15 mN/m or more to a liquid having surface tension of 50 mN/m, and a water solubility of 0 g or more and 0.025 g or less, and compound C2 is a compound having a spreading coefficient of more than 0 mN/m to a liquid having surface tension of 50 mN/m, a water solubility of 0 g or more and 0.025 g or less, and an interfacial tension of 20 mN/m or less to the liquid having surface tension of 50 mN/m.

Other and further objects, features and advantages of the invention will appear more fully from the following description, appropriately referring to the accompanying drawings.

BRIEF DESCRIPTION OF DRAWINGS

{FIG. 1}
FIG. 1 is a partial cutaway perspective view showing a preferable embodiment of an absorbent article according to the present invention.
{FIG. 2}
FIG. 2 is a partial cross-sectional perspective view schematically showing a partially engaging state of a preferable aspect of a topsheet in an absorbent article in the embodiment.
{FIG. 3}
FIG. 3 is an explanatory drawing showing an aspect in which, for the topsheet shown in FIG. 2, an arrangement portion of a liquid film cleavage agent is provided on a thickness intermediate position.
{FIG. 4}
FIG. 4 is an explanatory drawing showing an aspect in which, for the topsheet shown in FIG. 2, an arrangement

portion of a liquid film cleavage agent is provided on a surface facing an absorbent body side of the topsheet.

{FIG. 5}

FIG. 5 is a drawing substituted for photograph showing the results of cleavage action on a liquid film of quasi-menstrual blood by macro spreadability of a liquid film cleavage agent in a plane direction of a nonwoven fabric, in which FIG. 5(A) shows a state of the nonwoven fabric in which no liquid film cleavage agent is arranged, FIG. 5(B) shows a state of a nonwoven fabric in which an arrangement portion of the liquid film cleavage agent is formed in a dot shape having a diameter of 0.8 mm at 0.4 mass% of an arrangement proportion (arrangement portion OPU: Oil Per Unit) of a applying liquid to fibre mass of the arrangement portion, and FIG. 5(C) shows a state of a nonwoven fabric in which the arrangement proportion (arrangement portion OPU) of the applying liquid to the fibre mass of the arrangement portion shown in FIG. 5(B) is increased to 8.0 mass%.

{FIG. 6}

FIG. 6(A) is an explanatory drawing showing a content of a test for confirming cleavage action on a liquid film of quasi-menstrual blood by macro spreadability of a liquid film cleavage agent in a thickness direction of a topsheet, and FIG. 6(B) is an explanatory drawing showing a state in which an arrangement portion of the liquid film cleavage agent is formed on a surface facing an absorbent body side of the topsheet in FIG. 6(A).

{FIG. 7}

FIG. 7 is a drawing substituted for photograph showing the results of the test shown in FIG. 6, in which FIG. 7(A) shows a state of a top surface 1A of a topsheet 1 immediately after quasi-menstrual blood was injected, FIG. 7(B) shows a state after 20 seconds, and FIG. 7(C) shows a state after 30 seconds.

{FIG. 8}

FIG. 8 is a partial cross-sectional perspective view schematically showing a partially engaging state of another preferable aspect of a topsheet in an absorbent article in the embodiment.

## DESCRIPTION OF EMBODIMENTS

The present invention relates to an absorbent article capable of simultaneously realising reduction of residual liquid and reduction of sticky feeling on skin by using the aforesaid liquid film cleavage agent.

The present inventors realised reduction of residual liquid in a nonwoven fabric serving as a topsheet by using the liquid film cleavage agent described in Patent Literature 1.

Meanwhile, in the topsheet of the absorbent article, if an amount of the liquid film cleavage agent in contact with wearer's skin is excessively large, a sticky feeling on skin occurs easily in several cases, and there was room for improvement from a viewpoint of good texture.

The absorbent article of the present invention can simultaneously realise reduction of residual liquid and reduction of sticky feeling on skin.

The absorbent article according to the present invention will be described below by showing a sanitary towel 10 as a preferable embodiment thereof with reference to drawings.

In the present invention, unless otherwise specified, a side in contact with a human body (wearer's skin side) is referred to as a skin side, a skin-contact surface side or a surface side, and a side opposite thereto is referred to as a non-skin side, a skin non-contact surface side or a back surface side. When the absorbent article is worn, the position locating at the front side of the human body is referred to as the front, its edge is referred to as the front edge, and the position locating at the rear side is referred to as the rear, and its edge is referred to as the rear edge. Moreover, the normal direction of the top surface or back surface of the absorbent article is referred to as thickness direction, and its amount is referred to as the thickness.

As shown in FIG. 1, the sanitary towel 10 of the embodiment has a topsheet 1 arranged on the skin-contact surface side, a backsheet 2 arranged on the skin non-contact surface side, and a liquid-retainable absorbent body 3 interposed between the topsheet 1 and backsheet 2. Further, a leak-proof groove 5 compressed from the topsheet 1 to the absorbent body 3 is arranged on the skin-contact surface side of the sanitary towel 10, and the leak-proof groove 5 forms an annular shape in a plane view. In addition, the leak-proof groove 5 can be arbitrarily arranged, and a shape and the number thereof can be appropriately set according to a purpose of use or the like. Moreover, the sanitary towel 10 may contain other components. For example, the sanitary towel 10 may contain an intermediate sheet having liquid diffusibility between the topsheet 1 and the absorbent body 3, or may have a pair of wing portions for fixing the sanitary towel 10 to clothes on both sides in a longitudinal direction of the sanitary towel 10.

The sanitary towel 10 has a longitudinally long shape having a longitudinal direction (Y direction) and a lateral direction (X direction) perpendicular to the longitudinal direction. The sanitary towel 10 is worn by directing a portion on a topsheet 1 side toward a wearer's skin-contact surface side, disposing a portion in the longitudinal direction from a lower abdomen side to a buttocks side, and disposing a portion in the crosswise direction toward a direction along a line connecting right and left legs. In corresponding to the above-described wearing state, the sanitary towel 10 has, in the longitudinal direction (Y direction), a crotch portion C which covers a wearer's excretory portion, a front portion F forward

of the crotch portion C, corresponding to the lower abdomen side, and a rear portion R backward thereof, corresponding to the buttocks side. In the crotch portion C of the sanitary towel 10, a liquid-receiving portion Q1 that directly receives an excreted liquid exists in a centre in the crosswise direction of the crotch portion C. The liquid-receiving portion Q1, namely, the term "a centre in the crosswise direction" of the crotch portion C means that when the absorbent article is a sanitary towel, a region in 2.5 cm or less in a right-left crosswise direction from a line dividing a length in the crosswise direction into two equal parts in the crosswise direction of the sanitary towel, and a region arranged in a whole area in a longitudinal direction of the crotch portion C in a longitudinal direction of the sanitary towel. Here, when the absorbent article is a sanitary towel for nighttime use, the crotch portion C is the second region from the front of regions obtained by dividing the sanitary towel into four parts in the longitudinal direction, and when the absorbent article is a sanitary towel for daytime use, the crotch portion C is a middle region of regions obtained by dividing the sanitary towel into three parts in the longitudinal direction. Moreover, when the absorbent article is a nappy, the above-described liquid-receiving portion Q1 is a region in 3.5 cm or less in the right-left crosswise direction from a line dividing a length in the crosswise direction into two equal parts in the crosswise direction of the nappy, and a region in 8.0 cm or less in a front portion F direction from a line dividing a length in a longitudinal direction of the nappy into two equal parts in the longitudinal direction. When the absorbent article is a urine pad or an incontinence pad, the liquid-receiving portion Q1 is the same as in the sanitary towel for daytime use. In the present embodiment, the liquid-receiving portion Q1 is a region surrounded by the leak-proof groove 5 in the crotch portion.

Division positions of the crotch portion C, the front portion F and the rear portion R may be appropriately set according to the length of the absorbent article to be set for the purpose of use or the like. As the sanitary towel 10 of the embodiment, an example of a shape set as daytime use or the like is shown, and the length in the longitudinal direction is divided into about three equal parts, and the front portion F, the crotch portion C and the rear portion R are set. Specific examples of a shape different therefrom include a shape (not shown) in which a sanitary towel 10 has a wide rear flap covering buttocks as night use or the like, and a rear portion R is formed to be longer than a front portion F and a crotch portion C. In this case, the crotch portion C exists to the front of the sanitary towel, and the second region from the front portion F of the regions obtained by dividing the sanitary towel into four equal parts in the longitudinal direction may correspond to the crotch portion C, for example. Even if the sanitary towel 10 has any shape, the crotch portion C may be generally set as a site in a position at a predetermined distance from the front portion F.

The topsheet 1 is formed of a fibre sheet existing on a skin side of the absorbent body 3, and to be in contact with the wearer's skin. The fibre sheet has integrity of a sheet by bonding by thermal fusion of the fibres with each other (for example, a nonwoven fabric). For bonding by the thermal fusion, thermally fusible fibres are used as constituent fibres, for example. Specifically, the thermally fusible fibres are melted by heat treatment, and the fibres are fused with each other by melted components. In this case, the above-described melting may be induced in both the fibres to be fused, or in the fibres only on one side. Examples of the above-described heat treatment include hot-air treatment (airthrough treatment) and embossing compression treatment.

When the fibre sheet is a laminated sheet formed of a plurality of fibre layers, not only the fibres are bonded with each other within each fibre layer, but also the fibre layers are bonded with each other by the above-described thermal fusion or an adhesive. That is, the fibres in both the fibre layers are bonded with each other by the thermal fusion or the adhesive on a boundary surface between the fibre layers. In this case, as long as the integrity as the fibre sheet is retained, the fibre layers may be wholly or partly bonded with each other. In bonding between the fibre layers, from a viewpoint of allowing the excreted liquid to quickly pass from the topsheet to the absorbent body, and from a viewpoint of maintaining soft texture of the topsheet, the fibre layers are preferably bonded only by the thermal fusion without bonding by the adhesive.

When the topsheet 1 is formed of the laminated sheet in which the fibres in both the fibre layers are bonded with each other only by the thermal fusion, specific examples include (N1) to (N4) as described below.

(N1) A laminated nonwoven fabric in which two fibre webs (fibre layers in a state in which the fibres are not fused) are stacked, whereby the laminated nonwoven fabric is formed by hot-air treatment at a temperature at which the fibres may be melted.

(N2) A laminated nonwoven fabric in which a fibre web is stacked on a nonwoven fabric (fibre layer in a state in which fusion and bonding of the fibres are formed), and the nonwoven fabric and the fibre web are fused and bonded by embossing compression treatment, and the fibres between the fibre layers are further bonded by hot-air treatment at a temperature higher than a melting point of the fibres.

(N3) A laminated nonwoven fabric in which a fibre web is stacked on a nonwoven fabric, the nonwoven fabric and the fibre web are fused and bonded by embossing compression treatment, and subjected to hot-air treatment at a temperature lower than a melting point of the fibres.

(N4) A laminated nonwoven fabric in which other long fibres are spun and stacked on a long fibre layer formed by spinning a melted resin, and the fibres are bonded by embossing compression treatment.

In the (N1) and the (N2) described above, the fibre layers are wholly bonded with each other by the hot-air treatment. In the (N3) and the (N4) described above, the fibre layers are mainly bonded with each other in a part subjected to the embossing compression treatment.

The topsheet 1 has hydrophilicity as a whole, and receives excretion of a bodily fluid to have liquid permeability with which the bodily fluid is quickly sucked into the absorbent body 3 inside the absorbent article. As a shape of the topsheet 1, various forms ordinarily used for the absorbent article can be adopted without particular restriction. Specific examples thereof include nonwoven fabrics described in the paragraphs {0100} to {0110} and FIGs. 3 to 11 in WO 2016/098796 and the like.

In the present embodiment, as shown in FIG. 2, a surface on a top surface (surface facing the wearer's skin side, and a skin-contact surface) 1A side and a surface on a back surface (surface facing an absorbent body side, and a non-skin contact surface) 1B side of the topsheet 1 are formed in a concavo-convex shape. More specifically, the topsheet 1 has a structure in which a skin side fibre layer (hereinafter, also referred to as a skin side layer) 17 and a non-skin side fibre layer (hereinafter, also referred to as a non-skin side layer) 18 are laminated. Both the layers are partially bonded by a concave bonding portion 19. The concave bonding portion 19 is formed by the thermal fusion of the fibres with each other by the embossing compression treatment.

In the topsheet 1 of the present embodiment, the top surface 1A has the concavo-concave shape, whereby a contact area between the skin and the topsheet 1 is reduced, and the sticky feeling on skin or damp feeling is suppressed into the topsheet 1 excellent in dry feeling. Moreover, when a convex portion 17P in the concavo-convex shape has a dome shape filled with the fibres, cushioning properties in contact with the skin are improved, and such a case is preferable.

The topsheet 1 as shown in FIG. 2 preferably has the fibre structure described in the paragraphs [0010] to [0055] of JP-A-2015-110846. Specifically, the topsheet 1 has a structure in which the non-skin side layer 18 contains helically crimped fibres, and fibre density of the non-skin side layer 18 is adjusted to be higher than the fibre density of the skin side layer 17. According to this structure, the topsheet 1 is provided with quick liquid permeability by a capillary pressure difference, and bulkiness and high cushioning properties. Thus, action of the liquid film cleavage agent for reduction of residual liquid described later can be further enhanced.

The topsheet 1 has an arrangement portion 11 of the liquid film cleavage agent. The arrangement portion 11 is formed into an arrangement shown in FIG. 3 or an arrangement shown in FIG. 4. The topsheet 1 may wholly or partly have the arrangement portion 11 in a plane direction. When the topsheet 1 partly has the arrangement portion 11, from a viewpoint of dryness, the arrangement portion 11 preferably exists at least in the liquid-receiving region Q1. Upon taking into consideration a possibility of deviation of an attachment position of the sanitary towel 10 or an increase in an amount of the excreted liquid depending on physical conditions to induce diffusion of the excreted liquid from the liquid-receiving region Q1 to outside, from a viewpoint of reducing the residual liquid even if the excreted liquid is received in any position, the arrangement portion 11 of the liquid film cleavage agent preferably wholly exists in the plane direction of the topsheet 1. Meanwhile, from a viewpoint of suppressing the sticky feeling, the arrangement portion 11 preferably exists only in the liquid-receiving region Q1. On the occasion, the arrangement portion 11 may be arranged in a pattern wholly in the plane direction or wholly in the liquid-receiving region Q1, or the liquid-receiving region Q1 may be wholly formed into the arrangement portion 11. The above-described expression "arranged in a pattern" means that a plurality of arrangement portions 11 is separated from each other and regularly arranged. In this case, the arrangement portion 11 and the non-arrangement portion 12 described later are preferably alternately arranged in the plane direction of the topsheet 1.

In FIG. 3, the arrangement portion 11 of the liquid film cleavage agent exists in a thickness intermediate position 13 of the topsheet 1. A plurality of arrangement portions 11 exists in the plane direction of the topsheet 1 in the thickness intermediate position 13.

In FIG. 4, the arrangement portion 11 of the liquid film cleavage agent exists on a back surface 1B side (absorbent body side) of the topsheet 1. A plurality of arrangement portions 11 exists in the plane direction of the topsheet 1 on the back surface 1B side.

In addition, a part other than the arrangement portion 11 is the part in which no liquid film cleavage agent is arranged, and this part is shown as the non-arrangement portion 12 of the liquid film cleavage agent.

When the topsheet 1 is divided into substantially three equal parts in the thickness direction, when the arrangement portion 11 exists in a layer on a surface side of the skin side layer, which is a division closest to the skin side, the arrangement portion 11 of the liquid film cleavage agent is judged to exist on a top surface 1A side, when the arrangement portion 11 exists in a layer on the surface side of the non-skin side layer, which is a division farthest from the skin side layer, the arrangement portion 11 of the liquid film cleavage agent is judged to exist on the back surface 1B side (absorbent body side), and when the arrangement portion 11 exists in a layer of a middle division, the arrangement portion 11 of the liquid film cleavage agent is judged to exist in the thickness intermediate position 13.

In FIGs. 3 and 4, in order to understand an arrangement position of the arrangement portion 11, the arrangement portion 11 is patterned, but the arrangement portion 11 cannot be necessarily distinguished by visual observation in practice. Moreover, in FIGs. 3 and 4, the arrangement portions 11 are shown in a circular shape and separated from

each other. However, a specific aspect of an arrangement interval, the number of arrangement portions 11 or the like is not limited thereto, and from a viewpoint of an effect of reduction of residual liquid, the arrangement interval, the number or the like may be appropriately set.

In the case of FIG. 3, the liquid film cleavage agent in the arrangement portion 11 is present on the way of a path in which the bodily fluid is permeated through the topsheet 1. Further, in the case of FIG. 4, the liquid film cleavage agent in the arrangement portion 11 is present in the vicinity of an outlet from which the bodily fluid is transferred from the topsheet 1 to the absorbent body 3 side. In any case, the liquid film cleavage agent is spread (transferred) from a position of the arrangement portion 11 to exhibit the liquid film cleavage action described later on the body fluid. This spread action will be described later.

Here, the term "thickness intermediate position 13 of the topsheet 1" means a part inward from both a surface of the top surface 1A and a surface of the back surface 1B. This thickness intermediate position 13 is preferably a region of a central part of regions obtained by dividing a thickness of the topsheet into three equal parts, and more preferably a region in the vicinity of a centre of a thickness width. When the top surface 1A and the back surface 1B are formed into the concavo-convex shape, the thickness intermediate position 13 exists in a position inward from both the surface of the top surface 1A and the surface of the back surface 1B along the concavo-convex shape.

Moreover, as shown in FIG. 3, when the topsheet 1 is formed by laminating a plurality of fibre layers, the thickness intermediate position 13 can be formed as the boundary surface between the fibre layers. When the arrangement portion 11 exists on the boundary surface between the fibre layers, the arrangement portion 11 may exist on a surface on any side of both surfaces facing an adjacent fibre layer. In the topsheet 1 shown in FIG. 3, the arrangement portion 11 may exist in any surface of a surface 17A of the skin side layer 17, facing the non-skin side layer 18, and a surface 18A of the non-skin side layer 18, facing the skin side layer 17, or may exist in both the surfaces facing the layers. In addition, when the topsheet is formed of a plurality of fibre layers, the topsheet is not limited to two layers as shown in FIG. 3, and may have three or more layers. When the topsheet has three or more layers, the arrangement portion 11 only needs to exist at least on any of the boundary surfaces, and may exist on one boundary surface or on a plurality of boundary surfaces. Moreover, when the topsheet has three or more layers, the arrangement portion 11 may be arranged inside the fibre layer interposed by the fibre layers on the top surface and on the back surface.

The arrangement portion 11 can be formed in the "thickness intermediate position 13 of the topsheet 1" by various methods ordinarily used in a production process of the fibre sheet serving as the topsheet 1.

When the topsheet 1 is formed of a single fibre layer (nonwoven fabric), examples thereof include a method of providing (arranging) a liquid film cleavage agent on the way of fibre-stacking processing for forming a fibre web containing thermally fusible fibres. In this case, after providing the liquid film cleavage agent, the remaining necessary fibre-stacking processing is performed, whereby the fibres are fused with each other by heat treatment at a temperature higher than or equal to a melting point of the thermally fusible fibres to form the fibre web into the nonwoven fabric.

Moreover, when the topsheet 1 is formed of a plurality of fibre layers (nonwoven fabrics), for example, when the topsheet 1 is formed of two layers, the nonwoven fabric can be produced by performing processing steps (P1) to (P3) described below.

(P1) A step of providing (arranging) a liquid film cleavage agent on one surface of a first fibre layer having thermally fusible fibres.

(P2) A step of laminating a second fibre layer having thermally fusible fibres on the surface of the first fibre layer, on which the liquid film cleavage agent is provided (arranged).

(P3) A step of bonding the first fibre layer and the second fibre layer by thermal fusion by heat treatment at a temperature higher than or equal to a melting point of the above-described thermally fusible fibres.

In addition, the "first fibre layer" and the "second fibre layer" described above mean various fibre aggregates of the nonwoven fabric, the fibre web, the long fibre layer or the like. The "first fibre layer" and the "second fibre layer" may be laminated with each other by using the layers having forms identical to or different from each other. Moreover, the "heat treatment at a temperature higher than or equal to a melting point of the thermal fusible fibres" in the (P3) described above may include any one of the embossing compression treatment and the hot-air treatment, or may include both. When the embossing compression treatment is performed, after the embossing compression treatment, the hot-air treatment may be performed at a temperature "lower than or equal to the melting point" of the thermally fusible fibres, and not at the "temperature higher than or equal to the melting point of the thermal fusible fibres." Moreover, in the step of the (P1) described above, as a compound having a liquid film cleavage effect, one or more of compounds selected from the compound C1 and the compound C2 described later may be used (hereinafter, collectively referred to as "liquid film cleavage agent" for convenience in the present specification). Further, in the step of the (P1) described above, coating treatment may be performed in a mixed state of the above-described liquid film cleavage agent and the hydrophilising agent described later.

The nonwoven fabric produced as described above is cut into a suitable size and formed as the topsheet. The

topsheet is processed into the absorbent article through a step of laminating and bonding the topsheet with other components of the absorbent article by an ordinary method. Specifically, an absorbent body and a backsheet are laminated and bonded on one surface side (back surface side) of the topsheet in this order to form the absorbent article. In addition, the absorbent article may be produced by a production method containing a step of assembling other members in addition to the above-described components. Examples of the step include a step of laminating side sheets for side-leak prevention on both sides in a longitudinal direction of a topsheet, a step of forming an adhesive portion for fixing an absorbent article to clothes on a non-skin side of a backsheet, a step of assembling a member serving as a wing portion for being wound on clothes and fixing an absorbent article, or a step of interposing a second sheet having liquid diffusibility between a topsheet and an absorbent body.

The liquid film cleavage agent contained in the topsheet 1 is an agent having the following (Properties of eliminating liquid film). Specifically, the liquid film cleavage agent means an agent which inhibits formation of liquid film by cleaving liquid film formed between fibres or on a surface of the fibres of the nonwoven fabric when a liquid, for example, an excreted liquid (bodily fluid) such as a high viscosity liquid including menstrual blood or urine comes into contact with the nonwoven fabric. The liquid film cleavage agent has an effect of cleaving the formed liquid film and an effect of inhibiting formation of liquid film. The former and the latter can be referred to as principal action and secondary action, respectively. For example, the liquid film cleavage agent takes such action as described in paragraphs {0024} and {0025}, FIGs. 1 and 2 in the description of WO 2016/098796. As described above, the liquid film cleavage agent cleaves the liquid film itself that is formed between the fibres or on a fibre surface, instead of liquid reforming such as reduction of the surface tension of the liquid film, to inhibit the formation of liquid film while sweeping the liquid film to promote drainage of liquid from the nonwoven fabric.

(Properties of eliminating liquid film)

The liquid film cleavage agent used in the present invention has properties of eliminating the liquid film, and owing to such properties, the liquid film cleavage agent can develop an effect of eliminating the liquid film when the liquid film cleavage agent is applied to a test liquid mainly containing a plasma component or artificial urine. The artificial urine is prepared by adjusting a mixture having a proportion of 1.940 mass% of urea, 0.795 mass% of sodium chloride, 0.111 mass% of magnesium sulphate, 0.062 mass% of calcium chloride, 0.198 mass% of potassium sulphate, 0.005 mass% of red No. 2 (dye), water (96.882 mass%) and polyoxyethylene lauryl ether (0.007 mass%), to the surface tension of $53 \pm 1$ mN/m (23°C).

The test liquid is a liquid component extracted from defibrinated equine blood (manufactured by NIPPON BIO-TEST LABORATORIES INC.). Specifically, when 100 mL of the defibrinated equine blood is left to stand under conditions of a temperature of 22°C and a humidity of 65% for 1 hour, the defibrinated equine blood is separated into an upper layer and a lower layer, in which this upper layer is the test liquid. The upper layer mainly contains the plasma component and the lower layer mainly contains a blood cell component. In order to take out only the upper layer from the defibrinated equine blood which is separated into the upper layer and the lower layer, for example, Transfer Pipet (manufactured by Nippon Micro K.K) can be used.

The effect of eliminating the liquid film herein includes, with regard to a structure in which air is held by the liquid film formed of the test liquid or the artificial urine, both an effect of inhibiting liquid film formation of the structure and an effect of eliminating the formed structure, and it can be said that the agent of developing at least one effect has properties according to which the effect of eliminating the liquid film can be developed. Specifically, a degree of a certain agent having the "properties of eliminating the liquid film" can be judged by a large or small amount of the structure, namely the liquid film, when a state of easily producing the structure is formed, in which the structure formed of the test liquid or the artificial urine to which the agent is applied. That is, a standard sample is obtained by adjusting a temperature of the test liquid or the artificial urine to 25°C, and then putting 10 g thereof in a screw bottle (manufactured by Maruemu Corporation, No. 5, shell diameter: 27 mm, overall length: 55 mm). Moreover, as a measurement sample, a material prepared by adding 0.01 g of an agent of a measurement target as adjusted beforehand to 25°C to liquid same with the standard sample is obtained. After intensively shaking the standard sample and the measurement sample up and down twice in a vertical direction of the screw bottle, respectively, the samples are immediately placed on a horizontal plane. A liquid layer (lower layer) without the structure and a structure layer (upper layer) formed of a large number of the structures formed on the liquid layer are formed inside the screw bottle after shaking by shaking the samples. After elapse of 10 seconds from the time immediately after shaking, a height of the structure layer (height from a liquid level of the liquid layer to a top surface of the structure layer) for both the samples is measured. Then, when the height of the structure layer of the measurement sample reaches 90% or less relative to the height of the structure layer of the standard sample, the agent of the measurement target is deemed as the agent having the liquid film cleavage effect.

The liquid film cleavage agent used in the present invention is an agent satisfying the properties (an agent which can develop cleavage of the liquid film) by a single compound meeting the properties, a mixture combined with plurality of the single compound meeting the properties, or a combination of a plurality of compounds. That is, the liquid

film cleavage agent means only agent limited to a material having the liquid film cleavage effect according to the definition. Accordingly, when a third component not meeting the definition is contained in the compound applied into an absorbent article, such an agent is distinguished from the liquid film cleavage agent.

In addition, with regard to the liquid film cleavage agent and the third component, the "single compound" means a compound under a concept of including a compound which has the same composition formula, but has a different molecular weight by a difference in the number of repeating units.

As the liquid film cleavage agent, a material can be appropriately selected from the materials described in the paragraphs {0007} to {0186} in WO 2016/098796, and can be used.

The expression "the topsheet 1 contains the liquid film cleavage agent" means that the liquid film cleavage agent is mainly adhered to the surface of the fibres. However, insofar as the liquid film cleavage agent remains present on the surface of the fibres, it is also acceptable for the liquid film cleavage agent to be present inside the fibres, or to be present inside the fibres by internally incorporating. As a method of adhering (providing) the liquid film cleavage agent to the surface of the fibres, any of various commonly utilised methods can be adopted without particular restriction. Specific examples of the methods include applying treatment such as flexographic printing, inkjet printing, gravure printing, screen printing, spraying and brush applying. These processings may be performed after the fibres are formed into the web by various methods or after the web is then processed into the nonwoven fabric or after the nonwoven fabric is assembled into the absorbent article. The fibres on the surface to which the liquid film cleavage agent is adhered are dried, for example, by a dryer of a hot air blowing type at a temperature sufficiently lower than melting point of a fibre resin (for example, 120°C or less). Moreover, when the liquid film cleavage agent is adhered to the fibres using the above-described adhering method, a solution containing the liquid film cleavage agent which dissolves the liquid film cleavage agent in a solvent, an emulsified liquid, or an applying liquid in a state of a dispersion liquid, may be used, when necessary. In order for the liquid film cleavage agent to have the liquid film cleavage effect described later in the nonwoven fabric, the liquid film cleavage agent is required to be present in a fluid state when the liquid film cleavage agent touches bodily fluid. Owing to this, melting point of the compound contained in the liquid film cleavage agent is preferably 40°C or less, and more preferably 35°C or less. Further, the melting point of the compound contained in the liquid film cleavage agent according to the present invention is preferably -220°C or more, and more preferably -180°C or more.

Next, the action of the liquid film cleavage agent will be described below in relation with the arrangement in the present embodiment.

As mentioned above, the liquid film cleavage agent exhibits spreadability to a liquid film between the fibres and on the fibres in the arrangement portion 11 to cleave the liquid film. Further, the liquid film cleavage agent moves from a position of the arrangement portion 11 to any other region (a region that is not the arrangement portion, namely, the non-arrangement portion 12) of the topsheet 1 by the spreadability to exhibit the liquid film cleavage action. The former is referred to as micro spreadability, and the latter is referred to as macro spreadability. The macro spreadability of the liquid film cleavage agent is developed in both the directions of the plane direction and the thickness direction of the topsheet 1.

The macro spreadability of the liquid film cleavage agent is induced in the plane direction, and therefore an area ratio of the arrangement portion 11 of the liquid film cleavage agent can be suppressed at a low level. That is, in a region having the arrangement portion 11 in the topsheet 1, the non-arrangement portion 12 is further provided, whereby the arrangement portion 11 and the non-arrangement portion (part in which no liquid film cleavage agent is arranged) 12 can be mixed in the plane direction. In addition, the term "a region having the arrangement portion 11" means a whole region of the topsheet 1 in the plane direction in the thickness immediate position 13 of the topsheet 1, or in the back surface 1B (surface facing the absorbent body side) of the topsheet 1.

The macro spreadability in the plane direction is confirmed by the experiment as described below. That is, an arrangement portion of a liquid film cleavage agent (basis weight: 1.3 g/$m^2$, OPU: 0.4 mass%) was formed in a dot shape having a diameter of 0.8 mm on a surface of a nonwoven fabric having the same structure as the structure of the above-described topsheet 1, and quasi-menstrual blood (6.0 g) was added dropwise thereto. At this time, the liquid film cleavage agent was coloured with a red paint so that a region of the arrangement portion was able to be confirmed. The liquid film cleavage agent may be coloured with an arbitrary colour such as blue as long as the arrangement portion can be visually recognised. In addition, the quasi-menstrual blood is a material prepared by adjusting a viscosity of defibrinated equine blood manufactured by NIPPON BIO-TEST LABORATORIES INC. to 8.0 cP. Specifically, the used defibrinated equine blood was adjusted by TVB-10 Viscometer manufactured by Toki Sangyo Co., Ltd. under conditions of 30 rpm. When the defibrinated equine blood is allowed to stand, a part with high viscosity (red blood cells or the like) precipitates, and a part with low viscosity (plasma) remains as a supernatant. A mixing ratio in the parts was adjusted to be 8.0 cP (hereinafter, as the quasi-menstrual blood described in the present specification, blood prepared by the same method is used unless otherwise stated.).

Specifically, from a sanitary towel (trade name: LAURIER F, Shiawase Suhada, 22.5 cm, manufactured by Kao Corporation in 2016) as one example of the absorbent article, a topsheet was carefully removed after spraying the

sanitary towel with a cold spray to solidify an adhesive (in addition, in the present specification, upon removing a component from the absorbent article, this method is used in all cases.). In place of the topsheet, the above-described nonwoven fabric was laminated in such a manner that a surface of a concavo-convex structure existed on a skin-contact surface side, and an acrylic plate having a penetration hole having an inner diameter of 1 cm was placed in such a manner that centres of the penetration hole and the arrangement portion were overlapped. A predetermined load of 100 Pa was applied to the sanitary towel, 6.0 g of quasi-menstrual blood was injected from the penetration hole of the above-described acrylic plate under such a load, and after leaving the assembly to stand for 60 seconds from completion of injection, the acrylic plate was removed. Then, it was confirmed that the liquid film cleavage agent is concentrically spread in a range (K1) having a diameter of 4.6 mm being about 5.7 times a diameter of the arrangement portion 11 to exhibit the liquid film cleavage action, whereby redness M1 of the quasi-menstrual blood is dissolved (see FIGs. 5(A) and 5(B)). That is, in a wide range beyond a range of the arrangement portion 11 of the nonwoven fabric, the quasi-menstrual blood remaining as the liquid film was permeated to the absorbent body to reduce the residual liquid of the nonwoven fabric. When an arrangement amount of the liquid film cleavage agent in the arrangement portion 11 is increased, the liquid film cleavage agent may be spread in a wider range to exhibit the liquid film cleavage action. For example, when the basis weight is adjusted to 25.9 g/m$^2$ (OPU 8.0 mass%) in the arrangement portion 11 having the same diameter, it was confirmed that the liquid film cleavage agent is concentrically spread in a range (K2) having a diameter of 12 mm being about 15 times the diameter of the arrangement portion 11 to exhibit the liquid film cleavage action, whereby the redness M1 of the quasi-menstrual blood is dissolved (see FIGs. 5(A) and 5(C)).

Moreover, the macro spreadability of the liquid film cleavage agent is also induced in thickness direction.

Accordingly, in the present embodiment, when liquid flow settles down, as compared with a state during liquid injection and immediately after liquid injection, the liquid film cleavage agent is spread from the thickness immediate position 13 or the back surface 1B side of the topsheet 1 to the top surface 1A side being a liquid-receiving surface. On the top surface 1A being the liquid-receiving surface, a phenomenon of exhibiting the liquid film cleavage action may occur with a time difference in the thickness intermediate position 13 or on the back surface 1B side. Thus, even if the arrangement portion 11 of the liquid film cleavage agent exists in the thickness intermediate position 13 or on the back surface 1B side, and does not exist on the top surface 1A, the residual liquid on the topsheet 1 as a whole can be suppressed at a low level, and dryness at a high level can be realised. Moreover, dissolution of the redness can be confirmed on the top surface 1A side, and therefore a wearer can perceive, with the skin and eyes, that the residual liquid is suppressed at the low level.

Further, when the arrangement portion 11 exists in the thickness intermediate position 13, the liquid film cleavage agent may be spread not only in a top surface 1A direction of the topsheet 1, but also in a back surface 1B direction thereof. In this case, the liquid film cleavage agent is simultaneously spread from the thickness intermediate position 13 to the top surface 1A direction and the back surface 1B direction, whereby the liquid film cleavage action in the thickness of the topsheet 1 as a whole may be efficiently and promptly exhibited. Therefore, the effect of reduction of residual liquid is further improved when the arrangement portion 11 exists in the thickness intermediate position 13 than when the arrangement portion 11 exists on the back surface 1B side.

In addition thereto, the arrangement portion 11 of the liquid film cleavage agent exists in the thickness intermediate position 13 or on the back surface 1B side, whereby the non-arrangement portion on the top surface 1A side is interposed between the skin and the arrangement portion 11 of the liquid film cleavage agent, and thus a contact chance between the liquid film cleavage agent and the wearer's skin is suppressed, and prevention capability of the sticky feeling on skin is high. The arrangement portion OPU of the liquid film cleavage agent in the arrangement portion 11 in the thickness intermediate position 13 or on the back surface 1B side is appropriately set, whereby excessive spread of the liquid film cleavage agent to the top surface 1A can be controlled and a contact between the liquid film cleavage agent and the skin can be preferably suppressed. Therefore, the sticky feeling on skin can be made harder to feel in providing the arrangement portion 11 in the thickness intermediate position 13 or on the back surface 1B side than in providing the arrangement portion 11 on the top surface 1A. That is, a feeling of the topsheet 1 sticking to or clinging to the skin can be suppressed, whereby skin dry feeling may be kept at a high level.

In addition, a preferable range and a measuring method of an arrangement proportion (arrangement portion OPU) of the liquid film cleavage agent in the arrangement portion 11 will be described later together with a preferable range and a measuring method of an arrangement proportion of the liquid film cleavage agent to total fibre mass of the topsheet (hereinafter, also referred to as "average OPU" of the liquid film cleavage agent).

Further, when the topsheet 1 is the laminated sheet, a form of each fibre layer can be changed, and such a case is preferable. For example, a capillary pressure on the back surface 1B side can be increased than on the top surface 1A side to increase the capillary pressure difference between both the surfaces, whereby the liquid after the liquid film is cleaved by the liquid film cleavage agent can be further easily sucked into the absorbent body 3 than in a single layer. Specific examples of the form of each fibre layer for increasing the capillary pressure difference include a form in which basis weight in a non-skin side layer is increased than in a skin side layer, or a fibre diameter in the non-skin side layer is reduced than in the skin side layer to increase fibre density or a degree of hydrophilicity.

Moreover, in the laminated sheet, an arrangement pattern of the arrangement portion 11 of the liquid film cleavage agent, or the arrangement proportion of the liquid film cleavage agent can be set with satisfactory precision. Therefore, when the topsheet 1 is the laminated sheet, the liquid film cleavage action of the liquid film cleavage agent arranged with satisfactory precision in the thickness intermediate position 13 is combined with liquid sucking force by the above-described capillary pressure difference or the like, whereby a higher effect of reduction of residual liquid is exhibited, and such a case is preferable.

The macro spreadability in the thickness direction is confirmed by the experiment as described below. That is, in a concavo-convex topsheet (nonwoven fabric) 1 shown in FIG. 2, as shown in FIG. 6(B), an arrangement portion 11 (arrangement portion OPU: 0.4 mass%) was formed in a dot shape having a diameter of 0.8 mm in a convex top portion of a back surface 1B. In addition, similarly to the case in FIG. 5, a liquid film cleavage agent was coloured with a red paint so that a range of the arrangement portion was able to be confirmed. Then, as shown in FIG. 6(A), a topsheet 1 was placed on an absorbent body 3 with a top surface 1A upward, and an acrylic plate 100 having an input port of a cylinder 101 was placed on a sample. Then, 6.0 g of quasi-menstrual blood was injected thereinto. It was confirmed that redness M1 gradually disappears from a lower side to a top surface 1A side as time passes (a sign K6 in FIG. 7(B), a sign K7 in FIG. 7(C)) after 20 seconds (after liquid flow settled down) (FIG. 7(B)), and after 30 seconds (FIG. 7(C)) from leaving an assembly to stand, from a state immediately after injection of the liquid (FIG. 7(A)). That is, it was confirmed that the liquid film cleavage agent is spread from the back surface 1B side to the top surface 1A side, and the liquid film cleavage action on the quasi-menstrual blood in the thickness direction of the topsheet 1 is exhibited, whereby the quasi-menstrual blood remaining as the liquid film is permeated into the absorbent body.

A degree of the macro spreadability of the liquid film cleavage agent is determined depending on various factors. For example, as a spreading coefficient described later is larger, a spreading distance in the liquid drop becomes longer to exhibit higher spreadability. Moreover, as an arrangement proportion of the liquid film cleavage agent in the arrangement portion 11 is larger, the spreading distance in the liquid drop becomes longer to exhibit the higher spreadability.

Moreover, the liquid film cleavage agent exhibits preferable high spreadability for the liquid drop by moderately suppressing viscosity thereof. When the arrangement portion 11 of the liquid film cleavage agent exists in the thickness intermediate position 13 or on the back surface 1B side, upon considering spreading of the liquid film cleavage agent in the thickness direction from the arrangement portion 11 to the top surface 1A being the liquid-receiving surface, viscosity of the liquid film cleavage agent is preferably suppressed at a moderate level, as compared with a case where the arrangement portion 11 exists on the top surface 1A. Specifically, when the arrangement portion 11 of the liquid film cleavage agent exists in the thickness intermediate position 13 or the back surface 1B side, the viscosity of the liquid film cleavage agent is 0 cps or more; and preferably 6,000 cps or less, more preferably 600 cps or less, and further preferably 200 cps or less. In addition, a unit cps of the viscosity is converted according to an equation: $1\ \text{cps} = 1 \times 10^{-3}\ \text{Pa s}$.

(Method for measuring viscosity of liquid film cleavage agent)

The viscosity of the liquid of the liquid film cleavage agent can be measured by the following method.

First, 40 g of a liquid film cleavage agent is prepared. Next, the viscosity is measured on the liquid film cleavage agent in an environment range of a temperature of 25°C and a relative humidity (RH) of 65% by using Tuning Fork Vibro Viscometer SV-10 (manufactured by A&D Company, Limited). This operation is repeated three times, and an average value is adopted as the viscosity. In addition, when the liquid film cleavage agent is solid, the liquid film cleavage agent is heated to a temperature of a melting point of the liquid film cleavage agent plus 5°C to induce phase transition into liquid, and the measurement is performed with keeping the temperature conditions.

In addition, when the viscosity of the liquid film cleavage agent adhered to the fibre is measured, the liquid film cleavage agent is taken out from the fibre based on the method used in measurement of the spreading coefficient or the like described later. In this case, when only a small amount thereof can be taken out for the above-described measurement, the viscosity is measured in the same manner as the case of measurement of the spreading coefficient or the like described later.

As described above, in the present embodiment, the liquid (bodily fluid) in contact with the topsheet 1 easily passes through a significantly fine inter-fibre space or the like without being stayed therein by the above-described action of the liquid film cleavage agent, whereby reduction of residual liquid in the topsheet 1 may be realised at a higher level. Furthermore, even if an arrangement of the liquid film cleavage agent on the top surface 1A of the topsheet 1 is reduced or eliminated, the liquid film cleavage agent existing in the thickness intermediate position 13 or on the back surface 1B side develops the above-described macro spreadability, whereby the effect of reduction of residual liquid in the topsheet 1 as a whole can be obtained in the same manner to the case where the liquid film cleavage agent exists on the top surface 1A. Moreover, as mentioned above, the contact between the liquid film cleavage agent on the top surface 1A of the topsheet 1 and the skin can be suppressed. Thus, in the present embodiment, reduction of residual liquid and reduction of the sticky feeling on skin can be simultaneously realised.

In addition thereto, in the present embodiment, liquid flow on the surface on the top surface 1A of the topsheet

1 can be preferably suppressed.

That is, the liquid film cleavage agent has high spreadability to the liquid film, and therefore has smaller surface tension than a conventional hydrophilisation treatment agent or the like used for nonwoven fabric fibres. Therefore, when the liquid film cleavage agent is arranged, wettability of the fibres in an initial stage of contact with the liquid is somewhat reduced, as compared with a case where no liquid film cleavage agent is arranged. On the top surface 1A of the topsheet 1, flow of the bodily fluid (for example, the excreted liquid such as menstrual blood or urine) on the surface is further easily exhibited, as compared with the case where no liquid film cleavage agent is arranged.

In contrast thereto, in the present embodiment, the arrangement portion 11 of the liquid film cleavage agent is arranged in the thickness intermediate position 13 or on the back surface 1B side of the topsheet 1. That is, the present embodiment is configured in such a manner that reduction of hydrophilicity on the surface of the topsheet 1 by the liquid film cleavage agent is suppressed to retain the wettability on the surface. Thus, together with the effect of reduction of residual liquid by the liquid film cleavage agent, the liquid flow on the surface on the top surface 1A side of the topsheet 1 is prevented, whereby liquid permeability in the thickness direction can be retained. Furthermore, the liquid film cleavage agent can exhibit the liquid film cleavage action by the spreadability not only in the arrangement portion 11, but also in a wide range in the thickness direction and the plane direction of the topsheet 1. Thus, even if the liquid film cleavage agent exists in the thickness intermediate position 13 or on the back surface 1B side, the effect of reduction of residual liquid of the topsheet 1 as a whole can be obtained in the same manner to the case where the liquid film cleavage agent exists on the top surface 1A. In addition thereto, the liquid film cleavage agent may retain a passage space in which the bodily fluid is delivered from the topsheet 1 to the absorbent body 3 on the back surface 1B side of the topsheet 1. Moreover, the liquid sucking force of the absorbent body 3 can be easily transferred to the top surface 1A side through the passage space. Thus, cooperation performance of liquid sucking is enhanced between the absorbent body 3 and the topsheet 1, whereby liquid absorbency of the sanitary towel 10 may be improved. This liquid absorbency is further improved by formation of the top surface 1A side of the topsheet 1 in the concavo-convex shape as shown in Fig. 2.

From a viewpoint of suppressing the sticky feeling on skin on the top surface 1A of the topsheet 1, and from a viewpoint of enhancing prevention capability of bodily fluid flow on the surface, an arrangement of the arrangement portion 11 on the top surface 1A side of the topsheet 1 is preferably reduced.

Specifically, the area ratio of the arrangement portion 11 in an area of the topsheet 1 is preferably adjusted to be smaller on the top surface 1A (surface facing the wearer's skin side) of the topsheet 1 than in the thickness intermediate position 13 or on the back surface 1B (surface facing the absorbent body side) of the topsheet 1. Further, it is more preferable that no arrangement portion 11 exists on the top surface 1A (surface facing the wearer's skin side) of the topsheet 1. In addition, in FIG. 3 and FIG. 4, the present embodiment is shown as an aspect in which no arrangement portion 11 of the liquid film cleavage agent is provided on the top surface 1A.

The area ratio of the arrangement portion 11 is calculated according to a formula: {(total area of arrangement portion)/(total area of arrangement portion and non-arrangement portion)} $\times$ 100. The term "total area of arrangement portion and non-arrangement portion" means the area of the topsheet 1. The area of the topsheet 1 relating to calculation of the above-described area ratio is the area defined by an external shape of the topsheet 1 in any case of the thickness intermediate position 13, the top surface 1A and the back surface 1B of the topsheet 1.

(Method for confirming presence or absence of liquid film cleavage agent in thickness direction)

A topsheet 1 is carefully peeled off and removed from an absorbent article to be measured, and a sample cut out into 3.0 cm square is prepared, and the sample is arranged in a liquid-receiving region of the absorbent article. For example, a topsheet is removed from a sanitary towel (trade name "LAURIER F, Shiawase Suhada, 22.5 cm," manufactured by Kao Corporation in 2016) as one example of an absorbent article, and the sample is prepared. Then, the above-described sample is stacked on the liquid-receiving region of the above-described sanitary towel. The liquid-receiving region in the above-described sanitary towel is a region existing from a position of 7.5 cm to 15.0 cm from a front portion F, and in 2.5 cm or less in a crosswise direction from a line dividing a length of the sanitary towel in a transverse direction into two equal parts.

Then, an acrylic plate having a penetration hole having an inner diameter of 1 cm is placed in such a manner that centres of the penetration hole and the above-described sample are overlapped, and a predetermined load of 100 Pa is applied to the sanitary towel, and 6.0 g of quasi-menstrual blood is injected from the penetration hole of the acrylic plate under such a load. Immediately after injection, the acrylic plate is removed, and the above-described sample is immersed into liquid nitrogen, and the sample is cut along the crosswise direction of the topsheet 1 passing through the centre of the above-described sample by using a cutter. A cut cross section is observed with an optical microscope, and in a thickness intermediate position of the above-described sample, a part in which redness of the quasi-menstrual blood is further dissolved than on a top surface side and a back surface side is the arrangement portion 11, and a part other than the above-described part is the non-arrangement portion 12. Then, when the topsheet 1 is divided into substantially three equal parts in the thickness direction, if the part in which the redness is dissolved exists in a layer on a surface

side of a skin side layer being a division closest to the skin side, the arrangement portion 11 of the liquid film cleavage agent is judged to exist on the top surface side, if the part in which the redness is dissolved exists in a layer on the surface side of a non-skin side layer being a division farthest from the skin side, the arrangement portion 11 of the liquid film cleavage agent is judged to exist on the back surface side (absorbent body side), and if the part in which the redness is dissolved exists in a layer of a middle division, the arrangement portion 11 of the liquid film cleavage agent is judged to exist in the thickness intermediate position.

(Method for confirming presence or absence of liquid film cleavage agent in plane direction)

Presence or absence of the liquid film cleavage agent when the arrangement portion 11 of the liquid film cleavage agent of the topsheet 1 exists on the top surface 1A side or the back surface 1B side can be confirmed by the following method. That is, facial oil blotting paper is attached to each surface of the topsheet 1, and then a 4 mm-thick acrylic plate is placed thereon, and a load is applied thereto to be 600 g/cm$^2$ for 30 seconds with a weight from above. Immediately after application of the load, the facial oil blotting paper is peeled off, and the facial oil blotting paper is placed on a black mat, and a change in colour is visually confirmed. When the change in colour is found, the change shows that the liquid film cleavage agent is contained in the part. That is, a part of the topsheet 1 corresponding to a colour-change part of the facial oil blotting paper is the arrangement portion 11 of the liquid film cleavage agent, and a part other than the above-described part is the non-arrangement portion 12.

Various materials can be used as the above-described facial oil blotting paper, and specific examples thereof include Facial blotting paper made from gold leaf pounding paper, manufactured by KATANI co., ltd.

Presence or absence of the liquid film cleavage agent when the arrangement portion 11 in the topsheet 1 exists in the thickness immediate position as shown in FIG. 3 is confirmed by the same method as the above-described (Method for confirming presence or absence of liquid film cleavage agent in thickness direction), in which a topsheet 1 is removed, and a sample cut out into 3.0 cm square is prepared. Then, the above-described sample is stacked on a liquid-receiving region of the above-described sanitary towel.

Then, an acrylic cylindrical plate (height: 3.0 cm, thickness of acrylic plate: 3 mm) having an elliptical penetration hole having a major axis of 5.0 cm and a minor axis of 2.3 cm is placed in such a manner that centres of the penetration hole and the above-described sample are overlapped, and 6.0 g of quasi-blood having viscosity adjusted to 40 cP is injected from the penetration hole of the cylindrical plate to be uniformised in the plane direction. Immediately after completion of injection, the cylindrical plate is removed and the sample is left to stand, and a part in which redness is reduced and a part in which the redness is not reduced come out within 60 seconds from injection. At this time, the part in which the redness is reduced is the arrangement portion 11 and the part in which the redness is not reduced is the non-arrangement portion 12.

(Method for measuring area ratio of arrangement portion 11 and non-arrangement portion 12)

When the arrangement portion 11 in the topsheet 1 exists on the top surface 1A side or the back surface 1B side, the area ratio is measured according to the procedure described in the former part of the above-mentioned (Method for confirming presence or absence of liquid film cleavage agent in plane direction) for each entire surface of the topsheet 1. That is, a load is applied thereto, and an area of a part in which a colour-change part of facial oil blotting paper is copied on an OHP sheet is taken as an area of the arrangement portion 11, and a value obtained by subtracting the area of the above-described arrangement portion 11 from the area of the topsheet 1 is taken as an area of the non-arrangement portion 12.

When the arrangement portion 11 in the topsheet 1 exists in the thickness immediate position 13 as shown in FIG. 3, the area ratio is measured according to the procedure described in the latter part of the above-mentioned (Method for confirming presence or absence of liquid film cleavage agent in plane direction), in which no sample is cut out. That is, when quasi-menstrual blood is injected thereinto and the resulting material is left to stand, and a part in which redness is reduced and a part in which the redness is not reduced come out within 60 seconds from injection, a sample is immersed into liquid nitrogen. An OHP sheet is immediately placed thereon, and the part in which the redness is reduced is copied on the OHP sheet, and an area of the copied part is taken as an area of the arrangement portion 11, and a value obtained by subtracting the area of the above-described arrangement portion 11 from an area of the topsheet 1 is taken as an area of the non-arrangement portion 12. The above-described operation is performed in an arbitrary position of the topsheet 1.

The area ratio is calculated by using the measured areas of the arrangement portion 11 and the non-arrangement portion 12 according to a formula: {(total area of arrangement portion)/(total area of arrangement portion and non-arrangement portion)} $\times$ 100.

In the present embodiment, from a viewpoint of retaining high sucking capability of the bodily fluid in the topsheet 1, the capillary pressure on the back surface 1B side is preferably increased than on the top surface 1A side to increase

the capillary pressure difference between both the surfaces. As mentioned above, the liquid film cleavage agent somewhat reduces wettability of the fibres. That is, the liquid film cleavage agent has a possibility of somewhat increasing a contact angle of the fibres to influence the above-described capillary pressure difference. In contrast thereto, in the present embodiment, the capillary pressure difference between both the surfaces can be controlled to a suitable state by taking the following aspect.

As one aspect, the arrangement portion 11 and the non-arrangement portion 12 of the liquid film cleavage agent are preferably mixed in the thickness intermediate position 13 or on the back surface 1B side of the topsheet 1. In addition, even in this case, the liquid film cleavage agent can sufficiently exhibit the liquid film cleavage action also in the non-arrangement portion 12 by the macro spreadability as mentioned above.

According to the above-described mixing, reduction of hydrophilicity of the fibres by the liquid film cleavage agent can be preferably suppressed as a region as a whole on each surface in the thickness intermediate position 13 or on the back surface 1B side. That is, a degree of hydrophilicity on the absorbent body 3 side is increased than on the top surface 1A side for the thickness direction of the topsheet 1, whereby the capillary pressure difference between both the surfaces can be controlled to be large.

From a viewpoint of appropriately retaining the capillary pressure difference, the area ratio of the arrangement portion 11 is preferably 75% or less, more preferably 60% or less and further preferably 50% or less in the thickness intermediate position 13 and on the back surface 1B, respectively. Moreover, from a viewpoint of developing the liquid film cleavage effect effectively, the area ratio of the arrangement portion 11 is preferably 5% or more, more preferably 10% or more, and further preferably 20% or more.

A pattern of mixture of the arrangement portion 11 and the non-arrangement portion 12 in the plane direction may take various aspects. Specific examples thereof include a pattern in which both an arrangement portion 11 and a non-arrangement portion 12 are extended in a strip shape in a longitudinal direction (Y direction), and a strip-shaped arrangement portion 11 and a strip-shaped non-arrangement portion 12 are alternately arranged in a crosswise direction (X direction) (a vertical stripe pattern); a pattern in which stripes of an arrangement portion 11 and a non-arrangement portion 12 extending in a crosswise direction (X direction) are alternately arranged in a longitudinal direction (Y direction) (a lateral stripe pattern); a pattern in which a plurality of arrangement portions 11 patterned in a rhombus shape is separated from each other and arranged in a lattice-shaped non-arrangement portion 12 (a lattice design); a pattern in which a plurality of non-arrangement portions 12 patterned in a rhombus shape is separated from each other and arranged in a lattice-shaped arrangement portion 11; and a pattern in which arrangement portions 11 formed in a circle (dot shape) are separated from each other and arranged in a non-arrangement portion 12.

As another aspect, a hydrophilising agent is preferably arranged in the topsheet 1 as described below. That is, an arrangement amount of the hydrophilising agent is increased on the surface (back surface) 1B facing the absorbent body 3 side than on the surface (top surface) 1A facing the wearer's skin side of the topsheet 1. On the surface on which the arrangement portion 11 of the liquid film cleavage agent is formed, coating treatment is preferably performed in a mixed state of the liquid film cleavage agent and the hydrophilising agent. According to a gradient of the arrangement amount of the hydrophilising agent, reduction of hydrophilicity of the fibres by the liquid film cleavage agent can be preferably suppressed, and the capillary pressure difference between both the surfaces can be controlled so as to be increased.

From a viewpoint of increasing the capillary pressure difference, a mass ratio of a total amount of the arrangement amount of the hydrophilising agent on the back surface 1B of the topsheet 1 to a total amount of the arrangement amount of the hydrophilising agent on the top surface 1A of the topsheet 1 is preferably 1.2 or more, more preferably 1.7 or more, and further preferably 2.7 or more. Moreover, from a viewpoint of successfully discharging the liquid from an inside of the topsheet 1 to the absorbent body, the mass ratio is preferably 17.3 or less, more preferably 9.1 or less, and further preferably 5.9 or less. In addition, this mass ratio is calculated based on "Method for measuring arrangement proportion" of the hydrophilising agent mentioned later.

From a viewpoint of further elucidating the capillary pressure difference in the thickness direction of the topsheet, a mass ratio of a total amount of the hydrophilising agent in the applying liquid to a total amount of the applying liquid {(hydrophilising agent)/(applying liquid)} is preferably 0.3 or more, more preferably 0.5 or more, and further preferably 0.75 or more. Moreover, from a viewpoint of successfully obtaining an effect of the liquid film cleavage agent, the above-described mass ratio {(the hydrophilising agent in the applying liquid)/(the applying liquid)} is preferably 0.9 or less, more preferably 0.85 or less, and further preferably 0.8 or less. In addition, this mass ratio is calculated based on "Method for measuring arrangement proportion" of the liquid film cleavage agent and the hydrophilising agent in the applying liquid mentioned later.

In addition, the above-described mass ratio can be measured by applying mutatis mutandis the method described in the paragraph [0018] of WO 2016/098796.

From a viewpoint of increasing the capillary pressure difference in the thickness direction of the topsheet while providing moderate hydrophilicity at which the liquid is easily entered into the fibres on the surface of the nonwoven fabric forming the topsheet, a total amount of the hydrophilising agent ("hydrophilising agent previously coated on the

fibre web serving as the nonwoven fabric sample" and "hydrophilising agent in the applying liquid") is preferably 0.3 mass% or more, more preferably 0.6 mass% or more, and further preferably 0.9 mass% or more in terms of the arrangement proportion (average OPU) to the total fibre mass of the topsheet 1. Moreover, from a viewpoint of immediately and successfully transferring the liquid to the absorbent body, the average OPU of the total amount of the hydrophilising agent ("hydrophilising agent previously coated on the fibre web serving as the nonwoven fabric sample" and "hydrophilising agent in the applying liquid") is preferably 6 mass% or less, more preferably 3 mass% or less, and further preferably 2 mass% or less.

Meanwhile, from a viewpoint of effectively exhibiting the liquid film cleavage action, a total amount of the liquid film cleavage agent is preferably 0.1 mass% or more, more preferably 0.2 mass% or more, and further preferably 0.3 mass% or more in terms of the arrangement proportion (average OPU) to the total fibre mass of the topsheet 1. Moreover, from a viewpoint of effectively suppressing the liquid flow on the surface, the average OPU of the liquid film cleavage agent is preferably 10 mass% or less, more preferably 7.5 mass% or less, and further preferably 5 mass% or less.

In addition, the average OPU represents the arrangement proportion of the total amount of each component to the total fibre mass of the topsheet 1. When the component to be arranged is the liquid film cleavage agent, the average OPU represents the arrangement proportion of the total amount of the liquid film cleavage agent to the total fibre mass of the topsheet 1. Moreover, also when the component to be arranged is the hydrophilising agent and/or the applying liquid, the average OPU represents the arrangement proportion of the total amount of the hydrophilising agent and/or the applying liquid to the total fibre mass of the topsheet 1.

From a viewpoint of sufficiently spreading of the liquid film cleavage agent to the non-arrangement portion 12 in the plane direction and the thickness direction of the topsheet 1 to exhibit the liquid film cleavage action, the total amount of the liquid film cleavage agent is preferably 0.1 mass% or more, more preferably 0.4 mass% or more, and further preferably 0.8 mass% or more in terms of the arrangement proportion (arrangement portion OPU) to the fibre mass in the arrangement portion 11 in the topsheet 1. Moreover, from a viewpoint of reducing the sticky feeling on skin, which may be caused by a local increase in the arrangement amount of the liquid film cleavage agent, and from a viewpoint of achieving favorable texture of the topsheet 1, the arrangement portion OPU of the liquid film cleavage agent is preferably 8 mass% or less, more preferably 4 mass% or less, and further preferably 1.5 mass% or less.

In addition, the arrangement portion OPU represents the arrangement proportion of the total amount of each component to the fibre mass in the arrangement portion 11 in the topsheet 1. When the component to be arranged is the liquid film cleavage agent, the arrangement portion OPU represents the arrangement proportion of the total amount of the liquid film cleavage agent to the fibre mass in the arrangement portion 11 in the topsheet 1, and when the component to be arranged is the hydrophilising agent and/or the applying liquid, the arrangement portion OPU represents the arrangement proportion of the total amount of the hydrophilising agent and/or the applying liquid to the fibre mass in the arrangement portion 11 in the topsheet 1.

(Method for measuring arrangement proportion)

(1) Arrangement proportion of liquid film cleavage agent, hydrophilising agent and the like to total fibre mass of topsheet 1 (average OPU):

A topsheet is removed from a top surface of an absorbent article, and test pieces obtained by dividing the topsheet into three parts in a thickness direction are prepared in the same manner as the above-described (Method for confirming presence or absence of liquid film cleavage agent in plane direction). Mass of each test piece is measured, and then a liquid film cleavage agent, a hydrophilising agent and other components such as an adhesive attached to each test piece are removed by using a solvent such as ethanol and water. Mass obtained by totaling the liquid film cleavage agent, the hydrophilising agent and other components is elucidated from mass of a residue thereof.

Then, measurement conditions such as a column and a solvent are appropriately set, and then an extract is separated and isolated by using liquid chromatography, and a mass ratio of the liquid film cleavage agent, the hydrophilising agent and other components is elucidated from a mass ratio of an isolate. Moreover, the above-described mass ratio, the above-described mass of the residue and the above-described mass of the topsheet are used to calculate the above-mentioned average OPU (mass%) relating to each component.

In addition, among the isolates, whether any isolate corresponds to any of the liquid film cleavage agent, the hydrophilising agent and other components is judged after identifying a molecular structure of a substance according to the following measuring method, and obtaining a structure as a single substance corresponding thereto, and measuring physical properties of the structure.

(Method for identifying molecular structure)

A molecular weight of the isolate is measured using any of mass spectrometry (MS) and gel permeation chromatography (GPC). In addition thereto, a molecular skeleton is identified by using $^{1}$H-NMR, $^{13}$C-NMR or $^{29}$Si-NMR, a functional group owned by the isolate is identified by using IR, and a proportion of elements is elucidated by using elemental analysis, and then the molecular structure is identified by comprehensively analysing all the above-described information.

(2) Arrangement proportion of liquid film cleavage agent and hydrophilising agent to fibre mass in arrangement portion 11 (arrangement portion OPU):

The area ratio of the total arrangement portion 11 of each layer of the topsheet 1 obtained in the above-mentioned (Method for measuring area ratio of arrangement portion 11 and non-arrangement portion 12), and the arrangement proportion of each component to the total fibre mass of each test piece obtained in the (1) described above are used to calculate the arrangement proportion (arrangement portion OPU) of the liquid film cleavage agent and the hydrophilising agent to the fibre mass in the arrangement portion 11, by a formula: "(arrangement proportion of each component to fibre mass of each test piece)/(area ratio of total arrangement portion 11 of each layer of topsheet 1)".

Specific examples of another preferable aspect of the topsheet 1 used in the absorbent article according to present invention include a topsheet 20 assembling laminated fibre sheets as shown in FIG. 8.

A topsheet 20 has a double-layered structure having a hollow portion 21. All layers contain thermoplastic fibres. The topsheet 20 has a bonding portion 22 in which an upper layer sheet 20A being a skin side layer and a lower layer sheet 20B being a non-skin side layer are partially thermally fusion bonded. A non-bonding portion surrounded by the bonding portions 22 has a large number of convex portions 23 in which the upper layer sheet 20A is projected in a direction apart from the lower layer sheet 20B, with the hollow portion 21 inside thereof. The bonding portion 22 is a concave portion positioned between the adjacent convex portions 23 and 23 to form concavity and convexity of the top surface 1A together with the convex portion 23. The topsheet fabric 20 can be formed by a method to be ordinarily used. For example, the upper layer sheet 20A is imparted to a concavo-convex shape by engagement of two concavo-convex rolls, and then the lower layer sheet is laminated thereon to obtain the topsheet 20. From a viewpoint of shaping the nonwoven fabric by engagement of the concavo-convex rolls, any of the upper layer sheet 20A and the lower layer sheet 20B preferably contains thermally non-extensible and thermally non shrinkable thermally fusible fibres.

When the topsheet 20 is laminated on an absorbent body with the top surface 1A directed toward a skin-contact surface side, and used, the topsheet 20 is excellent in liquid permeability from a side of the top surface 1A to a side of the back surface 1B. Specifically, liquid permeation through the hollow portion 21 is excellent. Moreover, a wearer's body pressure is applied to the convex portions 23, and the liquid in the convex portion 23 directly migrates to a lower layer sheet 20B. Thus, residual liquid on the side of the top surface 1A is small. Such effect may be sustainably exhibited at a higher level by the effect of the liquid film cleavage agent or the cooperative effect of the liquid film cleavage agent and the phosphoric acid ester type anionic surfactant as mentioned above. That is, even if long-time use or a large amount of drain is experienced, a permeation passage of the liquid is ensured by liquid film cleaving, and therefore the liquid permeability as described above can be sufficiently exhibited.

In this topsheet 20, the expression: the arrangement portion 11 of the liquid film cleavage agent exists "in the thickness intermediate position of the topsheet 20" means that the arrangement portion 11 exists on a surface of the upper layer sheet 20A, facing the lower layer sheet 20B, or on a surface of the lower layer sheet 20B, facing the upper layer sheet 20A. Moreover, the expression: the arrangement portion 11 of the liquid film cleavage agent exists "on the absorbent body side of the topsheet 20" means that the arrangement portion 11 exists on a surface side on the absorbent body side of the lower layer sheet 20B. According to presence of the arrangement portion 11 of the liquid film cleavage agent as described above, reduction of residual liquid and reduction of the sticky feeling on skin in the absorbent article can be simultaneously realised by the liquid film cleavage action accompanied by the above-mentioned macro and micro spreadability of the liquid film cleavage agent.

Next, the hydrophilising agent and the liquid film cleavage agent will be described.

As the hydrophilising agent in the present invention, an agent used in the topsheet can be used without restriction. In particular, an agent having difficulty in reducing the liquid film cleavage action of the liquid film cleavage agent is preferable. Specifically, an agent having difficulty in reducing surface tension of the bodily fluid as a target of liquid film cleavage is preferable. That is, an agent having a slow dissolution rate or low solubility in the above-described bodily fluid is preferable. In the topsheet 1 in which such a hydrophilising agent is arranged in combination with the liquid film cleavage agent, the spreadability of the liquid film cleavage agent on the liquid film is retained, whereby the liquid film cleavage action may be sufficiently exhibited.

The hydrophilising agent preferably contains one or more of compounds selected from a nonionic surfactant (D1), and a compound having surface tension of 42 mN/m or more (D2), as specific examples of the agent having the slow dissolution rate or low solubility in the bodily fluid. Hereinafter, these are referred to as a hydrophilising agent (D1) and a hydrophilising agent (D2), respectively.

It is preferable that the hydrophilising agent (D1) contains one or more kinds of surfactants selected from a

hydrocarbon-based nonionic surfactant and a silicone-based nonionic surfactant as the component for enhancing hydrophilicity on the fibre surface of the nonwoven fabric arranging the liquid film cleavage agent, and the surfactants have a hydrophobic group whose hydrophobicity is lower than the hydrophobicity of a fluorocarbon group. More specifically, examples of the hydrophobic group preferably include a nonionic surfactant having "an alkyl chain or a silicone chain, excluding a polyhydric alcohol fatty acid skeleton". Moreover, examples of a structure of a hydrophilic group of the nonionic surfactant constituting the hydrophilising agent (D1) preferably include a polyethylene oxide type, a polyol (polyhydric alcohol) type, a block polymer type, a nitrogen-containing type and the like.

Examples of the polyethylene oxide type nonionic surfactant preferably include polyoxyethylene alkyl ether, polyoxyethylene fatty acid ester and the like.

Examples of the polyol (polyhydric alcohol) type nonionic surfactant preferably include sorbitan monoalkylate, polyoxyethylene sorbitan monoalkylate, glycerol monoalkylate, polyglyceryl monoalkylate, alkyl glucoside, pentaerythritol monoalkylate and the like.

Examples of the block polymer type nonionic surfactant preferably include alkyl ether of polyoxyethylene polyoxypropylene glycol, polyoxyethylene polyoxypropylene alkylether and the like.

Examples of the nitrogen-containing nonionic surfactant preferably include polyoxyethylene alkyl amine, alkyl polyoxyethylene fatty acid amide and the like.

Among these, it is preferable to have at least a "polyoxyethylene (hereinafter, also referred to as "POE")" group as the hydrophilic group.

The hydrophilising agent (D1) independently has solubility in water. That is, the hydrophilising agent (D1) is the surfactant, and an aqueous solution thereof has foamability and foam stability, which is used to judge the solubility in water by the following method. Specifically, the expression "independently has solubility in water" means that, when the hydrophilising agent (D1) is independently immersed into water for 20 minutes to prepare a 2.5 wt% aqueous solution of the hydrophilising agent (D1), a foaming height judged by the following measurement is 10 mm or more.

(Method for measuring foaming height after shaking aqueous solution of hydrophilising agent (D1))

First, 0.2 g of the hydrophilising agent (D1) is weighed, poured into a screw bottle (No. 4, shell diameter: 24 mm, overall length: 53 mm, manufactured by Maruem Co., Ltd.), and 8.0 g of deionised water is further poured thereinto, and then the resulting mixture is allowed to stand for 20 minutes to sufficiently dissolve the component into the deionised water to prepare an aqueous solution.

Next, the above-mentioned method described in (Properties of eliminating liquid film) is applied mutatis mutandis thereto to measure a height of a structure layer generated by shaking. Specifically, after intensively shaking the screw bottle up and down twice, the screw bottle is immediately placed on a horizontal plane. After elapse of 10 seconds from the time immediately after shaking, a height of the structure layer (height from a liquid level of the liquid layer to a top surface of the structure layer in which air is held) for the solution is measured. This height is taken as the foaming height after shaking the aqueous solution of the hydrophilising agent (D1).

The hydrophilising agent (D1) preferably contains a compound having an HLB value higher than the HLB value of the liquid film cleavage agent, from a viewpoint of enhancing the hydrophilicity on the fibre surface. The HLB value is more preferably 10 or more, further preferably 11 or more, and particularly preferably 13 or more. Moreover, the HLB value is preferably 20 or less, more preferably 17 or less, and further preferably 15 or less, from a viewpoint of preventing solubility of the agent in bodily fluid from increasing. The HLB value in the range can be obtained by appropriately setting an overall molecular weight by selecting the addition number of moles of oxyethylene in a POE chain and an alkyl chain length in the nonionic surfactant.

(Method for measuring HLB value of hydrophilising agent (D1))

The HLB can be determined by the following calculation formula.

Griffin method:

$$\text{HLB value} = 20 \times (\text{total of formula weight of hydrophilic portion})/(\text{molecular weight})$$

In addition, when the molecular structure of a target component is unknown, the above-described measurement is performed by elucidating a molecular structure and a molecular weight by such an analysis method as in the above-mentioned (Method for identifying molecular structure), and then, when necessary, obtaining the structure as the single substance as commercially available item or by chemical synthesis. A means for identifying the structure and obtaining

the structure as the single substance is the same as in other measurements in the present specification.

Moreover, the nonionic surfactant of the hydrophilising agent (D1) preferably has the properties and the structure described below, from a viewpoint of suppressing reduction of the surface tension of bodily fluid. Thus, spreadability (spreading coefficient described later) of the liquid film cleavage agent can be retained at a level as high as possible. That is, bodily fluid in contact with the nonwoven fabric of the embodiment is subjected to liquid film cleavage before the surface tension is reduced, and formation of the liquid film is inhibited.

The nonionic surfactant of the hydrophilising agent (D1) preferably contains no solvent during production, and preferably in liquid state in the component as a single substance. On the other hand, during use of the nonwoven fabric produced, the nonionic surfactant preferably has, when the nonionic surfactant is exposed to bodily fluid, the properties in which a rate of dissolution in water is delayed for the reason of (i) or (ii) described below: (i) the nonionic surfactant is gelled by being exposed to bodily fluid, or (ii) the nonionic surfactant is solidified upon being exposed to bodily fluid. According to such properties, in the hydrophilising agent (D1), the rate of dissolution in bodily fluid is reduced, and the liquid film cleavage can be induced before reducing the surface tension of bodily fluid. In addition, the term "liquid state" means that the nonionic surfactant has fluidity, and the "solidified" indicates that flowability is lost.

With regard to the temperature conditions of the hydrophilising agent (D1), when a melting point is 5°C or more in the single substance, the hydrophilising agent (D1) is easily solidified during storage of a product, and can maintain a solid state at least until the time of wearing, and therefore reduction of the rate of dissolution is easily induced upon being exposed to bodily fluid.

The nonionic surfactant of the hydrophilising agent (D1) preferably contains a compound having the structure of (D11) or (D12) described below.

(D11) A hydrocarbon-based nonionic surfactant having the foaming height of 10 mm or more after shaking the aqueous solution, and having, as the hydrophilic group, a group consisting of a POE group, or a group containing a POE group and any other polyoxyalkylene (hereinafter, also referred to as "POA") group (a polyoxypropylene (hereinafter, also referred to as "POP") group, a polyoxybutylene (hereinafter, also referred to as "POB") group or the like), and having, as a hydrophobic group, a straight-chain or a branched-chain hydrocarbon chain (excluding a polyhydric alcohol fatty acid skeleton).

(D12) A silicone-based nonionic surfactant having the foaming height of 10 mm or more after shaking the aqueous solution, and having, as the hydrophilic group, a group consisting of a POE group, or a group containing a POE group and any other POA group (a POP group, a POB group and the like), and having, as a hydrophobic group, a straight-chain or a branched-chain silicone chain.

In the (D11) and the (D12), when the hydrophilic group contains the POE group and any other POA group, it is preferable that the addition number of moles of the POE group is the largest in the hydrophilic group (it is preferable that the addition number of moles of oxyethylene is larger than the addition number of moles of any other oxyalkylene).

Moreover, in the (D11), it is preferable to satisfy conditions according to the following formula.

When the addition number of moles of oxyethylene in the POE group is taken as n, and the molecular weight is taken as X,

$$44\,n/X \geq 0.5$$

(in the formula, "0.5" means "10/20 = 0.5" when HLB 10 is used as a reference.)

In the hydrocarbon-based nonionic surfactant in the (D11), the addition number of moles of oxyethylene in the POE group that forms the hydrophilic group is preferably 5 moles or more, and more preferably 7 moles or more, from a viewpoint of enhancing the hydrophilicity. Moreover, the addition number of moles of oxyethylene in the POE group is preferably 15 moles or less, and more preferably 10 moles or less, from a viewpoint of reducing the rate of dissolution of bodily fluid. In addition thereto, the hydrocarbon chain that forms the hydrophobic group preferably has an alkyl chain length of about 10 or more and 22 or less carbon atoms. The number of carbon atoms is more preferably 10 or more, and further preferably 12 or more, from a viewpoint of reducing the rate of dissolution. Moreover, the number of carbon atoms is more preferably 22 or less, and further preferably 18 or less, from a viewpoint of easily liquefying the (D11) during coating.

Moreover, in the silicone-based nonionic surfactant in the (D12), the addition number of moles of oxyalkyl in the POA group including the POE group that forms the hydrophilic group is preferably 30 moles or more.

In all the hydrocarbon-based nonionic surfactant in the (D11) and the silicone-based nonionic surfactant in the (D12), the hydrophobic group has a straight-chain structure, whereby the surfactant is easily solidified, compared with the surfactant having a branched-chain structure, and the surface tension of bodily fluid is hard to be reduced, and such a case is preferable. More specifically, any of nonionic surfactants (D11-1) to (D12-2) described below are preferable.

The term "straight-chain" means that the structure contains no tertiary or more-branched carbon atom in an intermediate of a continuous carbon bond.

(D11-1)

A hydrocarbon-based nonionic surfactant being a straight-chain POE alkyl ether, having, as a hydrophilic group, a POE group in which the addition number of moles of oxyethylene is 5 moles or more, and having, as a hydrophobic group, a straight-chain hydrocarbon chain in which the number of carbon atoms is about 10 or more and 22 or less. The number of carbon atoms is preferably 12 or more, from a viewpoint of reducing the rate of dissolution. Moreover, the number of carbon atoms is preferably 18 or less, from a viewpoint of easily liquefying the surfactant during coating.

(D11-2)

A hydrocarbon-based nonionic surfactant having, as a hydrophilic group, a copolymer of a POP group and a POE group, in which the addition number of moles of oxyethylene in the POE group is 5 moles or more, and the addition number of moles of oxypropylene in the POP group is 3 moles or less, and having, as a hydrophobic group, a straight-chain hydrocarbon chain. The addition number of moles of oxyethylene in the POE group is more preferably 7 moles or more, from a viewpoint of enhancing the hydrophilicity. Moreover, the addition number of moles of oxyethylene in the POE group is preferably 15 moles or less, and more preferably 10 moles or less, from a viewpoint of reducing the rate of dissolution of bodily fluid.

(D12-1)

A silicone-based nonionic surfactant being a side chain-modified silicone, having a POE group in a straight-chain silicone chain.

(D12-2)

A silicone-based nonionic surfactant prepared by performing side-chain modification of a straight-chain silicone chain, in which the surfactant has, as a hydrophilic group, a copolymer in which the addition number of moles of oxyethylene in a POE group and the addition number of moles of oxypropylene in a POP group are 30 moles or more in total, and the addition number of moles of oxyethylene in the POE group is larger than the addition number of moles of oxypropylene in the POP group. A total of the addition number of moles in the POE group and the POP group is more preferably 40 moles or more, from a viewpoint of enhancing the hydrophilicity. Moreover, a total of the addition number of moles in the POE group and the POP group is preferably 100 moles or less, and more preferably 90 moles or less, from a viewpoint of adjusting viscosity to an easily handling level in a production step.

The nonionic surfactant of the hydrophilising agent (D1) is formed into the above-mentioned structure, and in particular is the hydrocarbon-based nonionic surfactant having 5 moles or more in the addition number of moles of oxyethylene in the POE group, or the silicone-based surfactant in which the addition number of moles of oxyalkylene in the POA group is 30 moles or more and the addition number of moles of oxyethylene in the POE group is larger than the addition number of moles of oxypropylene in the POP group, whereby water solubility is higher than the liquid film cleavage agent. The hydrophilising agent (D1) is the surfactant having the water solubility, which is different from the liquid film cleavage agent, whereby the aqueous solution has the foamability and the foam stability, and therefore the foaming height after shaking the aqueous solution can be adjusted to 10 mm or more. The foaming height of the hydrophilising agent (D1) is preferably 20 mm or less, more preferably 18 mm or less, and further preferably 15 mm or less, from a viewpoint of preferably suppressing reduction of the surface tension of bodily fluid.

In a compound contained in the nonionic surfactant of the hydrophilising agent (D1), a melting point is preferably 5°C or more, more preferably 10°C or more, and further preferably 15°C or more, from a viewpoint of reducing the rate of dissolution in bodily fluid. The melting point is preferably less than 25°C, more preferably 24°C or less, and further preferably 22°C or less, from a viewpoint of easily liquefying the surfactant during coating. The melting point in the range can be obtained by shortening the alkyl chain length or adjusting the addition number of moles of oxyethylene in the POE group to 12 or less in the nonionic surfactant.

The nonionic surfactant of the hydrophilising agent (D1) is further solidified as the melting point is higher, and a straight-chain molecule has a lower rate of diffusion, namely, a lower rate of dissolution, whereby the nonionic surfactant preferably has a melting point of 5°C or more, and the straight-chain hydrocarbon chain or silicone chain.

(Method for measuring melting point of hydrophilising agent (D1))

In the case of solid, a sample obtained is put in a vial, and then the vial is put in a thermostatic humidity chamber that can be visually observed from outside, and the sample is acclimatised for about 30 minutes, and then while the temperature is raised by 1°C as temperature conditions, the temperature at which the substance is melted is observed and is taken as the melting point.

In the case of liquid, a sample obtained is put in a vial, and then the vial is put in a thermostatic humidity chamber that can be visually observed from outside, temperature is decreased to -20°C, and the sample is acclimatised for about 30 minutes, and then while the temperature is raised by 1°C as temperature conditions, the temperature at which the substance is melted is observed and is taken as the melting point.

A mass average molecular weight of the nonionic surfactant of the hydrophilising agent (D1) is preferably 50 or more, more preferably 100 or more, and further preferably 200 or more, from a viewpoint of weakening irritation to skin. The mass average molecular weight is preferably 1,500 or less, more preferably 1,000 or less, and further preferably 750 or less, from a viewpoint of adjusting the melting point to a level of easily handling the surfactant in the production step. The mass average molecular weight of the nonionic surfactant of the hydrophilising agent (D1) can be measured by a liquid separation technique such as GPC and liquid chromatography mass spectrometry (LC-MS). In the case of a high molecular weight, the mass average molecular weight is preferably measured by GPC, and in the case of a low molecular weight, the mass average molecular weight is preferably measured by LC-MS. A column and a solvent can be appropriately selected. In addition, the compound is identified by the method described in (Method for identifying molecular structure) mentioned above in the preceding paragraph of the measurement of the mass average molecular weight, when necessary.

Specific examples of a material satisfying a preferable range of the melting point and the mass average molecular weight in the hydrophilising agent (D1) include a hydrocarbon-based nonionic surfactant having a melting point of 20°C or more, having, as a hydrophilic group, a POE group in which the addition number of moles of oxyethylene is 9 moles, and having, as a hydrophobic group, a lauryl group.

Next, the hydrophilising agent (D2) will be described.

The hydrophilising agent (D2) has surface tension of 42 mN/m or more. The surface tension is adjusted to be higher than the surface tension of the liquid film cleavage agent. The hydrophilising agent (D2) has such a range of the surface tension, whereby wettability on the fibre surface can be enhanced. At the same time, the surface tension of the hydrophilising agent (D2) has a value close to the surface tension of 50 mN/m that is assumed as the surface tension of bodily fluid that forms the liquid film, and therefore even if the hydrophilising agent (D2) is dissolved in bodily fluid, reduction of the surface tension of bodily fluid can be suppressed. Thus, the spreadability (spreading coefficient described later) of the liquid film cleavage agent can be retained at a level as high as possible. From this viewpoint, in the compound contained in the hydrophilising agent (D2), the surface tension is preferably 42.5 mN/m or more, more preferably 43 mN/m or more, and further preferably 44 mN/m or more. Moreover, in the compound contained in the hydrophilising agent (D2), the surface tension is preferably 60 mN/m or less, more preferably 55 mN/m or less, and further preferably 50 mN/m or less, from a viewpoint of development of the hydrophilicity.

(Method for measuring surface tension of hydrophilising agent (D2))

The surface tension of the hydrophilising agent (D2) is measured in an environmental region of a temperature of 25°C and a relative humidity (RH) of 65%, by a plate method. On the above occasion, a platinum plate (purity of 99.9%, 25 mm width and 10 mm length) and an automatic surface tensiometer "CBVP-Z" (trade name, manufactured by Kyowa Interface Science Co., Ltd.) are used.

The hydrophilising agent (D2) preferably contains a compound consisting of a structure having the hydrophilic group without a hydrophobic structure in order to be formed into a material having the above-described surface tension. The structure having a hydrophilic group is formed of a structure having a POE group or a structure having a block copolymer of a POE group and a POP group. The expression "without a hydrophobic structure" means a structure without an alkyl chain having 5 or more carbon atoms, a silicone chain or a fluorine chain.

The compound of the hydrophilising agent (D2) is preferably polyethylene glycol (PEG) composed of the POE group, or a copolymer of a POE compound and a POP compound, and more preferably PEG, polypropylene glycol (PPG), a copolymer thereof, or polypropylene diglycerol.

A mass average molecular weight of the compound of the hydrophilising agent (D2) is preferably 200 or more, more preferably 300 or more, and further preferably 400 or more, from a viewpoint of suppressing volatility. The mass average molecular weight is preferably 10,000 or less, more preferably 8,000 or less, and further preferably 6,000 or less, from a viewpoint of adjusting the viscosity to an easily coating level.

Moreover, when the compound of the hydrophilising agent (D2) contains the copolymer of the POE compound and the POP compound, a polymerisation ratio of the POE compound to the POP compound (POE/POP) in terms of a

mass ratio is preferably 3/17 or more, more preferably 4/25 or more, and further preferably 5/35 or more, from a viewpoint of suppressing the rate of dissolution in bodily fluid. The polymerisation ratio is preferably 10/65 or less, more preferably 8/50 or less, and further preferably 6/40 or less in terms of a mass ratio, from a viewpoint of enhancing the hydrophilicity.

The hydrophilising agent (D2) more preferably contains PEG having a mass average molecular weight of 400, or a compound obtained by polymerising PEG and PPG at a ratio of about 1:7 in terms of a mass ratio.

Next, a preferable aspect of the liquid film cleavage agent in the present invention will be described. The preferable aspect of the liquid film cleavage agent can include the agent described in the paragraphs {0013} to {0088} in WO 2016/098796. Specifically, the present invention can include the agents in the first aspect and the second aspect described below.

In a compound contained in the liquid film cleavage agent in the first aspect, a spreading coefficient to a liquid having surface tension of 50 mN/m is 15 mN/m or more, and water solubility is 0 g or more and 0.025 g or less. In addition, the compound having the properties is referred to as a compound C1 in several cases.

In a compound contained in the liquid film cleavage agent in the second aspect, a spreading coefficient to the liquid having surface tension of 50 mN/m is more than 0 mN/m, namely, in a positive value, water solubility is 0 g or more and 0.025 g or less, and interfacial tension to the liquid having surface tension of 50 mN/m is 20 mN/m or less. In addition, the compound having the properties is referred to as a compound C2 in several cases.

Definitions of "spreading coefficient to the liquid having surface tension of 50 mN/m" and "water solubility" of the liquid film cleavage agent, as specified in the first aspect and the second aspect, and measuring methods of surface tension ($\gamma_w$) of a liquid film (liquid having surface tension of 50 mN/m), surface tension ($\gamma_o$) of a liquid film cleavage agent, interfacial tension ($\gamma_{wo}$) between the liquid film and the liquid film cleavage agent, and water solubility of the liquid film cleavage agent are as described in the paragraphs {0015} to {0022} in WO 2016/098796.

In the compound contained in the liquid film cleavage agent in the first aspect, the spreading coefficient is 15 mN/m or more, whereby the liquid film cleavage agent has high mobility on the surface of the liquid film formed in a narrow interfibre space region, namely, high diffusivity. Further, the liquid film cleavage agent also has the spreadability moving from a region provided with the topsheet 1 to a region provided with no topsheet. From a viewpoint of effectively exhibiting these spreadability described above, the spreading coefficient of the compound contained in the liquid film cleavage agent is preferably 20 mN/m or more, more preferably 25 mN/m or more, and further preferably 30 mN/m or more. On the other hand, an upper limit thereof is not particularly limited, but from Expression (1) described in paragraph [0015] of WO 2016/098796, the surface tension of the liquid which forms the liquid film serves as the upper limit of the spreading coefficient of the liquid film cleavage agent, in such a manner that a value of the upper limit is 50 mN/m when a liquid having surface tension of 50 mN/m is used, a value of the upper limit is 60 mN/m when a liquid having surface tension of 60 mN/m is used, and a value of the upper limit is 70 mN/m when a liquid having surface tension of 70 mN/m is used. Therefore, from a viewpoint of using the liquid having surface tension of 50 mN/m in the present invention, the upper limit is 50 mN/m or less.

Moreover, in the compound contained in the liquid film cleavage agent in the first aspect, when the water solubility is 0 g or more and 0.025 g or less, the liquid film cleavage agent is hard to dissolve and forms the interface with the liquid film to make the above-described diffusivity more effective. From the same viewpoint, the water solubility of the compound contained in the liquid film cleavage agent is preferably 0.0025 g or less, more preferably 0.0017 g or less, and further preferably less than 0.0001 g. Moreover, the water solubility is preferably smaller, and is 0 g or more, and from a viewpoint of the diffusivity on the liquid film, the water solubility is practically adjusted to $1.0 \times 10^{-9}$ g or more. In addition, the water solubility is considered to be applied also to menstrual blood, urine or the like which contains water as a main component.

Further, the liquid film cleavage agent in the first aspect preferably contains a compound having the interfacial tension of 20 mN/m or less to the liquid having surface tension of 50 mN/m. It means that "interfacial tension ($\gamma_{wo}$) of the liquid film cleavage agent to the liquid film" being one variable for determining the value of the spreading coefficient (S) in Expression (1) mentioned above is preferably 20 mN/m or less. The spreading coefficient of the liquid film cleavage agent is improved by suppressing "interfacial tension ($\gamma_{wo}$) of the liquid film cleavage agent to the liquid film," and the liquid film cleavage agent easily moves from the surface of the fibres to the vicinity of the centre of the liquid film, and the above-mentioned action becomes clearer. From this viewpoint, "the interfacial tension to the liquid having surface tension of 50 mN/m" of the compound contained in the liquid film cleavage agent is more preferably 17 mN/m or less, further preferably 13 mN/m or less, still further preferably 10 mN/m or less, particularly preferably 9 mN/m or less, and especially preferably 1 mN/m or less. On the other hand, a lower limit thereof is not particularly limited, and only needs to be more than 0 mN/m from a viewpoint of insolubility into the liquid film. In addition, if the interfacial tension is 0 mN/m, that is, if the liquid film cleavage agent is soluble, the interface between the liquid film and the liquid film cleavage agent cannot be formed, and therefore Expression (1) does not hold, and spreading of the agent does not occur.

As is known from the expression, a numerical value of the spreading coefficient changes depending on the surface tension of a target liquid. For example, when the surface tension of the target liquid is 72 mN/m, the surface tension of the liquid film cleavage agent is 21 mN/m, and the interfacial tension of these is 0.2 mN/m, the spreading

coefficient becomes 50.8 mN/m.

Moreover, when the surface tension of the target liquid is 30 mN/m, the surface tension of the liquid film cleavage agent is 21 mN/m, and the interfacial tension of these is 0.2 mN/m, the spreading coefficient becomes 8.8 mN/m.

In any cases, in an agent in which the spreading coefficient is larger, a liquid film cleavage effect becomes larger.

In this description, the numerical value in the surface tension of 50 mN/m is defined. However, even if the surface tension is different, there exists no change in a magnitude relationship of the numerical value of the spreading coefficient between substances. Therefore, even if the surface tension of bodily fluid should be changed depending on a daily physical condition or the like, the agent in which the spreading coefficient is larger shows a superb liquid film cleavage effect.

The surface tension of the compound contained in the liquid film cleavage agent in the first aspect is preferably 32 mN/m or less, more preferably 30 mN/m or less, further preferably 25 mN/m or less, and particularly preferably 22 mN/m or less. Moreover, the surface tension is preferably smaller, and a lower limit thereof is not particularly limited. From a viewpoint of durability of the liquid film cleavage agent, the surface tension is practically 1 mN/m or more, and may be 20 mN/m or more.

Even when the surface tension of the target liquid which forms the liquid film is decreased, liquid film cleavage action can be effectively exhibited by adjusting the surface tension of the liquid film cleavage agent to a level equal to or less than the range as described above.

Next, in a compound contained in the liquid film cleavage agent in a second aspect, to have the "interfacial tension thereof to the liquid having surface tension of 50 mN/m" of 20 mN/m or less, as mentioned above, means that the diffusivity of the liquid film cleavage agent on the liquid film is improved. Thus, even when the spreading coefficient is comparatively small as in the case where the "spreading coefficient to the liquid having surface tension of 50 mN/m" is less than 15 mN/m, the diffusivity is high, and therefore a large amount of the liquid film cleavage agent is dispersed to the liquid film from the surface of the fibres, and the same action as the action in the case of the first aspect can be produced by thrusting away the liquid film in many positions.

From a viewpoint of further effectively exhibiting the action of the liquid film cleavage agent, the "interfacial tension to the liquid having surface tension of 50 mN/m" of the compound contained in the liquid film cleavage agent in the second aspect is preferably 17 mN/m or less, more preferably 13 mN/m or less, further preferably 10 mN/m or less, still further preferably 9 mN/m or less, and particularly preferably 1 mN/m or less. A lower limit is not particular limited in the same manner as the first aspect, and from a viewpoint of insolubility in the liquid film (the liquid having surface tension of 50 mN/m), the interfacial tension is practically adjusted to be more than 0 mN/m, and may be 3 mN/m or more.

Moreover, from a viewpoint of further effectively exhibiting the action of the liquid film cleavage agent, the "spreading coefficient to the liquid having surface tension of 50 mN/m" of the compound contained in the liquid film cleavage agent in the second aspect is preferably 9 mN/m or more, more preferably 10 mN/m or more, and further preferably 15 mN/m or more. An upper limit thereof is not particularly limited, but from a viewpoint in which the surface tension of the liquid which forms the liquid film serves as the upper limit from Expression (1), the spreading coefficient is substantially 50 mN/m or less.

Further preferable ranges of the surface tension and the water solubility of the compound contained in the liquid film cleavage agent in the second aspect are the same with the ranges in the first aspect.

As described above, the compound contained in the liquid film cleavage agent in the first aspect has the spreading coefficient and the water solubility described above, and the compound contained in the liquid film cleavage agent in the second aspect has the spreading coefficient, the interfacial tension and the water solubility described above, whereby the compounds can spread on the surface of the liquid film without being dissolved, and can thrust away a layer of the liquid film from the vicinity of the centre of the liquid film. Thus, the liquid film is destabilised and cleaved.

The topsheet 1 containing the liquid film cleavage agent in the first aspect and the topsheet 1 containing the liquid film cleavage agent in the second aspect each preferably further arrange a phosphoric acid ester type anionic surfactant. Thus, hydrophilicity on the surface of the fibres is improved and wettability is improved to increase a contact area in which the liquid film and the liquid film cleavage agent are brought into contact. Further, because blood and urine contain a surface active substance having a phosphoric acid group which originates in a living body, when the surfactant having the phosphoric acid group is used together with the liquid film cleavage agent, the surfactant has a compatibility with the liquid film cleavage agent, and a fine affinity for phospholipid contained in blood and urine. Thereby, the liquid film cleavage agent easily moves onto the liquid film, and cleavage of the liquid film is further induced. An arrangement ratio of the total amount of the liquid film cleavage agent to the total amount of the phosphoric acid ester type anionic surfactant is preferably 1:1 to 19:1, more preferably 2:1 to 15:1, and further preferably 3:1 to 10:1 in terms of a mass ratio (liquid film cleavage agent: phosphoric acid ester type anionic surfactant). In particular, the arrangement ratio is preferably 5:1 to 19:1, more preferably 8:1 to 16:1, and further preferably 11:1 to 13:1 in terms of a mass ratio.

The phosphoric acid ester type anionic surfactant can be used without particular restriction. For example, the materials described in the paragraph {0031} in WO 2016/098796 and the like can be contained.

Next, specific examples of the compound contained in the liquid film cleavage agents in the first aspect and

the second aspect will be described. These are in the above-mentioned specific numerical value range to have properties of being insoluble in water or hardly soluble in water, and exhibit the liquid film cleavage action. In contrast, the surfactant or the like to be used as the conventional fibre treating agent is basically a water-soluble agent which is practically dissolved in water and used, and is not the liquid film cleavage agent according to the present invention.

The liquid film cleavage agent in the first aspect and the second aspect preferably contains a compound having a mass average molecular weight of 500 or more. The mass average molecular weight greatly influences viscosity of the liquid film cleavage agent. The liquid film cleavage agent is hard to flow down when the liquid passes through an interfibre place by keeping high viscosity, and sustainability of the liquid film cleavage effect in the nonwoven fabric can be kept. From a viewpoint of adjusting the viscosity to a level at which the liquid film cleavage effect is sufficiently sustained, the mass average molecular weight of the compound contained in the liquid film cleavage agent is more preferably 1,000 or more, further preferably 1,500 or more, and particularly preferably 2,000 or more. On the other hand, from a viewpoint of adjusting the viscosity to a level at which migration of the liquid film cleavage agent from the fibres having the liquid film cleavage agent arranged therein to the liquid film, namely the macro diffusivity and the micro diffusivity are held, the mass average molecular weight thereof is preferably 50,000 or less, more preferably 20,000 or less, and further preferably 10,000 or less. Measurement of the mass average molecular weight is performed by using GPC "CCPD" (trade name, manufactured by TOSOH CORPORATION). Measurement conditions are as described below. Moreover, calculation of equivalent molecular weight is performed by polystyrene.

Separation column: GMHHR-H+GMHHR-H (cation)
Eluent: L FAMIN DM20/$CHCl_3$
Solvent flow rate: 1.0 mL/min
Separation column temperature: 40°C

Moreover, as mentioned below, the liquid film cleavage agent in the first aspect preferably contains a compound having at least one kind structure selected from the group consisting of the following structures X, X-Y and Y-X-Y.

The structure X designates a siloxane chain having a structure in which any of basic structures of >C(A)- (C designates a carbon atom, moreover, <, > and - each designate a bonding hand, hereinafter, the same applies.), $-C(A)_2-$, -C(A)(B)-, >C(A)-C($R^1$)<, >C($R^1$)-, $-C(R^1)(R^2)-$, $-C(R^1)_2-$, >C<, $-Si(R^1)_2O-$ and $-Si(R^1)(R^2)O-$ is repeated, or two or more kinds thereof are combined; or a mixed chain thereof. The structure X has, in an end of the structure X, a hydrogen atom or at least one kind of group selected from the group consisting of $-C(A)_3$, $-C(A)_2B$, $-C(A)(B)_2$, $-C(A)_2-C(R^1)_3$, $-C(R^1)_2A$, $-C(R^1)_3$, $-OSi(R^1)_3$, $-OSi(R^1)_2(R^2)$, $-Si(R^1)_3$ and $-Si(R^1)_2(R^2)$.

The above-described $R^1$ and $R^2$ each independently designate various substituents such as a hydrogen atom, an alkyl group (the number of carbon atoms is preferably 1 to 20, for example, a methyl group, an ethyl group or a propyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.) and a halogen atom (for example, a fluorine atom is preferable.). A and B each independently designate a substituent including an oxygen atom or a nitrogen atom, such as a hydroxy group, a carboxylic acid group, an amino group, an amide group, an imino group and a phenol group. When a plurality of $R^1$, $R^2$, A and B exists for each in the structure X, these may be identical to or different from each other. Moreover, a continuous inter-C (carbon atoms) or inter-Si bonding is ordinarily a single bond, but may include a double bond or a triple bond, and the inter-C or inter-Si bonding may include a linking group such as an ether group (-O-), an amide group ($-CONR^A-$: $R^A$ is a hydrogen atom or a monovalent group), an ester group (-COO-), a carbonyl group (-CO-) or a carbonate group (-OCOO-). The number of bonding of one C and one Si with any other C or Si is 1 to 4, and a long-chain silicone chain (siloxane chain) or a mixed chain may be branched or may have a radial structure.

Y designates a hydrophilic group having hydrophilicity, the group containing an atom selected from a hydrogen atom, a carbon atom, an oxygen atom, a nitrogen atom, a phosphorus atom and a sulphur atom. Specific examples thereof include a hydrophilic group alone, such as a hydroxy group, a carboxylic acid group, an amino group, an amide group, an imino group, a phenol group, a POA group (the number of carbon atoms of an oxyalkylene group is preferably 1 to 4, for example, a POE group, and a POP group are preferable.), a sulphonic acid group, a sulphate group, a phosphoric acid group, a sulphobetaine group, a carbobetaine group, a phosphobetaine group (the betaine group means a betaine residual group formed by removing one hydrogen atom from each betaine compound.) and a quaternary ammonium group; or a hydrophilic group formed from a combination thereof. In addition thereto, specific examples thereof also include a group and a functional group listed in $M^1$ as mentioned below. In addition, when a plurality of Y exists, these groups may be identical to or different from each other.

In the structures X-Y and Y-X-Y, Y is bonded with X or a group at an end of X. When Y is bonded with the group at the end of X, for example, the group at the end of X is bonded with Y after hydrogen atoms and the like in the number identical with the number of bonding with Y are eliminated.

In this structure, the above-mentioned spreading coefficient, water solubility and interfacial tension can be

satisfied by selecting the hydrophilic groups Y, A and B from the groups specifically described. Thus, an objective liquid film cleavage effect is developed.

The above-described liquid film cleavage agent preferably contains a compound in which the structure X is a siloxane structure. Further, as specific examples of the above-described structures X, X-Y, and Y-X-Y, the liquid film cleavage agent preferably contains a compound composed of a siloxane chain in which structures represented by any of the following Formulas (1) to (11) are arbitrarily combined. Furthermore, from a viewpoint of the liquid film cleavage action, it is preferable that the compound has a mass average molecular weight in the range mentioned above.

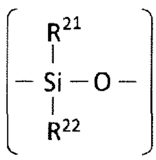 ••• (1) 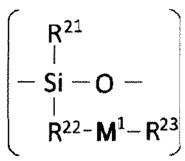 ••• (5) 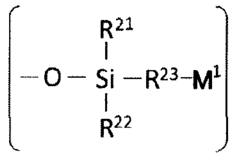 ••• (9)

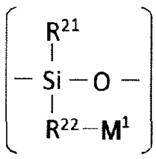 ••• (2) 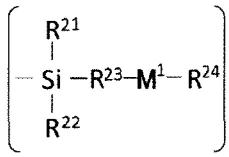 ••• (6) 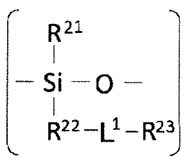 ••• (10)

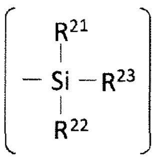 ••• (3) 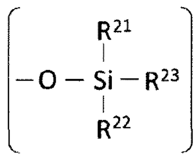 ••• (7) 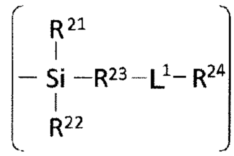 ••• (11)

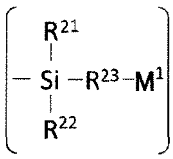 ••• (4) 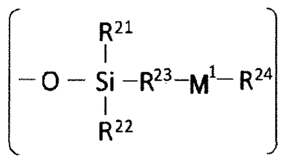 ••• (8)

In Formulas (1) to (11), $M^1$, $L^1$, $R^{21}$ and $R^{22}$ designate the following monovalent or polyvalent (divalent or more valent) group. $R^{23}$ and $R^{24}$ designate the following monovalent or polyvalent (divalent or more valent) group or a single bond.

$M^1$ designates a POE group, a POP group, a POB group, a group having a POA group in combination therewith, an erythritol group, a xylitol group, a sorbitol group, a hydrophilic group having a plurality of hydroxy groups such as a glycerol group or an ethylene glycol group (a hydrophilic group formed by removing one hydrogen atom from the above-described compound having a plurality of hydroxy groups such as erythritol), a hydroxy group, a carboxylic acid group, a mercapto group, an alkoxy group (the number of carbon atoms is preferably 1 to 20, for example, a methoxy group is preferable.), an amino group, an amide group, an imino group, a phenol group, a sulphonic acid group, a quaternary ammonium group, a sulphobetaine group, a hydroxysulphobetaine group, a phosphobetaine group, an imidazolium betaine group, a carbobetaine group, an epoxy group, a carbinol group, a (meth)acrylic group or a functional group in combination therewith. In addition, when $M^1$ is a polyvalent group, $M^1$ designates a group formed by further removing one or more hydrogen atoms from each of the groups or the functional group as mentioned above.

$L^1$ designates a linking group of an ether group, an amino group (the amino group adoptable as $L^1$ is represented by $>NR^C$ ($R^C$ is a hydrogen atom or a monovalent group).), an amide group, an ester group, a carbonyl group or a carbonate group.

$R^{21}$, $R^{22}$, $R^{23}$ and $R^{24}$ each independently designate an alkyl group (the number of carbon atoms is preferably 1 to 20, for example, a methyl group, an ethyl group, a propyl group, an isopropyl group, a butyl group, a pentyl group, a hexyl group, a heptyl group, 2-ethylhexyl group, a nonyl group or a decyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.), a fluoroalkyl group, an aralkyl group, a hydrocarbon group in combination therewith, or a halogen atom (for example, a fluorine atom is

preferable.). In addition, when $R^{22}$ and $R^{23}$ are a polyvalent group, $R^{22}$ and $R^{23}$ designate a polyvalent hydrocarbon group formed by further removing one or more hydrogen atoms or fluorine atoms from the above-described hydrocarbon group.

Moreover, when $R^{22}$ or $R^{23}$ is bonded with $M^1$, specific examples of a group adoptable as $R^{22}$ or $R^{23}$ include, in addition to each of the groups, the hydrocarbon group or the halogen atom described above, an imino group adoptable as $R^{32}$.

Above all, the liquid film cleavage agent preferably contains a compound having the structure represented by any of Formulas (1), (2), (5) and (10) as X, and contains a compound having the structure represented by any of the above-described formulas other than these formulas as a group formed of an end of X or formed of the end of X and Y. Further, a group formed of X or formed of the end of X and Y preferably contains a compound composed of a siloxane chain having at least one structure represented by any of the above-described Formulas (2), (4), (5), (6), (8) and (9).

Specific examples of the above-described compound include organic-modified silicone (polysiloxane) of a silicone-based surfactant. Specific examples of organic-modified silicone being modified with a reactive organic group include amino-modified silicone, epoxy-modified silicone, carboxy-modified silicone, diol-modified silicone, carbinol-modified silicone, (meth)acrylic-modified silicone, mercapto-modified silicone and phenol-modified silicone. Moreover, specific examples of organic-modified silicone being modified with a nonreactive organic group include polyether-modified silicone (including POA-modified silicone), methylstyryl-modified silicone, long-chain alkyl-modified silicone, higher fatty acid ester-modified silicone, higher alkoxy-modified silicone, higher fatty acid-modified silicone and fluorine-modified silicone. The spreading coefficient at which the above-described liquid film cleavage action is produced can be obtained by appropriately changing a molecular weight of a silicone chain, a modification ratio, the addition number of moles of a modifying group, or the like in corresponding to kinds of the organic-modified silicone, for example. The term "long chain" herein means a material in which the number of carbon atoms is 12 or more, and preferably 12 to 20. Moreover, the term "higher" means a material in which the number of carbon atoms is 6 or more, and preferably 6 to 20.

Above all, modified silicone having a structure in which the liquid film cleavage agent being the modified silicone has at least one oxygen atom in a modifying group, such as POA-modified silicone, epoxy-modified silicone, carbinol-modified silicone and diol-modified silicone, is preferable, and POA-modified silicone is particularly preferable. POA-modified silicone is hard to be permeated into the fibres, and easy to remain on the surface thereof because POA-modified silicone has a polysiloxane chain. Moreover, the affinity with water is improved, and the interfacial tension is small by adding a hydrophilic POA chain, and therefore move onto a surface of the liquid film as mentioned above is easily induced, and such a case is preferable. Moreover, even if thermal fusion processing such as embossing is applied, POA-modified silicone easily remains on the surface of the fibres in the part, and the liquid film cleavage action is hardly reduced. The liquid film cleavage action is sufficiently developed particularly in an embossed part in which the liquid is easy to accumulate, and such a case is preferable.

Specific examples of the POA-modified silicone include compounds represented by any of the following Formulas [I] to [IV]. Further, the POA-modified silicone preferably has the mass average molecular weight in the above-mentioned range from a viewpoint of the liquid film cleavage action.

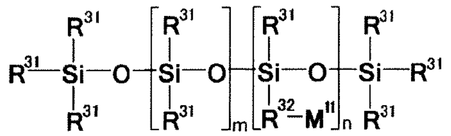

[I]

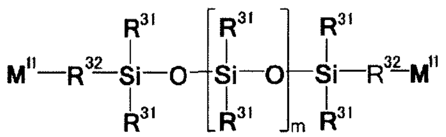

[II]

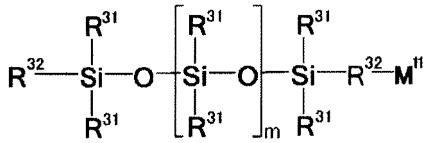

[III]

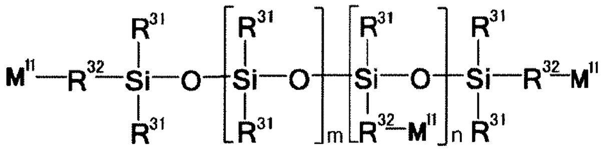

[IV]

In the formulas, $R^{31}$ designates an alkyl group (the number of carbon atoms is preferably 1 to 20, for example, a methyl group, an ethyl group, a propyl group, an isopropyl group, a butyl group, a pentyl group, a hexyl group, a heptyl group, a 2-ethyl-hexyl group, a nonyl group or a decyl group is preferable.). $R^{32}$ designates a single bond or an alkylene group (the number of carbon atoms is preferably 1 to 20, for example, a methylene group, an ethylene group, a propylene group or a butylene group is preferable.), and preferably designates the alkylene group. A plurality of $R^{31}$ and a plurality of $R^{32}$ may be each identical to or different from each other. $M^{11}$ designates a group having a POA group, and the POA group is preferable. Specific examples of the above-described POA group include a POE group, a POP group, a POB group or a material in which constituent monomers thereof are copolymerised. Then, m and n are each independently an integer of 1 or more. In addition, signs of these repeating units are separately determined in each of Formulas [I] to [IV], and do not always represent an identical integer, and may be different from each other.

Moreover, POA-modified silicone may have either or both of modifying groups of POE modification and POP modification. Moreover, the modified silicone preferably has a methyl group in $R^{31}$ as an alkyl group of a silicone chain in order to have insolubility in water and low interfacial tension. A material having this modifying group or the silicone chain is not particularly limited, but materials described in the paragraphs {0006} and {0012} in JP-A-2002-161474 are exemplified. Further specific examples thereof include POE·POP-modified silicone, POE-modified silicone and POP-modified silicone. Specific examples of the POE-modified silicone include POE (3)-modified dimethyl silicone to which 3 moles of POE are added. Specific examples of the POP-modified silicone include POP (10)-modified dimethyl silicone, POP (12)-modified dimethyl silicone and POP (24)-modified dimethyl silicone, to which 10 moles of POP, 12 moles of POP and 24 moles of POP are added, respectively.

The spreading coefficient and the water solubility of the compound contained in the above-mentioned first aspect can be adjusted in predetermined ranges, for example, in the POA-modified silicone, by the addition number of moles of POA groups (the number of bonding oxyalkylene groups which form a POA group based on 1 mole of POA-modified silicone), the following modification ratio or the like. In this compound contained in the liquid film cleavage agent, the surface tension and the interfacial tension can be adjusted to predetermined ranges in the same manner, respectively.

From the above-described viewpoint, the addition number of moles of the POA groups is preferably 1 or more. The number of moles is adjusted to a value equal to or more than the lower limit, whereby the interfacial tension is reduced for the above-described liquid film cleavage action, and the spreading coefficient increases, and therefore a sufficient liquid film cleavage effect can be obtained. Moreover, from the same viewpoint, the addition number of moles is more preferably 3 or more, and further preferably 5 or more. On the other hand, the addition number of moles is preferably 30 or less, more preferably 20 or less, and further preferably 10 or less. The addition number of moles is adjusted to a value equal to or less than the upper limit, whereby the POA-modified silicone becomes hydrophobic, and therefore the water solubility can be kept at a low level.

The modification ratio of the modified silicone is preferably 5% or more, more preferably 10% or more, and further preferably 20% or more, in order to retain the hydrophilicity necessary for the liquid film cleavage action (in particular, the spreadability). Moreover, the modification ratio is preferably 95% or less, more preferably 70% or less, and further preferably 40% or less, in order to keep the water insolubility necessary for the liquid film cleavage action. In addition, the modification ratio of the modified silicone means a proportion of the number of repeating units of a modified siloxane bonding portion based on the total number of repeating units of a siloxane bonding portion in one molecule of the modified silicone. For example, the modification ratio is expressed by the expression: $(n/m + n) \times 100\%$ in Formulas [I] and [IV], the expression: $(2/m) \times 100\%$ in Formula [II], and the expression: $(1/m) \times 100\%$ in Formula [III].

Moreover, the spreading coefficient and the water solubility mentioned above each can be set in a predetermined range, in addition to the material described above, in POA-modified silicone, by simultaneously using a water-soluble POE group, and a water-insoluble POP group and a water-insoluble POB group as a modifying group, by changing a molecular weight of a water-insoluble silicone chain; introducing an amino group, an epoxy group, a carboxy group, a hydroxy group, a carbinol group or the like thereinto in addition to POA modification as the modifying group; or the like.

Polyalkylene-modified silicone that can be used as the liquid film cleavage agent is preferably contained in 0.02 mass% or more and 5 mass% or less in terms of an arrangement proportion (OPU) to fibre mass. The arrangement proportion (OPU) of the polyalkylene modified silicone is more preferably 1 mass% or less, and further preferably 0.4 mass% or less. Thus, a nonwoven fabric has preferable texture. Moreover, from a viewpoint of effectively exhibiting the liquid film cleavage effect by the polyalkylene-modified silicone, the arrangement proportion (OPU) is more preferably 0.04 mass% or more, and further preferably 0.1 mass% or more. In this case, the liquid film cleavage agent may contain

a compound other than the polyalkylene-modified silicone. A total amount of the liquid film cleavage agent in the case is preferably within the above-mentioned range.

In addition, the fibre mass herein means fibre mass of the nonwoven fabric as a whole (the same applies also in an arrangement proportion (OPU) described below.) containing the arrangement portion 6 and the non-arrangement portion 7.

As mentioned later, the liquid film cleavage agent in the second aspect preferably contains a compound having at least one kind structure selected from the group consisting of the following structures Z, Z-Y and Y-Z-Y.

The structure Z designates a hydrocarbon chain having a structure in which any of basic structures of >C(A)- (C: carbon atom), $-C(A)_2-$, -C(A)(B)-, $>C(A)-C(R^3)<$, $>C(R^3)-$, $-C(R^3)(R^4)-$, $-C(R^3)_2-$ and >C< is repeated, or two or more kinds thereof are combined. The structure Z has, at an end thereof, a hydrogen atom or at least one kind of group selected from the group consisting of $-C(A)_3$, $-C(A)_2B$, $-C(A)(B)_2$, $-C(A)_2-C(R^3)_3$, $-C(R^3)_2A$ and $-C(R^3)_3$.

The above-described $R^3$ and $R^4$ each independently designate various kinds of substituents such as a hydrogen atom, an alkyl group (the number of carbon atoms is preferably 1 to 20, for example, a methyl group, an ethyl group, a propyl group, an isopropyl group, a butyl group, a pentyl group, a hexyl group, a heptyl group, a 2-ethyl-hexyl group, a nonyl group or a decyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.), a fluoroalkyl group, or an aralkyl group, or a hydrocarbon group in combination therewith, or a fluorine atom. A and B each independently designate a substituent containing an oxygen atom or a nitrogen atom, such as a hydroxy group, a carboxylic acid group, an amino group, an amide group, an imino group or a phenol group. When a plurality of $R^3$, $R^4$, A or B is each included in the structure Z, these may be identical to or different from each other. Moreover, a continuous inter-C (carbon atoms) bonding is ordinarily a single bond, but may include a double bond or a triple bond, and the inter-C bonding may include a linking group such as an ether group, an amide group, an ester group, a carbonyl group or a carbonate group. The number of bonding of one C with any other C is 1 to 4, and a long-chain hydrocarbon chain may have a branched structure or may have a radial structure.

Y designates a hydrophilic group having hydrophilicity, the hydrophilic group containing an atom selected from a hydrogen atom, a carbon atom, an oxygen atom, a nitrogen atom, a phosphorus atom and a sulphur atom. Specific examples thereof include: a hydroxy group, a carboxylic acid group, an amino group, an amide group, an imino group and a phenol group; a POA group (the number of carbon atoms of an oxyalkylene group is preferably 1 to 4, for example, a POE group, a POP group, a POB group, or a POA group in combination therewith is preferable.); a hydrophilic group having a plurality of hydroxy groups, such as an erythritol group, a xylitol group, a sorbitol group, a glycerol group and an ethylene glycol group; a hydrophilic group alone, such as a sulphonic acid group, a sulphate group, a phosphoric acid group, a sulphobetaine group, a carbobetaine group, a phosphobetaine group, a quaternary ammonium group, an imidazolium betaine group, an epoxy group, a carbinol group and a methacrylic group; or a hydrophilic group formed of a combination thereof. In addition, when Y is plural, the Ys may be identical to or different from each other.

In the structures Z-Y and Y-Z-Y, Y is bonded with Z or a group at an end of Z. When Y is bonded with the group at the end of Z, the group at the end of Z is bonded with Y, for example, after hydrogen atoms and the like in the number identical with the number of bonding with Y are eliminated.

In this structure, the spreading coefficient, the water solubility and the interfacial tension mentioned above can be satisfied by selecting the hydrophilic groups Y, A and B from the groups specifically described. Thus, an objective liquid film cleavage effect is developed.

The compound contained in the liquid film cleavage agent is preferably a compound obtained by arbitrarily combining structures represented by any of the following Formulas (12) to (25) as specific examples of the structures Z, Z-Y and Y-Z-Y. Further, from a viewpoint of the liquid film cleavage action, it is preferable that this compound has a mass average molecular weight in the above-mentioned range.

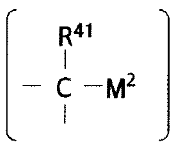 ••• (12) 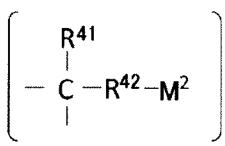 ••• (13) 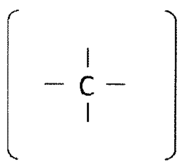 ••• (14)

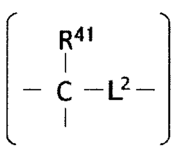 ••• (15) 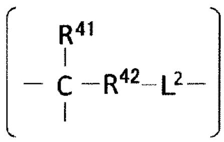 ••• (16) 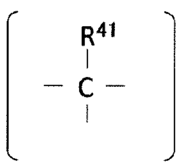 ••• (17)

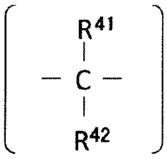 ··· (18)

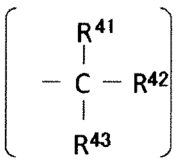 ··· (19)

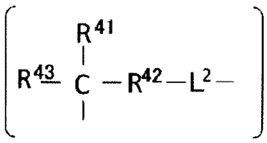 ··· (20)

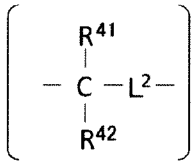 ··· (21)

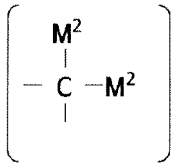 ··· (22)

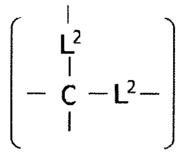 ··· (23)

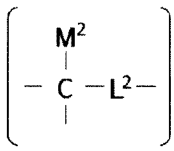 ··· (24)

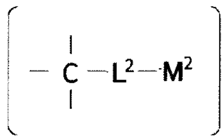 ··· (25)

In Formulas (12) to (25), $M^2$, $L^2$, $R^{41}$, $R^{42}$ and $R^{43}$ designate the following monovalent or polyvalent (divalent or more valent) group.

$M^2$ designates a POE group, a POP group, a POB group, a group having a POA group in combination therewith, an erythritol group, a xylitol group, a sorbitol group, a hydrophilic group having a plurality of hydroxy groups such as a glycerol group or an ethylene glycol group, a hydroxy group, a carboxylic acid group, a mercapto group, an alkoxy group (the number of carbon atoms is preferably 1 to 20, for example, a methoxy group is preferable.), an amino group, an amide group, an imino group, a phenol group, a sulphonic acid group, a quaternary ammonium group, a sulphobetaine group, a hydroxysulphobetaine group, a phosphobetaine group, an imidazolium betaine group, a carbobetaine group, an epoxy group, a carbinol group, a (meth)acrylic group or a functional group in combination therewith.

$L^2$ designates a linking group such as an ether group, an amino group, an amide group, an ester group, a carbonyl group, a carbonate group, a POE group, a POP group, a POB group, or a POA group in combination therewith.

$R^{41}$, $R^{42}$ and $R^{43}$ each independently designate various substituents such as a hydrogen atom, an alkyl group (the number of carbon atoms is preferably 1 to 20, for example, a methyl group, an ethyl group, a propyl group, an isopropyl group, a butyl group, a pentyl group, a hexyl group, a heptyl group, 2-ethyl-hexyl group, a nonyl group or a decyl group is preferable.), an alkoxy group (the number of carbon atoms is preferably 1 to 20, for example, a methoxy group or an ethoxy group is preferable.), an aryl group (the number of carbon atoms is preferably 6 to 20, for example, a phenyl group is preferable.), a fluoroalkyl group, an aralkyl group, a hydrocarbon group in combination therewith, or a halogen atom (for example, a fluorine atom is preferable.).

When $R^{42}$ is a polyvalent group, $R^{42}$ designates a group formed by further removing one or more hydrogen atoms from the above-described each substituent.

In addition, at an end of the bonding hand described in each structure, any other structure may be arbitrarily linked, or a hydrogen atom may be introduced.

Further, specific examples of the above-described compounds include the following compounds, but are not limited thereto.

First, examples thereof include a polyether compound and a nonionic surfactant. Specific examples thereof include POA alkyl ether represented by any of formulas in Formula [V]; POA glycol which is represented by Formula [VI] and has a mass average molecular weight of 1,000 or more, Steareth, Beheneth, PPG myristyl ether, PPG stearyl ether and PPG behenyl ether. As the POA alkyl ether, lauryl ether to which POP is added in 3 moles or more and 24 moles or less, and preferably in 5 moles, or the like is preferable. As the polyether compound, PPG having a mass average molecular weight of 1,000 to 10,000 and preferably 3,000 to which PPG is added in 17 moles or more and 180 moles or less, and preferably in about 50 moles or the like is preferable. In addition, the measurement of the mass average molecular weight can be performed by the above-mentioned measuring method.

The polyether compound or nonionic surfactant is preferably arranged in 0.1 mass% or more and 5 mass% or less in terms of an arrangement proportion (OPU) to fibre mass. The arrangement proportion (OPU) of the polyether compound or the nonionic surfactant is more preferably 1 mass% or less, and further preferably 0.4 mass% or less. Thus, a nonwoven fabric has preferable texture. Moreover, from a viewpoint of effectively exhibiting the liquid film cleavage effect by the polyether compound or the nonionic surfactant, the arrangement proportion (OPU) is more preferably 0.15 mass% or more, and further preferably 0.2 mass% or more.

$$C_mH_n-L^{21}-\left(C_aH_bO\right)_m-L^{21}-R^{51}$$

or

$$C_mH_n-L^{21}-\left(C_aH_bO\right)_m-R^{51} \qquad [V]$$

or

$$C_mH_n-L^{21}-\left(C_aH_bO\right)_m-H$$

$$R^{51}-\left(C_aH_bO\right)_m-R^{51} \qquad [VI]$$

In the formulas, $L^{21}$ designates a linking group such as an ether group, an amino group, an amide group, an ester group, a carbonyl group, a carbonate group, a POE group, a POP group, or a POB group, or a POA group in combination therewith. $R^{51}$ designate various substituents such as a hydrogen atom, a methyl group, an ethyl group, a propyl group, an isopropyl group, a butyl group, a pentyl group, a hexyl group, a heptyl group, 2-ethyl-hexyl group, a nonyl group, a decyl group, a methoxy group, an ethoxy group, a phenyl group, a fluoroalkyl group, an aralkyl group, a hydrocarbon group in combination therewith, or a fluorine atom. Moreover, a, b, m and n each are independently an integer of 1 or more. $C_mH_n$ herein designates an alkyl group (n = 2m + 1), and $C_aH_b$ designates an alkylene group (a = 2b). In addition, the number of carbon atoms and the number of hydrogen atoms are each independently determined in each of Formulas [V] and [VI], and do not always represent an identical integer and may be different from each other. Hereinafter, a same rule applies also to m, m', m", n, n' and n" in Formulas [VII] to [XV]. In addition, "m" in $-(C_aH_bO)_m-$ is an integer of 1 or more. Values of the repeating units are each independently determined in each of Formulas [V] and [VI], and do not always represent an identical integer and may be different from each other.

The spreading coefficient, the surface tension and the water solubility described above in the second aspect each can be set in a predetermined range, in the polyether compound or the nonionic surfactant, for example, by the number of moles of a POA group, or the like. From this viewpoint, the number of moles of the POA group is preferably 1 or more and 70 or less. At the number 1 or more, the above-described liquid film cleavage action is effectively exhibited. From this viewpoint, the number of moles is more preferably 5 or more, and further preferably 7 or more. On the other hand, the addition number of moles is preferably 70 or less, more preferably 60 or less, and further preferably 50 or less. Thus, entanglement of molecular chains is moderately weakened and the agent is excellent in the diffusivity in the liquid film, and such a case is preferable.

Moreover, the spreading coefficient, the surface tension, interface tension and the water solubility described above each can be set in a predetermined range, in the polyether compound or the nonionic surfactant, by simultaneously using a water-soluble POE group and a water-insoluble POP group and a water-insoluble POB group, by changing a chain length of a hydrocarbon chain, by using a material having a branched chain in a hydrocarbon chain, by using a material having a double bond in a hydrocarbon chain, by using a material having a benzene ring or a naphthalene ring in a hydrocarbon chain, by appropriately combining the above, or the like.

Second, examples include a hydrocarbon compound having 5 or more carbon atoms. From a viewpoint in which spreading on the surface of the liquid film is further enhanced in a state of fluid, the number of carbon atoms is preferably 100 or less, and more preferably 50 or less. The hydrocarbon compound, excluding polyorganosiloxane, is not limited to a straight chain, and may have a branched chain, in which the chain is not particularly limited to a saturated chain or an unsaturated chain. Moreover, the hydrocarbon compound may have a substituent such as ester and ether in an intermediate and an end thereof. Above all, the hydrocarbon compound in fluid at ordinary temperature is preferable and used alone. The hydrocarbon compound is preferably arranged in 0.1 mass% or more and 5 mass% or less in terms of an arrangement proportion (OPU) to fibre mass. The arrangement proportion (OPU) of the hydrocarbon compound is preferably 1 mass% or less, more preferably 0.99 mass% or less, and further preferably 0.4 mass% or less. Thus, a nonwoven fabric has preferable texture. Moreover, from a viewpoint of effectively exhibiting the liquid film cleavage effect by the arrangement proportion of the hydrocarbon compound, the arrangement proportion (OPU) is more preferably 0.15 mass% or more, and further preferably 0.2 mass% or more.

Examples of the hydrocarbon compound include oil or fat, such as natural oil or natural fat. Specific examples thereof include palm oil, camellia oil, castor oil, coconut oil, corn oil, olive oil, sunflower oil, tall oil, and a mixture thereof.

Moreover, specific examples thereof include fatty acid as represented by Formula [VII], such as caprylic acid, capric acid, oleic acid, lauric acid, palmitic acid, stearic acid, myristic acid, behenic acid, and a mixture thereof.

$$C_mH_n - COOH \qquad [VII]$$

In Formula [VII], m and n each independently are an integer of 1 or more. $C_mH_n$ herein designates a hydrocarbon group of the above-described fatty acid.

Examples of straight-chain or branched-chain, saturated or unsaturated, or substituted or unsubstituted polyhydric alcohol fatty acid ester or a mixture of polyhydric alcohol fatty acid ester include glycerol fatty acid ester or pentaerythritol fatty acid ester as represented by Formula [VIII-I] or [VIII-II], and specific examples thereof include glyceryl tricaprylate, glyceryl tripalmitate and a mixture thereof. In addition, a certain amount of monoester, diester and trimester is typically included in the mixture of glycerol fatty acid ester or pentaerythritol fatty acid ester. Specific preferable examples of the glycerol fatty acid ester include a mixture of glyceryl tricaprylate and glyceryl tricapryate. Moreover, from a viewpoint of reducing the interfacial tension to obtain a higher spreading coefficient, polyhydric alcohol fatty acid ester into which a POA group is introduced in a degree at which water insolubility can be maintained may be used.

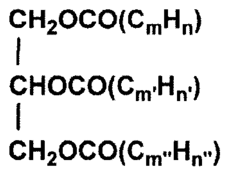

[VIII-I]

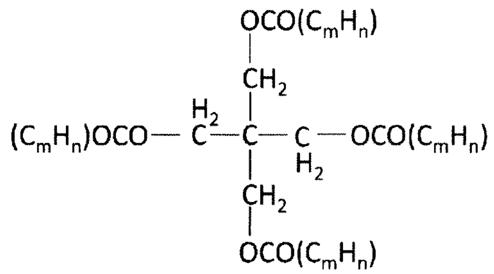

[VIII-II]

In Formulas [VIII-I] and [VIII-II], m, m', m", n, n' and n" each are independently an integer of 1 or more. A plurality of m or a plurality of n each may be identical to or different from each other. $C_mH_n$, $C_m'H_n'$ and $C_m"H_n"$ each herein designate a hydrocarbon group of the above-described fatty acid.

Examples of the fatty acid or a fatty acid mixture in which the straight-chain or branched-chain, or the saturated or unsaturated fatty acid forms ester with polyol having a large number of hydroxy groups, and a part of hydroxy groups remain without being esterified, include a partially esterified product of glycerol fatty acid ester, sorbitan fatty acid ester or pentaerythritol fatty acid ester as represented by any of formulas in Formula [IX], any of formulas in Formula [X] or any of formulas in Formula [XI]. Specific examples thereof include ethylene glycol monomyristate, ethylene glycol dimyristate, ethylene glycol palmitate, ethylene glycol dipalmitate, glyceryl dimyristate, glyceryl dipalmitate, glyceryl monooleate, sorbitan monooleate, sorbitan monostearate, sorbitan dioleate, sorbitan tristearyl, pentaerythritol monostearate, pentaerythritol dilaurate, pentaerythritol tristearate, and a mixture thereof. In addition, a certain quantity of a completely esterified compound is typically included in the mixture formed of the partially esterified product of glycerol fatty acid ester, sorbitan fatty acid ester, pentaerythritol fatty acid ester or the like.

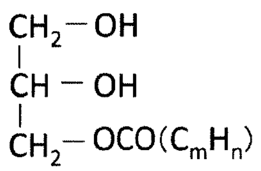

or

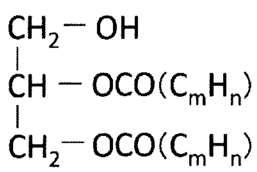

[IX]

In Formula [IX], m and n each are independently an integer of 1 or more. A plurality of m or a plurality of n each may be identical to or different from each other. $C_mH_n$ herein designates a hydrocarbon group of the above-described

fatty acid.

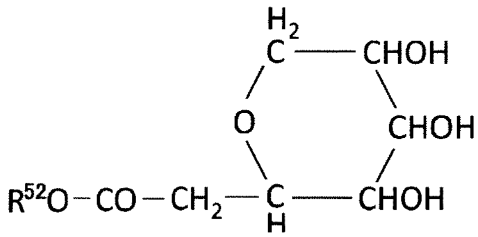

or

[X]

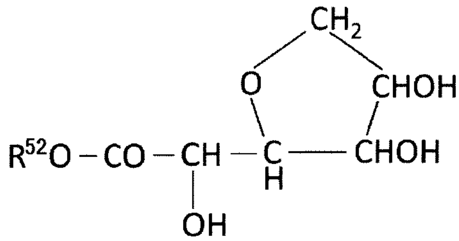

In Formula [X], $R^{52}$ designates a straight-chain or branched-chain, or saturated or unsaturated hydrocarbon group (an alkyl group, an alkenyl group, an alkynyl group or the like) having 2 or more and 22 or less carbon atoms. Specific examples thereof include a 2-ethyl-hexyl group, a lauryl group, a myristyl group, a palmityl group, a stearyl group, a behenyl group, an oleyl group and a linoleic group.

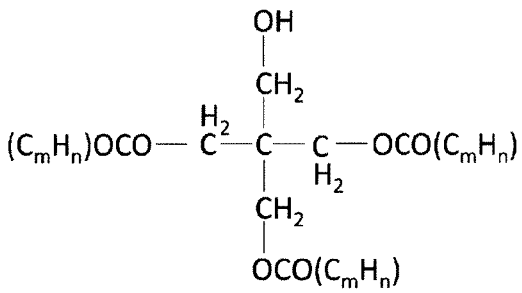

[XI]

or

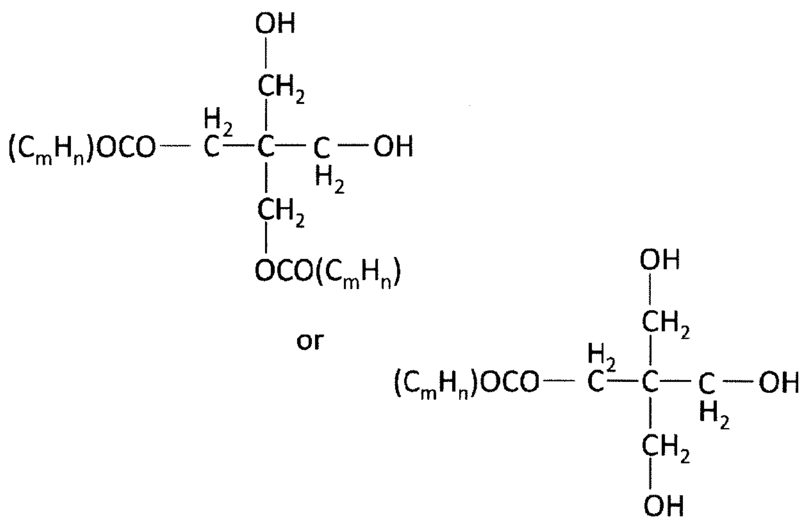

or

In Formula [XI], m and n each are independently an integer of 1 or more. A plurality of m or a plurality of n each may be identical to or different from each other. $C_mH_n$ herein designates a hydrocarbon group of the above-described

fatty acid.

Moreover, examples include sterol, phytosterol and a sterol derivative. Specific examples thereof include cholesterol, sitosterol, stigmasterol, ergosterol, and a mixture thereof, each having a sterol structure of Formula [XII].

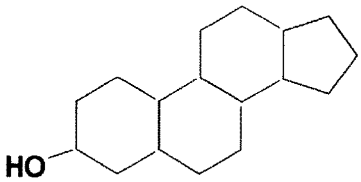

[XII]

Specific examples of alcohol include lauryl alcohol, myristyl alcohol, cetyl alcohol, stearyl alcohol, cetostearyl alcohol, behenyl alcohol and a mixture thereof, as represented by Formula [XIII].

$$C_mH_n - OH \qquad [XIII]$$

In Formula [XIII], m and n each are independently an integer of 1 or more. $C_mH_n$ herein designates a hydrocarbon group of the above-described alcohol.

Specific examples of the fatty acid ester include isopropyl myristate, isopropyl palmitate, cetyl ethylhexanoate, triethylhexanoin, octyldodecyl myristate, ethylhexyl palmitate, ethylhexyl stearate, butyl stearate, myristyl myristate, stearyl stearate, cholesteryl isostearate and a mixture thereof, as represented by Formula [XIV].

$$C_mH_n - COO - C_mH_n \qquad [XIV]$$

In Formula [XIV], m and n each are independently an integer of 1 or more. Two pieces of $C_mH_n$ herein may be identical to or different from each other. $C_mH_n$ in $C_mH_n$-COO- designates a hydrocarbon group of each fatty acid described above. $C_mH_n$ in $-COOC_mH_n$ designates a hydrocarbon group derived from alcohol which forms ester.

Moreover, specific examples of the wax include ceresin, paraffin, vaseline, mineral oil and liquid isoparaffin, as represented by Formula [XV].

$$C_mH_n \qquad [XV]$$

In Formula [XV], m and n each are independently an integer of 1 or more.

The spreading coefficient, the surface tension, the water solubility and the interfacial tension each mentioned above in the second aspect can be set in a predetermined range, in the above-described hydrocarbon compound having the number of carbon atoms of 5 or more, for example, by introducing a small amount of hydrophilicity POE group thereinto at a degree at which water insolubility can be maintained, by introducing a POP group or POB group which is hydrophobic but can reduce the interfacial tension, by changing a chain length of a hydrocarbon chain, by using a material having a branched chain in a hydrocarbon chain, by using a material having a double bond in a hydrocarbon chain, by using a material having a benzene ring or a naphthalene ring in a hydrocarbon chain, or the like.

In the nonwoven fabric according to the present invention, in addition to the above-mentioned liquid film cleavage agent, other components may be arranged thereinto, when necessary. Moreover, the liquid film cleavage agent in the first aspect and the liquid film cleavage agent in the second aspect may be used in combination in addition to an aspect of separate using. This point is the same for the first compound in the liquid film cleavage agent in the second aspect (namely, the polyether compound or the nonionic surfactant described above), and the second compound (namely, the above-described hydrocarbon compound having 5 or more carbon atoms). At this time, as the compound exhibiting the liquid film cleavage effect, one or more of compounds selected from the compound C1 and the compound C2 mentioned above may be used.

In addition, in the topsheet according to the present invention, when the liquid film cleavage agent or the phosphoric acid ester type anionic surfactant being arranged thereinto is identified, the identifying method described in the above-described measuring method for the surface tension ($\gamma_w$) of the liquid film (the liquid having surface tension of 50 mN/m) or the like can be used.

Moreover, when a component of the liquid film cleavage agent is a compound in which a main chain has a siloxane chain, or a hydrocarbon compound in which the number of carbon atoms is 1 or more and 20 or less, an arrangement proportion thereof (OPU) to its fibre mass can be determined by dividing the arrangement proportion of the liquid film cleavage agent by the fibre mass based on mass of substance obtained by the above-described analytical method.

The sanitary towel 10 in the present embodiment can achieve reduction of residual liquid by the above-described

action of the liquid film cleavage agent into a material having excellent liquid permeability. In addition thereto, the sticky feeling on skin on the top surface 1A of the topsheet 1 is effectively reduced. As a result, in the sanitary towel 10, excellent liquid absorption performance and a good wearing feeling can be realised.

In addition, as described in the paragraphs {0089} to {0092} in WO 2016/098796, for the nonwoven fabric constituting the topsheet 1, the interfibre distance necessary for liquid permeability of the topsheet 1 can be reduced than ever before. Thus, a topsheet 1 having softer texture can be obtained by using fibres thinner than ever before.

In the topsheet 1 of the sanitary towel 10 in the embodiment, as described in the paragraphs {0095} to {0096} in WO 2016/098796, the liquid film cleavage agent is preferably localised at least around part of the points that fibres are entangled to each other or the points that fibres are fusion bonded to each other.

In production of the topsheet 1, the arrangement portion 11 of the liquid film cleavage agent can be formed by using various methods. For example, a method described in the paragraphs {0098} to {0099} in WO 2016/098796 can be used. Among these, coating by a spray, coating by a slot coater, and coating by a gravure system, a flexographic system and a dipping system are preferable. From a viewpoint of the above-mentioned localisation and a viewpoint of preferably controlling a coating range on the back surface 1B side of the topsheet 1, a coating method by the flexographic system is particularly preferable.

As a raw material of a constituent member in the sanitary towel 10 of the embodiment, the raw material adopted for an article of this kind can be used without particular restriction.

The topsheet 1 has liquid permeability, and a material used in the article of this kind can be used without particular restriction. From a viewpoint of rapidly absorbing an excreted body fluid and transferring the fluid to the absorbent body, and from a viewpoint of satisfactory texture, a hydrophilic thermally bonded nonwoven fabric is preferable, and an air-through nonwoven fabric is particularly preferable. The fibres to be used are preferably thermoplastic resin fibres subjected to hydrophilic treatment and three-dimensionally crimped fibres, such as secondary or tertiary crimped fibres. Specific examples of the topsheet having the concavo-convex shape shown in FIG. 2 include a material assembling a nonwoven fabric described in the paragraphs {0010} to {0055} in JP-A-2015-110846.

Specifically, polyethylene, polypropylene, polyester, nylon and composite fibres thereof are prepared, and in a step prior to cutting the fibres into a predetermined length to form staples, various hydrophilising agents are coated thereon. Specific examples of the hydrophilising agent include various alkyl sulphonates typified by $\alpha$-olefin sulphonate, acrylate, an acrylate-acrylamide copolymer, ester amide, a salt of ester amide, PEG and a derivative thereof, a water-soluble polyester resin, various silicone derivatives, various saccharide derivatives and a mixture thereof, and the hydrophilic treatment with such hydrophilising agents known to a skilled person in the art can be used thereto.

As the absorbent body 3, the raw material used in the article of this kind can be arbitrarily adopted without particular restriction. Specific examples thereof include a material prepared by covering an absorbent core formed of a hydrophilic fibre aggregate with a core-wrapping sheet. A superabsorbent polymer may be further arranged in the absorbent core. Specific examples of the raw material of the core-wrapping sheet include a paper, a pulp sheet and a hydrophilic nonwoven fabric, manufactured by hydrophilic fibres as the raw material.

Moreover, the absorbent body 3 may be a material processed into a sheet form. Specific examples of the material of the sheet form include a paper and a pulp sheet, manufactured by hydrophilic fibres as the raw material. Moreover, specific examples thereof include a water-absorbing sheet prepared by putting and fixing an absorbent core formed of an aggregate of a superabsorbent polymer member between two substrate sheets (absorbent paper or nonwoven fabric) (for example, the water-absorbing sheet described in JP-A-8-246395 and the polymer sheet described in JP-A-2004-275225).

Specific examples of the fibres of the fibre aggregate or the hydrophilic fibres include natural fibres of wood pulp such as softwood pulp and hardwood pulp or plant pulp, regenerated fibres such as cupra and rayon, semi-synthetic fibres of acetate, and synthetic fibres of polyolefins, polyamides or polyesters, and one kind thereof can be used alone or two or more kinds thereof can be mixed and used.

As a raw material of the backsheet 2, a moisture-permeable film alone, or a laminate in which a film and a nonwoven fabric are laminated, or a water-repellent nonwoven fabric (SMS, SMMS or the like) can be used. From a viewpoint of a cost aspect, matching with a non-slip adhesive, or the like, a moisture-permeable film alone is most preferably used as a leak-proofing material. Specific examples of the film material in this case include a film prepared by stretching, into a predetermined dimension, a film obtained by melting and kneading a thermoplastic resin and an inorganic filler having no compatibility therewith, and extruding the resulting material, and perforating micropores thereinto; or a non-porous film having substantially high compatibility with moisture and capability of discharging water vapour, such as an osmosis membrane.

The sanitary towel 10 in the present embodiment is not limited to the above-described configuration, and may be in various modified forms, or may contain other components. For example, a leak-proof groove 5 may be in a combination of grooves separated into a plurality, in place of the annular form. Moreover, the absorbent article may be a material having a long stretching rear portion flap portion or the like so as to cover wearer's buttocks in the rear portion R, or a material having a pair of wing portions to be fixed to a crotch of underwear in the crotch portion C. Further, water-repellent side sheets for preventing side leak of the excreted liquid may be arranged on both sides on the topsheet 1 in the longitudinal direction (Y direction). Further, the absorbent article may have an adhesive portion to be fixed to underwear on the skin non-contact surface side of the backsheet 2, or may further have a release sheet or the like releasably covering the adhesive portion.

Furthermore, in the sanitary towel 10 of the embodiment, the liquid film cleavage agent may be further incorporated into any other member in addition to the topsheet 1.

The absorbent article according to the present invention is not limited to the above-described sanitary towel, and various articles which absorb and hold the excreted liquid can be formed. For example, the absorbent article may be a pantyliner, an incontinence pad, a nappy and a urine pad.

EXAMPLES

Hereinafter, the present invention will be described more in detail with reference to Examples, but the present invention is not limited thereto. In addition, both terms "part" and "%" in the Examples are based on mass unless otherwise noted. Moreover, as described above, a spreading coefficient, an interfacial tension, a surface tension and a water solubility were values to be measured in an environmental region of a temperature of 25°C and a relative humidity (RH) of 65%. A surface tension, a water solubility and an interfacial tension of a liquid film cleavage agent in the Examples described below were measured by the measuring method described in the paragraphs {0015} to {0022} in WO 2016/098796 described above. In addition, a sign "-" in the following Tables means that neither an agent shown in a name of an item is used, nor the result has an applicable value in the item, or the like. Moreover, a leftward arrow "←" means that the content is the same as described in an adjacent column indicated by the arrow.

(Example 1)

(1) Preparation of nonwoven fabric sample (raw material of topsheet sample)

As a raw material fibre nonwoven fabric serving as a skin-side layer 17, concentric type sheath-core thermally fusible composite fibres having fineness of 1.2 dtex and formed of a core: polyethylene terephthalate and a sheath: a polyethylene component were used to produce a card web by a carding method, and the card web was subjected to hot-air treatment with an air-through heat treatment machine into a sheet, and a skin-side fibre nonwoven fabric having basis weight of 18 g/m$^2$ was prepared. As a raw material fibre web serving as a non-skin side layer 18, side-by-side type latently crimpable composite fibres having fineness of 2.3 dtex and formed of two components of polyethylene and polypropylene were used, and a non-skin side fibre web having basis weight of 22 g/m$^2$ was prepared. In addition, 0.4 mass% of a hydrophilising agent based on fibre mass was coated to the raw material nonwoven fabric and the raw material fibre web used.

Next, the skin-side fibre nonwoven fabric and the non-skin side fibre web were stacked, and the resulting material was subjected to ultrasonic embossing from a side of the upper layer fibre web with the same embossing pattern as in Example 1 described in the description of JP-A-2015-186543. Thus, a concave bonding portion 19 was formed in which the skin-side fibre nonwoven fabric and the non-skin side fibre web were fusion bonded.

Then, treatment of blowing hot air at a temperature of 110°C was performed for 10 seconds in a thickness direction from above on a side of the non-skin side fibre web serving as the non-skin side layer. Thus, in a region between concave bonding portions 19 and 19, the latently crimpable fibres of the non-skin side fibre web were crimped to shrink the non-skin side fibre web, and simultaneously the skin-side fibre nonwoven fabric was projected in a convex shape to obtain a nonwoven fabric having basis weight of 74 g/m$^2$ and formed of the skin-side layer 17 and the non-skin side layer 18 as shown in FIG. 2. The nonwoven fabric obtained was cut into a piece of 225 mm in height by 70 mm in width, and applied as a nonwoven fabric sample in Example 1.

(2) Preparation of applying liquid

An applying liquid containing the following liquid film cleavage agent was prepared.

<Liquid film cleavage agent>

A compound being POE-modified dimethyl silicone (trade name "KF-6015," manufactured by Shin-Etsu Chemical Co., Ltd.), in which X in a structure X-Y was formed of a dimethyl silicone chain composed of $-Si(CH_3)_2O-$, Y was formed of a POE chain composed of $-(C_2H_4O)-$, an end group of the POE chain was a methyl group ($CH_3$), a modification ratio was 20%, and the addition number of moles of POE was 3.

A spreading coefficient of the liquid film cleavage agent to a liquid having surface tension of 50 mN/m and the

like are shown in Tables 1 and 2. These numerical values were obtained by measurement by the above-mentioned measuring method, and are similar also in Examples and Reference Examples described below. On the occasion, as a "liquid having surface tension of 50 mN/m," a solution was used, in which the solution was prepared by adding, to 100 g of deionised water, 3.75 μL of POE sorbitan monolaurate (trade name "RHEODOL Super TW-L120," manufactured by Kao Corporation) being a nonionic surface active substance by a micropipette (trade name "ACURA 825," manufactured by Socorex Isba SA), and adjusting the surface tension to 50±1 mN/m. Moreover, the water solubility was measured by adding the agent for every 0.0001 g. As a result, a sample which was observed to be not dissolved even in 0.0001 g was taken as "less than 0.0001 g," and a sample which was observed to be dissolved in 0.0001 g but not dissolved in 0.0002 g was taken as "0.0001g." The numerical values other than the above were measured by the same methods.

As a result of measuring viscosity by the method shown in (Method for measuring viscosity of liquid film cleavage agent) described above, the viscosity of the liquid film cleavage agent was 163 cps.

(3) Preparation of topsheet sample

The above-described applying liquid was wholly coated to a surface of the non-skin side layer of the above-described nonwoven fabric sample (surface facing a back surface 1B, namely, an absorbent body 3 side of a topsheet 1) by a flexographic printing system to prepare a topsheet sample in Example 1. The applying liquid was wholly coated to the surface, and therefore a whole surface of the back surface 1B served as an arrangement portion 11 of a liquid film cleavage agent. That is, an area ratio of the arrangement portion 11 of the liquid film cleavage agent to an area of the topsheet 1 was 100% on the back surface 1B.

An average arrangement proportion (average OPU, hereinafter, referred to as "Arrangement proportion 1") of the applying liquid to total fibre mass of the topsheet sample, and an arrangement proportion (arrangement portion OPU, hereinafter, referred to as "Arrangement proportion 2") of the applying liquid to fibre mass in the arrangement portion 11 were adjusted as shown in Table 1. In addition, an average arrangement proportion of the hydrophilising agent previously coated on the fibre web serving as the nonwoven fabric sample to fibre mass of the topsheet sample is referred to as "Arrangement proportion 3", and an average arrangement proportion of the hydrophilising agent to the fibre mass of the topsheet sample is referred to as "Arrangement proportion 4".

Moreover, an average arrangement proportion (hereinafter, referred to as "Arrangement proportion 5") of the hydrophilising agent and the liquid film cleavage agent to the total fibre mass of the topsheet sample was adjusted as shown in Table 1.

(4) Preparation of sanitary towel sample

Then, a topsheet was removed from a sanitary towel (trade name "LAURIER F," Shiawase Suhada, 22.5 cm, manufactured by Kao Corporation in 2016), the above-described topsheet sample was laminated as a topsheet in such a manner that the top surface 1A was disposed to be above. Upon lamination, a hot-melt adhesive was intermittently coated in a summit form between the topsheet sample and the absorbent body to be 4.0 g/$m^2$ in coating basis weight, and the topsheet sample and the absorbent body were bonded. Further, a circumference thereof was sealed and fixed.

(Example 2)

A sanitary towel sample in Example 2 was prepared in the same manner to Example 1 except that the arrangement portion of a liquid film cleavage agent was formed in a vertical stripe pattern.

At this time, a longitudinal direction of the arrangement portion 11 and the non-arrangement portion 12 was directed toward a longitudinal direction of the topsheet sample (that is, directed toward a machine direction (MD) during production of a nonwoven fabric sample serving as a topsheet.). A width of the arrangement portion 11 and a width of the non-arrangement portion 12 were adjusted to 1.25 mm and 3.75 mm, respectively, and an area ratio of the arrangement portion was adjusted to be 25%.

(Examples 3 and 4)

Sanitary towel samples in Examples 3 and 4 were prepared in the same manner to Example 2 except that the arrangement proportions 1, 2 and 5 were changed as shown in Table 1.

(Example 5)

A sanitary towel sample in Example 5 was prepared in the same manner to Example 2 except that the applying liquid was applied, in a vertical stripe pattern, on the non-skin side surface (surface facing the non-skin side fibre web)

of the skin side fibre web before formation of a nonwoven fabric sample. That is, the arrangement portion 11 of a liquid film cleavage agent was formed in a thickness intermediate position of a topsheet 1.

In addition, the skin-side fibre web is shrunk together with the non-skin side fibre web by hot-air treatment (MD shrinkage: 76%, CD shrinkage: 86%). In view of the shrinkage, the vertical stripe pattern of the arrangement portion 11 was formed. That is, a width of the arrangement portion 11 and a width of the non-arrangement portion 12 were adjusted to 1.45 mm and 4.34 mm, respectively. The width of the arrangement portion 11 and the width of the non-arrangement portion 12 in the topsheet sample became 1.25 mm and 3.75 mm, respectively, by the above-described CD shrinkage. An area ratio of the arrangement portion 11 was not changed and was 25%. The arrangement proportions 1, 2 and 5 were adjusted as shown in Table 1.

(Examples 6 to 9)

Sanitary towel samples in Examples 6 to 9 were prepared in the same manner to Example 5 except that the applying liquid was wholly coated on the non-skin side surface of the skin-side fibre web, and the arrangement proportions 1, 2 and 5 were changed as shown in Table 1.

(Example 10)

A sanitary towel sample in Example 10 was prepared in the same manner to Example 2 except that the liquid film cleavage agent used in Example 2 and the following hydrophilising agent were blended at a mass ratio of 0.65 {(hydrophilising agent in applying liquid)/(total amount of applying liquid)} under conditions of room temperature of 25°C to prepare an applying liquid.

<Hydrophilising agent (D2)>

PEG (trade name "Polyethylene glycol 400," manufactured by Wako Pure Chemical Corporation)

Mass average molecular weight: 400

Surface tension: 42.8 mN/m

Melting point: about 6°C

The arrangement proportions 1, 2 and 5 were adjusted as shown in Table 1.

(Example 11)

A sanitary towel sample in Example 11 was prepared in the same manner to Example 10 except that the arrangement proportions 1, 2 and 5 were changed as shown in Table 1.

(Example 12)

A sanitary towel sample in Example 12 was prepared in the same manner to Example 6 except that the liquid film cleavage agent used in Example 6 and the following hydrophilising agent were blended at a mass ratio of 0.5 {(hydrophilising agent in applying liquid)/(total amount of applying liquid)} under conditions of room temperature of 25°C to prepare an applying liquid, and the applying liquid was wholly coated to the non-skin side surface (surface facing the non-skin side fibre web) of the skin side fibre web before formation of a nonwoven fabric sample at the arrangement proportions 1, 2 and 5 adjusted as shown in Table 1.

<Hydrophilising agent (D1)>

POE(9) lauryl ether (trade name "EMULGEN 109P," manufactured by Kao Corporation)

Mass average molecular weight: 582
HLB: 13.6
Melting point: 20°C

(Example 13)

A sanitary towel sample in Example 13 was prepared in the same manner to Example 6 except that PPG, in which X in a structure X was formed of a POP chain, and the number of moles of POP groups was 52, was used as the liquid film cleavage agent.

(Example 14)

A sanitary towel sample in Example 14 was prepared in the same manner to Example 6 except that POP-modified dimethyl silicone, in which X in a structure X-Y was a dimethyl silicone chain composed of $SiOC_2H_6$, Y was formed of a POP chain composed of $C_3H_6O$, and the addition number of moles of POP was 12, was used as the liquid film cleavage agent.

(Example 15)

A sanitary towel sample in Example 15 was prepared in the same manner to Example 6 except that caprylic/capric triglyceride, in which Z in a structure Z-Y was $*\text{-O-CH}(CH_2O\text{-}*)_2$ ("*" designates a bonding portion.), Y was formed of a hydrocarbon chain of $C_8H_{15}O$- or $C_{10}H_{19}O$-, and a fatty acid composition was composed of 82% of caprylic acid and 18% of capric acid, was used as the liquid film cleavage agent.

(Example 16)

A sanitary towel sample in Example 16 was prepared in the same manner to Example 6 except that liquid isoparaffin was used as the liquid film cleavage agent.

(Example 17)

A sanitary towel sample in Example 17 was prepared in the same manner to Example 6 except that POP alkyl ether, in which Z was formed of a hydrocarbon chain composed of $-CH_2-$, Y was formed of a POP chain composed of $-(C_3H_6O)-$, and the number of moles of POP groups was 15, was used as the liquid film cleavage agent.

(Example 18)

A sanitary towel sample in Example 18 was prepared in the same manner to Example 6 except that epoxy-modified dimethyl silicone, in which X in a structure X-Y was formed of a dimethyl silicone chain composed of $-Si(CH_3)_2O-$, Y was formed of an epoxy group composed of $-(RC_2H_3O)-$, and a modification ratio was 32%, was used as the liquid film cleavage agent.

(Example 19)

A raw material nonwoven fabric serving as an upper layer sheet being a skin-side layer and a raw material nonwoven fabric serving as a lower layer sheet being a non-skin side layer, forming a topsheet having a concavo-convex shape shown in FIG. 8, were prepared by the following method. That is, thermally non-extensible and thermally non-shrinkable concentric type sheath-core thermally fusible composite fibres formed of a core: polyethylene terephthalate and a sheath: a polyethylene component were applied, and a nonwoven fabric was prepared, according to the method described in JP-A-2004-174234, by using the fibres having fineness of 2.3 dtex for the upper layer sheet, and the fibres having fineness of 2.3 dtex for the lower layer sheet. Basis weight of the nonwoven fabric was adjusted to 41 $g/m^2$. The nonwoven fabric obtained was cut into a piece of 400 mm in height by 105 mm in width, and the piece was used as the raw material nonwoven fabric for each of the upper layer sheet and the lower layer sheet used in Example 19.

The similar applying liquid to Example 1 was wholly coated to a non-skin side surface (surface facing the lower layer sheet) of the raw material nonwoven fabric serving as the upper layer sheet in Example 19, and the resulting material was laminated with the nonwoven fabric serving as the lower layer sheet, and bonding portions shown in FIG. 8 were intermittently formed by embossing to prepare a topsheet sample in Example 19. That is, an arrangement portion 11 of the liquid film cleavage agent was formed in a thickness intermediate position of a topsheet 1. The arrangement proportions 1, 2 and 5 were adjusted as shown in Table 2.

Next, a topsheet was removed from a nappy for babies (trade name "Merries Sarasara Air Through M size, tape type," manufactured by Kao Corporation in 2017), and, in place thereof, the topsheet sample was laminated with the top surface 1A upward. A periphery thereof was fixed, and a nappy for babies sample in Example 19 was prepared.

(Comparative Example 1)

A sanitary towel sample in Comparative Example 1 was prepared in the same manner to Example 1 except that the raw material nonwoven fabric sample (the applying liquid was not arranged) in Example 1 was applied as the topsheet sample.

Although the sanitary towel sample in Comparative Example 1 had no hydrophilising agent derived from the applying liquid, the raw material nonwoven fabric contained a hydrophilising agent (hydrophilising agent not derived from the applying liquid) from the beginning, and the sanitary towel sample had the hydrophilising agent as a whole.

(Comparative Example 2)

A baby nappy sample in Comparative Example 2 was prepared in the same manner to Example 19 except that the raw material nonwoven fabric sample (the applying liquid was not arranged) in Example 19 was applied as the topsheet sample.

Although the baby nappy sample in Comparative Example 2 had no hydrophilising agent derived from the applying liquid, the raw material nonwoven fabric contained a hydrophilising agent (hydrophilising agent not derived from the applying liquid) from the beginning, and the baby nappy sample had the hydrophilising agent as a whole.

(Reference Example 1)

A sanitary towel sample in Reference Example 1 was prepared in the same manner to Example 1 except that a surface having the arrangement portion 11 of a liquid film cleavage agent was applied as a top surface 1A of a topsheet sample.

(Reference Example 2)

A sanitary towel sample in Reference Example 2 was prepared in the same manner to Reference Example 1 except that a pattern was formed in a lattice design (longitudinal pitch of the arrangement portion 11: 13 mm, lateral pitch: 0.8 mm, width of the arrangement portion 11: 0.8 mm, area ratio: 23.4%), and the arrangement proportions 1, 2 and 5 were adjusted as shown in Table 2.

(Testing method)

(1) Amount of residual liquid in topsheet

<Examples 1 to 18, Comparative Example 1, and Reference Examples 1 and 2>

On a surface of each sanitary towel sample, an acrylic plate having a permeation hole with an inner diameter of 1 cm was stacked, and a predetermined load of 100 Pa was applied to the sanitary towel. Under such a load, 6.0 g of the above-described quasi-blood corresponding to menstrual blood was poured thereinto from the permeation hole of the acrylic plate. After 60 seconds from pouring the quasi-blood, the acrylic plate was removed therefrom. Next, mass (W2) of a nonwoven fabric sample was measured, a difference (W2 - W1) from mass (W1) of the nonwoven fabric sample, a weight of which was measured in advance, before pouring the quasi-blood thereinto, was calculated. The operation described above was performed 3 times, and a mean value in 3 times operation was taken as an amount of residual liquid (mg). The amount of residual liquid serves as an indication as to what degree wearer's skin is wetted, and as the amount of residual liquid is lower, better results are obtained.

<Example 19 and Comparative Example 2>

Each baby nappy sample was extended in a plane form and horizontally placed so that a skin side (topsheet side) may be directed upward. Under this state, a procedure of injecting 40 g of artificial urine thereto at a flow rate of 5 g/sec, and then being left to stand for 10 minutes was repeated 3 times in total (injected by 120 g in total), and then mass (W2) of a nonwoven fabric sample was measured to calculate a difference (W2 - W1) from previously measured mass (W1) of the nonwoven fabric sample before flowing the artificial urine. In the same manner as the measurement of the amount of the residual liquid in the sanitary towel sample, the operation described above was performed 3 times, and a mean value in 3 times operation was taken as an amount of residual liquid (mg). An injection point of the artificial urine was adjusted in a lateral centre portion in a position separated by 155 mm longitudinally inward from an end portion on a ventral portion side in a longitudinal direction (lengthwise direction) of an absorbent body.

(2) Sticky feeling on surface on skin-contact surface side of topsheet

Each sanitary towel sample was fixed to a balance (electromagnetic balance, manufactured by Kensei Co., Ltd., trade name GX-6100) so that a topsheet may be faced up, and a ball of an index finger and a ball of a middle finger were pressed to the topsheet with a load of 2 to 3 kg, and reciprocated 40 times over 10 cm in a longitudinal direction of a test sample. Then, the test sample was rubbed between the ball of the index finger and the ball of the middle finger described above, and a ball of a thumb, and a sticky feeling of the test sample was evaluated in terms of 1 to 5 when the sample (without arrangement of the liquid film cleavage agent) in Comparative Example 1 was deemed as "5" and the sample (average CPU of the liquid film cleavage agent: 0.4 mass%) in Reference Example 1 was deemed as "2" as criteria. An average value of four adult men and women (two men and two women) in total was taken as an evaluation value relating to prevention capability of the sticky feeling. A higher point shows that the sample is superior in prevention capability of the sticky feeling on skin.

5: Not felt (equivalent to the sample in Comparative Example 1)
4: Rather not felt (stickiness is not necessarily felt, but a difference is felt from the sample in Comparative Example 1)
3: Rather felt (stickiness is felt, but weaker than in Reference Example 1)
2: Somewhat felt (equivalent to the sample in Reference Example 1)
1: Definitely felt (felt further than in Reference Example 1)

The results in each test with regard to Examples, Comparative Examples, and Reference Examples described above are shown in the following Tables 1 and 2.

Table 1

| | Ex 1 | Ex 2 | Ex 3 | Ex 4 | Ex 5 | Ex 6 | Ex 7 | Ex | Ex 9 | Ex 10 | Ex 11 | Ex 12 |
|---|---|---|---|---|---|---|---|---|---|---|---|---|
| Liquid film cleavage agent | POE (3)-modified dimethyl silicone | ← | ← | ← | ← | ← | ← | ← | ← | ← | ← | ←- |
| Mass average molecular weight | 4,000 | ← | ← | ← | ← | ← | ← | ← | ← | ← | ← | ← |
| Water solubility (g) | Less than 0.0001 | ← | ← | ← | ← | ← | ← | ← | ← | ← | ← | ← |
| Surface tension (mN/m) | 21.0 | ← | ← | ← | ← | ← | ← | ← | ← | ← | ← | ← |
| Spreading coefficient to liquid having surface tension of 50 mN/m (mN/m) | 28.8 | ← | ← | ← | ← | ← | ← | ← | ← | ← | ← | ← |
| Interfacial tension to liquid having surface tension of 50 mN/m (mN/m) | 0.2 | ← | ← | ← | ← | ← | ← | ← | ← | ← | ← | ← |
| Viscosity (cps) | 163 | ← | ← | ← | ← | ← | ← | ← | ← | ← | ← | ← |
| Hydrophilising agent in applying liquid | - | - | - | - | - | - | - | - | - | PEG | ← | POE(9)lauryl ether |
| Mass ratio of total amount of hydrophilising agent in applying liquid to total amount of applying liquid | 0 | ← | ← | ← | ← | ← | ← | ← | ← | 0.65 | ← | 0.5 |

(continued)

| | | | Ex 1 | Ex 2 | Ex 3 | Ex 4 | Ex 5 | Ex 6 | Ex 7 | Ex | Ex 9 | Ex 10 | Ex 11 | Ex 12 |
|---|---|---|---|---|---|---|---|---|---|---|---|---|---|---|
| Surface side having arrangement portion | | | Back surface | 4 | ← | ← | Intermediate | ← | ← | 4 | 4 | Back surface | ← | Intermediate |
| Arrangement pattern of arrangement portion (pattern) | | | Wholly | Stripe | ← | ← | ← | Wholly | ← | ← | ← | Stripe | ← | Wholly |
| Shape of topsheet | | | FIG. 2 | ← | ← | ← | ← | ← | ← | ← | ← | ← | ← | ← |
| | | | Ex 1 | Ex 2 | Ex 3 | Ex 4 | Ex 5 | Ex 6 | Ex 7 | Ex 8 | Ex 9 | Ex 10 | Ex 11 | Ex 12 |
| Arrangement proportion 1 | *1 | | 0.4 | 2.0 | 1.1 | 0.4 | ← | ← | 1.0 | 1.5 | 2.0 | 0.14 Liquid 0.05/ Hydro 0.09 | 1.10 Liquid 0.40/ Hydro 0.70 | 1.50 Liquid 0.75/ Hydro 0.75 |
| Arrangement proportion 2 | *2 | | 0.7 | 14.4 | 7.8 | 0.7 | 3.6 | 0.9 | 2.2 | 3.4 | 4.5 | 1.01 Liquid 0.36/ Hydro 0.65 | 7.94 Liquid 2.71/ Hydro 5.23 | 3.36 Liquid 1.68/ Hydro 1.68 |
| Arrangement proportion 3 | *3 | Top surface | 0.4 | ← | ← | ← | ← | ← | ← | ← | ← | ← | ← | ← |
| | | Intermediate | 0.4 | ← | ← | ← | ← | ← | ← | ← | ← | ← | ← | ← |
| | | Back surface | 0.4 | ← | ← | ← | ← | ← | ← | ← | ← | ← | ← | ← |
| Arrangement proportion 4 | *4 | Top surface | 0.4 | ← | ← | ← | ← | ← | ← | ← | ← | ← | ← | ← |
| | | Intermediate | 0.4 | ← | ← | ← | ← | ← | ← | ← | ← | ← | ← | 3.73 |
| | | Back surface | 0.4 | ← | ← | ← | ← | ← | ← | ← | ← | 0.56 | 1.67 | 0.4 |

(continued)

| | | | Ex 1 | Ex 2 | Ex 3 | Ex 4 | Ex 5 | Ex 6 | Ex 7 | Ex 8 | Ex 9 | Ex 10 | Ex 11 | Ex 12 |
|---|---|---|---|---|---|---|---|---|---|---|---|---|---|---|
| Arrangement proportion 5 | *5 | | 0.8 | 2.4 | 1.5 | 0.8 | ← | 0.8 | 1.4 | 1.9 | 2.4 | 0.54 Liquid 0.05/ Hydro 0.49 | 1.50 Liquid 0.40/ Hydro 1.10 | 1.90 Liquid 0.75/ Hydro 1.15 |
| *6 | | Top surface | 0 | ← | ← | ← | ← | ← | ← | ← | ← | ← | ← | ← |
| | | Intermediate | 0 | ← | ← | ← | 25 | 100 | ← | ← | ← | 0 | ← | 100 |
| | | Back surface | 100 | 25 | ← | ← | 0 | ← | ← | ← | ← | 25 | ← | 0 |
| Amount of residual liquid (6 g injection) (mg) | | | 110 | 123 | 112 | 97 | 74 | 84 | 86 | 88 | 90 | 87 | 73 | 81 |
| Prevention capability of sticky feeling and slipping feeling | | | 5 | 5 | 5 | 5 | 4 | 5 | 4 | 4 | 3 | 5 | 3 | 5 |

Remarks: 'Ex' means Example according to this invention.

*1: Average arrangement proportion of the applying liquid to the total fibre mass of the topsheet (average OPU) (mass%)

*2: Average arrangement proportion of the applying liquid to the fibre mass in the arrangement portion of the topsheet (arrangement portion OPU) (mass%)

*3: Average arrangement proportion of the hydrophilising agent previously coated on the fibre web serving as the nonwoven fabric sample to the fibre mass of the topsheet in each surface (mass%)

*4: Average arrangement proportion of the hydrophilising agent to the fibre mass of the topsheet in each surface (mass%)

*5: Average arrangement proportion of the hydrophilising agent and the liquid film cleavage agent to the total fibre mass of the topsheet (mass%)

*6: Area proportion of the arrangement portion of the applying liquid (%) {(total area of the arrangement portion)/(sum of total area of the arrangement portion and the non-arrangement portion) × 100}

Note 1) The "Arrangement proportion 4" in each Examples shows a total content proportion of "the hydrophilising agent previously coated on the fibre web serving as the nonwoven fabric sample" and "the hydrophilising agent in the applying liquid".

Note 2) "Average arrangement proportion of applying liquid" in Examples 1 to 9 shows a content proportion of the liquid film cleavage agent.

Note 3) The "Arrangement proportion 1", "Arrangement proportion 2" and "Arrangement proportion 5" in Examples 10 to 12 show, in a lower row, each arrangement proportion of the liquid film cleavage agent (represented as "Liquid") and the hydrophilising agent (represented as "Hydro"), and in an upper row, a total arrangement proportion of the liquid film cleavage agent and the hydrophilising agent.

Note 4) The "Arrangement proportion 5" in each Examples shows a total content proportion of the "hydrophilising agent previously coated on the fibre web serving as the nonwoven fabric sample", "the hydrophilising agent in the applying liquid" and "the liquid film cleavage agent".

Table 2

| | Ex 13 | Ex 14 | Ex 15 | Ex 16 | Ex 17 | Ex 18 | Ex 19 |
|---|---|---|---|---|---|---|---|
| Liquid film cleavage agent | PPG | POP (12)-modified dimethyl silicone | Caprylic/ capric triglyceride | Liquid isoparaffin | POP(15) alkyl ether | Epoxymodified dimethyl silicone | POE(3)-modified dimethyl silicone |
| Mass average molecular weight | 3,000 | 8,000 | 550 | 450 | 1,140 | 35,800 | 4,000 |
| Water solubility (g) | 0.025 or less | ← | Less than 0.0001 | ← | ← | ← | ← |
| Surface tension (mN/m) | 33.0 | 21.0 | 28.9 | 27.0 | 31.8 | 21.0 | 21.0 |
| Spreading coefficient to liquid having surface tension of 50 mN/m (mN/m) | 17.0 | 29.0 | 8.8 | 14.5 | 5.4 | 26.0 | 28.8 |
| Interfacial tension to liquid having surface tension of 50 mN/m (mN/m) | 1.0 | 0.5 | 12.3 | 8.5 | 12.8 | 3.0 | 0.2 |
| Viscosity (cps) | 603 | 250 | 24.1 | 20.0 | 129 | 1,515 | 163 |
| Hydrophilising agent in applying liquid | - | - | - | - | - | - | - |
| Mass ratio of total amount of hydrophilising agent in applying liquid to total amount of applying liquid | 0 | ← | ← | ← | ← | ← | ← |

(continued)

| | Ex 13 | Ex 14 | Ex 15 | Ex 16 | Ex 17 | Ex 18 | Ex 19 |
|---|---|---|---|---|---|---|---|
| Surface side having arrangement portion | Intermediate | ← | ← | ← | ← | ← | ← |
| Arrangement pattern of arrangement portion (pattern) | Wholly | ← | ← | ← | ← | ← | ← |
| Shape of topsheet | FIG. 2 | ← | ← | ← | ← | ← | FIG. 8 |

| | C Ex 1 | C Ex2 | R Ex 1 | R Ex 2 |
|---|---|---|---|---|
| Liquid film cleavage agent | - | - | POE (3)-modified dimethyl silicone | ← |
| Mass average molecular weight | - | - | 4,000 | ← |
| Water solubility (g) | - | - | Less than 0.0001 | ← |
| Surface tension (mN/m) | - | - | 21.0 | ← |
| Spreading coefficient to liquid having surface tension of 50 mN/m (mN/m) | - | - | 28.8 | ← |

(continued)

| | C Ex 1 | C Ex2 | R Ex 1 | R Ex 2 |
|---|---|---|---|---|
| Interfacial tension to liquid having Surface tension of 50 mN/m (mN/m) | - | - | 0.2 | ← |
| Viscosity (cps) | - | - | 163 | ← |
| Hydrophilising agent in applying liquid | - | - | - | - |
| Mass ratio of total amount of hydrophilising agent in applying liquid to total amount of applying liquid | - | - | - | - |
| Surface side having arrangement portion | Not coated | ← | Top surface | ← |
| Arrangement pattern of arrangement portion (pattern) | Not coated | ← | Wholly | Lattice |
| Shape of topsheet | FIG. 2 | FIG. 8 | FIG. 2 | ← |

| | | Ex 13 | Ex 14 | Ex 15 | Ex 16 | Ex 17 | Ex 18 | Ex 19 | C Ex 1 | C Ex 2 | R Ex 1 | R Ex 2 |
|---|---|---|---|---|---|---|---|---|---|---|---|---|
| Arrangement proportion 1 | *1 | 0.4 | ← | ← | ← | ← | ← | 0.7 | - | - | 0.4 | 0.3 |
| Arrangement proportion 2 | *2 | 0.9 | ← | ← | ← | ← | ← | 1.2 | - | - | 0.9 | 2.9 |

(continued)

| | | | Ex 13 | Ex 14 | Ex 15 | Ex 16 | Ex 17 | Ex 18 | Ex 19 | C Ex 1 | C Ex 2 | R Ex 1 | R Ex 2 |
|---|---|---|---|---|---|---|---|---|---|---|---|---|---|
| Arrangement proportion 3 | *3 | Top surface | 0.4 | ← | ← | ← | ← | ← | ← | ← | ← | ← | ← |
| | | Intermediate | 0.4 | ← | ← | ← | ← | ← | ← | ← | ← | ← | ← |
| | | Back surface | 0.4 | ← | ← | ← | ← | ← | ← | | ← | ← | ← |
| Arrangement proportion 4 | *4 | Top surface | 0.4 | ← | ← | ← | ← | ← | ← | ← | ← | ← | ← |
| | | Intermediate | 0.4 | ← | ← | ← | ← | ← | ← | ← | ← | ← | ← |
| | | Back surface | 0.4 | ← | ← | ← | ← | ← | ← | ← | ← | ← | ← |
| Arrangement proportion 5 | *5 | | 0.8 | ← | ← | ← | ← | ← | 1.1 | 0.4 | ← | 0.8 | 0.7 |
| *6 | | Top surface | 0 | ← | ← | ← | ← | ← | ← | - | - | 100 | ← |
| | | Intermediate | 100 | ← | ← | ← | ← | ← | ← | - | - | 0 | ← |
| | | Back surface | 0 | ← | ← | ← | ← | ← | ← | - | - | 0 | ← |
| Amount of residual liquid (6 g injection) (mg) | | | 173 | 90 | 138 | 163 | 123 | 120 | 69 | 198 | 99 | 72 | 81 |
| Prevention capability of sticky feeling and slipping feeling | | | 5 | 5 | 5 | 5 | 5 | 5 | 4 | 5 | 5 | 2 | 1 |

Remarks: 'Ex' means Example according to this invention, 'C Ex' means Comparative Example, and 'R Ex' means Reference Example.
*1: Average arrangement proportion of the applying liquid to the total fibre mass of the topsheet (average OPU) (mass%)
*2: Average arrangement proportion of the applying liquid to the fibre mass in the arrangement portion of the topsheet (arrangement portion OPU) (mass%)
*3: Average arrangement proportion of the hydrophilising agent previously coated on the fibre web serving as the nonwoven fabric sample to the fibre mass of the topsheet in each surface (mass%)
*4: Average arrangement proportion of the hydrophilising agent to the fibre mass of the topsheet in each surface (mass%)
*5: Average arrangement proportion of the hydrophilising agent and the liquid film cleavage agent to the total fibre mass of the topsheet (mass%)
*6: Area proportion of the arrangement portion of the applying liquid (%) {(total area of the arrangement portion)/(sum of total area of the arrangement portion and the non-arrangement portion) $\times$ 100}
Note 1) The "Arrangement proportion 4" in each Examples, Comparative Examples and Reference Examples shows a total content proportion of "the hydrophilising agent previously coated on the fibre web serving as the nonwoven fabric sample" and "the hydrophilising agent in the applying liquid".
Note 2) The "Arrangement proportion 5" in each Examples, Comparative Examples and Reference Examples shows a total content proportion of the "hydrophilising agent previously coated on the fibre web serving as the nonwoven fabric sample", "the hydrophilising agent in the applying liquid" and "the liquid film cleavage agent".

As shown in Tables 1 and 2, while the amount of residual liquid in Comparative Example 1 in which the liquid film cleavage agent was not contained therein was 198 mg, and the amount of residual liquid in Comparative Example 2 was 99 mg, the amounts of residual liquids in Examples 1 to 18 were suppressed at a level as low as about 40 to about 90% of the amount in Comparative Example 1, and the amount of residual liquid in Example 19 was suppressed at a level as low as about 70% or less of the amount in Comparative Example 2, whereby effective cleavage of the liquid film was confirmed. That is, in Examples 1 to 19, an effect of reduction of residual liquid in a liquid film cleavage agent was high. In particular, in Examples 10 to 12, the arrangement portion contained both the liquid film cleavage agent and the hydrophilising agent, and therefore liquid sucking capability was accompanied, whereby the amount of residual liquid was reduced to about a half or less of the amount in Comparative Examples, and the effect of reduction of residual liquid was higher.

On the other hand, in Reference Examples 1 and 2, the liquid film cleavage agent was arranged on the top surface (surface facing a wearer's skin side) of the topsheet, and therefore stickiness on the surface was felt further than in Comparative Example 1.

In contrast thereto, in Examples 1 to 19, the liquid film cleavage agent was arranged on the back surface (surface facing an absorbent body side) side or in the thickness intermediate position of the topsheet, and therefore the prevention capability of the sticky feeling was higher than in Reference Examples 1 and 2.

As described above, in Examples 1 to 19, it was possible to simultaneously realise reduction of residual liquid and reduction of sticky feeling on skin.

Having described our invention as related to this embodiments, aspects and Examples, it is our intention that the invention not be limited by any of the details of the description, unless otherwise specified, but rather be construed broadly within its spirit and scope as set out in the accompanying claims.

This application claims priority on Patent Application No. 2017-119148 filed in Japan on June 16, 2017.

DESCRIPTION OF SYMBOLS

1 Topsheet
2 Backsheet
3 Absorbent body
5 Leak-proof groove
10 Sanitary towel
11 Arrangement portion
12 Non-arrangement portion
13 Thickness intermediate position of topsheet
17 Skin side layer
18 Non-skin side layer
1A Top surface of topsheet
1B Back surface of topsheet

## Claims

**1.** An absorbent article comprising:

a topsheet,
a backsheet, and
an absorbent body interposed between the topsheet and the backsheet,
wherein the topsheet has an arrangement portion of one or more of compounds selected from the following compound C1 and the following compound C2 in a thickness intermediate position or on an absorbent body side, wherein
compound C1 is a compound having a spreading coefficient of 15 mN/m or more to a liquid having a surface tension of 50 mN/m, and a water solubility of 0 g or more and 0.025 g or less, and
compound C2 is a compound having a spreading coefficient of more than 0 mN/m to a liquid having a surface tension of 50 mN/m, a water solubility of 0 g or more and 0.025 g or less, and an interfacial tension of 20 mN/m or less to the liquid having a surface tension of 50 mN/m.

**2.** The absorbent article according to claim 1, wherein, in a region having the arrangement portion, a non-arrangement

portion of the compound C1 and the compound C2 is further provided.

3. The absorbent article according to claim 1 or 2, wherein a plurality of the arrangement portions exists in the plane direction of the topsheet.

4. The absorbent article according to any one of claims 1 to 3, wherein, in the thickness immediate position of the topsheet, or in the surface facing the absorbent body side of the topsheet, the region having the arrangement portion is a whole region of the topsheet in the plane direction.

5. The absorbent article according to any one of claims 1 to 4, wherein the area ratio of the arrangement portion in an area of the topsheet is preferably adjusted to be smaller on the surface facing the wearer's skin side of the topsheet than in the thickness intermediate position or on the surface facing the absorbent body side of the topsheet, and it is more preferable that no arrangement portion exists on the surface facing the wearer's skin side of the topsheet.

6. The absorbent article according to any one of claims 1 to 5, wherein the topsheet contains a hydrophilising agent, and an arrangement amount of the hydrophilising agent is increased on the surface facing the absorbent body side of the topsheet than on the surface facing the wearer's skin side of the topsheet.

7. The absorbent article according to any one of claims 1 to 6, wherein the viscosity of the one or more of compounds selected from the compound C1 and the compound C2 is 0 cps or more; and preferably 6,000 cps or less, more preferably 600 cps or less, and further preferably 200 cps or less.

8. Use of one or more of compounds selected from the following compound C1 and the following compound C2 for liquid film cleavage in an absorbent article,

wherein the absorbent article comprises a topsheet, a backsheet, and an absorbent body interposed between the topsheet and the backsheet, and
wherein the topsheet has an arrangement portion of one or more of compounds selected from the following compound C1 and the following compound C2 in a thickness intermediate position or on an absorbent body side, wherein

compound C1 is a compound having a spreading coefficient of 15 mN/m or more to a liquid having a surface tension of 50 mN/m, and a water solubility of 0 g or more and 0.025 g or less, and
compound C2 is a compound having a spreading coefficient of more than 0 mN/m to a liquid having a surface tension of 50 mN/m, a water solubility of 0 g or more and 0.025 g or less, and an interfacial tension of 20 mN/m or less to the liquid having a surface tension of 50 mN/m.

9. A method for producing a nonwoven fabric, comprising:

a step of providing one or more of compounds selected from the following compound C1 and the following compound C2 on one surface of a first fibre layer having thermally fusible fibres;
a step of laminating a second fibre layer having thermally fusible fibres on the surface of the first fibre layer, on which one or more of compounds selected from the compound C1 and the compound C2 is provided; and
a step of bonding the first fibre layer and the second fibre layer by thermal fusion by heat treatment at a temperature higher than or equal to a melting point of the thermally fusible fibres,
wherein
compound C1 is a compound having a spreading coefficient of 15 mN/m or more to a liquid having a surface tension of 50 mN/m, and a water solubility of 0 g or more and 0.025 g or less, and
compound C2 is a compound having a spreading coefficient of more than 0 mN/m to a liquid having a surface tension of 50 mN/m, a water solubility of 0 g or more and 0.025 g or less, and an interfacial tension of 20 mN/m or less to the liquid having a surface tension of 50 mN/m.

10. The method for producing a nonwoven fabric according to claim 9, wherein in the step of providing one or more of compounds selected from the compound C1 and the compound C2, coating treatment is performed in a mixed state of a hydrophilising agent and the one or more of compounds selected from the compound C1 and the compound C2.

11. The method for producing a nonwoven fabric according to claim 9 or 10,

wherein the heat treatment at a temperature higher than or equal to a melting point of the thermal fusible fibres is an embossing compression treatment, and
wherein after the embossing compression treatment, a hot-air treatment is performed at a temperature lower than or equal to the melting point of the thermally fusible fibres.

**12.** The method for producing a nonwoven fabric according to any one of claims 9 to 11, wherein the nonwoven fabric is a topsheet for an absorbent article.

**13.** A method for producing an absorbent article, wherein the nonwoven fabric produced by the production method according to any one of claims 9 to 11 is used as a topsheet,
the method comprising a step of laminating and bonding an absorbent body and a backsheet on one surface side of the topsheet.

## Patentansprüche

**1.** Ein absorbierender Gegenstand, umfassend:

ein Topsheet,
ein Backsheet und
einen Absorptionskörper, welcher zwischen dem Topsheet und dem Backsheet angeordnet ist,

wobei das Topsheet einen Anordnungsteil einer oder mehrerer Verbindungen, ausgewählt aus der folgenden Verbindung C1 und der folgenden Verbindung C2, in einer Dicken-Zwischenposition oder auf einer Absorptionskörper-Seite aufweist,
wobei

Verbindung C1 eine Verbindung ist, welche einen Spreitungskoeffizienten von 15 mN/m oder mehr zu einer Flüssigkeit mit einer Oberflächenspannung von 50 mN/m und eine Wasserlöslichkeit von 0 g oder mehr und 0,025 g oder weniger aufweist, und
Verbindung C2 eine Verbindung ist, welche einen Spreitungskoeffizienten von mehr als 0 mN/m zu einer Flüssigkeit mit einer Oberflächenspannung von 50 mN/m, eine Wasserlöslichkeit von 0 g oder mehr und 0,025 g oder weniger, und eine Grenzflächenspannung von 20 mN/m oder weniger zu der Flüssigkeit mit einer Oberflächenspannung von 50 mN/m aufweist.

**2.** Der absorbierende Gegenstand gemäß Anspruch 1, wobei in einem Bereich, welcher den Anordnungsteil aufweist, ferner ein Nicht-Anordnungsteil der Verbindung C1 und der Verbindung C2 bereitgestellt ist.

**3.** Der absorbierende Gegenstand gemäß Anspruch 1 oder 2, wobei eine Mehrzahl der Anordnungsteile in der Ebenenrichtung des Topsheets vorhanden ist.

**4.** Der absorbierende Gegenstand gemäß einem der Ansprüche 1 bis 3, wobei in der Dicken-Zwischenposition des Topsheets oder in der Oberfläche des Topsheets, welche der Seite des Absorptionskörpers zugewandt ist, der Bereich mit dem Anordnungsteil ein ganzer Bereich des Topsheets in der Ebenenrichtung ist.

**5.** Der absorbierende Gegenstand gemäß einem der Ansprüche 1 bis 4, wobei das Flächenverhältnis des Anordnungsteils in einer Fläche des Topsheets vorzugsweise derart eingestellt ist, dass es auf der Hautseite des Trägers zugewandten Oberfläche des Topsheets kleiner ist als in der Dicken-Zwischenposition oder auf der der Absorptionskörper-Seite zugewandten Oberfläche des Topsheets, und es ist stärker bevorzugt, dass auf der der Hautseite des Trägers zugewandten Oberfläche des Topsheets kein Anordnungsteil vorhanden ist.

**6.** Der absorbierende Gegenstand gemäß einem der Ansprüche 1 bis 5, wobei das Topsheet ein Hydrophilisierungsmittel enthält und eine Anordnungsmenge des Hydrophilisierungsmittels auf der Oberfläche des Topsheets, welche der Absorptionskörper-Seite zugewandt ist, größer ist als auf der Oberfläche des Topsheets, die der Hautseite des Trägers zugewandt ist.

**7.** Der aborbierende Gegenstand gemäß einem der Ansprüche 1 bis 6, wobei die Viskosität der einen oder mehreren Verbindungen, ausgewählt aus der Verbindung C1 und der Verbindung C2, 0 cps oder mehr; und vorzugsweise

6000 cps oder weniger, stärker bevorzugt 600 cps oder weniger, und noch stärker bevorzugt 200 cps oder weniger, beträgt.

**8.** Verwendung einer oder mehrerer Verbindungen, ausgewählt aus der folgenden Verbindung C1 und der folgenden Verbindung C2, zur Flüssigkeitsfilmspaltung in einem absorbierenden Gegenstand,
wobei der absorbierende Gegenstand ein Topsheet, ein Backsheet und einen Absorptionskörper, welcher zwischen dem Topsheet und dem Backsheet angeordnet ist, umfasst, und
wobei das Topsheet einen Anordnungsteil einer oder mehrerer Verbindungen, ausgewählt aus der folgenden Verbindung C1 und der folgenden Verbindung C2, in einer Dicken-Zwischenposition oder auf einer Absorptionskörper-Seite aufweist,
wobei

Verbindung C1 eine Verbindung ist, welche einen Spreitungskoeffizienten von 15 mN/m oder mehr zu einer Flüssigkeit mit einer Oberflächenspannung von 50 mN/m und eine Wasserlöslichkeit von 0 g oder mehr und 0,025 g oder weniger aufweist, und
Verbindung C2 eine Verbindung ist, welche einen Spreitungskoeffizienten von mehr als 0 mN/m zu einer Flüssigkeit mit einer Oberflächenspannung von 50 mN/m, eine Wasserlöslichkeit von 0 g oder mehr und 0,025 g oder weniger, und eine Grenzflächenspannung von 20 mN/m oder weniger zu der Flüssigkeit mit einer Oberflächenspannung von 50 mN/m aufweist.

**9.** Ein Verfahren zur Herstellung eines Vliesstoffs, umfassend:

einen Schritt des Bereitstellens einer oder mehrerer Verbindungen, ausgewählt aus der folgenden Verbindung C1 und der folgenden Verbindung C2, auf einer Oberfläche einer ersten Faserschicht mit thermisch schmelzbaren Fasern;
einen Schritt des Laminierens einer zweiten Faserschicht mit thermisch schmelzbaren Fasern auf die Oberfläche der ersten Faserschicht, auf welcher eine oder mehrere Verbindungen, ausgewählt aus der Verbindung C 1 und der Verbindung C2, bereitgestellt ist/sind; und
einen Schritt des Bindens der ersten Faserschicht und der zweiten Faserschicht mittels thermischem Verschmelzen durch Wärmebehandlung bei einer Temperatur, welche höher als oder gleich einem Schmelzpunkt der thermisch schmelzbaren Fasern ist,
wobei
Verbindung C1 eine Verbindung ist, welche einen Spreitungskoeffizienten von 15 mN/m oder mehr zu einer Flüssigkeit mit einer Oberflächenspannung von 50 mN/m und eine Wasserlöslichkeit von 0 g oder mehr und 0,025 g oder weniger aufweist, und
Verbindung C2 eine Verbindung ist, welche einen Spreitungskoeffizienten von mehr als 0 mN/m zu einer Flüssigkeit mit einer Oberflächenspannung von 50 mN/m, eine Wasserlöslichkeit von 0 g oder mehr und 0,025 g oder weniger, und eine Grenzflächenspannung von 20 mN/m oder weniger zu der Flüssigkeit mit einer Oberflächenspannung von 50 mN/m aufweist.

**10.** Das Verfahren zur Herstellung eines Vliesstoffs gemäß Anspruch 9, wobei in dem Schritt des Bereitstellens einer oder mehrerer Verbindungen, ausgewählt aus der Verbindung C1 und der Verbindung C2, eine Beschichtungsbehandlung in einem gemischten Zustand aus einem Hydrophilisierungsmittel und der einen oder mehreren Verbindungen, ausgewählt aus der Verbindung C1 und der Verbindung C2, durchgeführt wird.

**11.** Das Verfahren zur Herstellung eines Vliesstoffs gemäß Anspruch 9 oder 10, wobei die Wärmebehandlung bei einer Temperatur, welche höher als oder gleich einem Schmelzpunkt der thermisch schmelzbaren Fasern ist, eine Prägekompressionsbehandlung ist, und
wobei nach der Prägekompressionsbehandlung eine Heißluftbehandlung bei einer Temperatur, welche niedriger als oder gleich dem Schmelzpunkt der thermisch schmelzbaren Fasern ist, durchgeführt wird.

**12.** Das Verfahren zur Herstellung eines Vliesstoffs gemäß einem der Ansprüche 9 bis 11, wobei der Vliesstoff ein Topsheet für einen absorbierenden Gegenstand ist.

**13.** Ein Verfahren zur Herstellung eines absorbierenden Gegenstandes, wobei der durch das Herstellungsverfahren gemäß einem der Ansprüche 9 bis 11 hergestellte Vliesstoff als ein Topsheet verwendet wird,
wobei das Verfahren einen Schritt des Laminierens und Bindens eines Absorptionskörpers und eines Backsheets auf einer Oberflächenseite des Topsheets umfasst.

## Revendications

**1.** Article absorbant comprenant :

une feuille de dessus,
une feuille de dessous, et
un corps absorbant interposé entre la feuille de dessus et la feuille de dessous,
dans lequel la feuille de dessus a une portion de disposition d'un ou plusieurs composés choisis parmi le composé C1 qui suit et le composé C2 qui suit à une position d'épaisseur intermédiaire ou sur un côté corps absorbant,
dans lequel
le composé C1 est un composé ayant un coefficient d'étalement de 15 mN/m ou plus vis-à-vis d'un liquide ayant une tension de surface de 50 mN/m, et une solubilité dans l'eau de 0 g ou plus et 0,025 g ou moins, et
le composé C2 est un composé ayant un coefficient d'étalement supérieur à 0 mN/m vis-à-vis d'un liquide ayant une tension de surface de 50 mN/m, une solubilité dans l'eau de 0 g ou plus et 0,025 g ou moins, et une tension à l'interface de 20 mN/m ou moins vis-à-vis du liquide ayant une tension de surface de 50 mN/m.

**2.** Article absorbant selon la revendication 1, dans lequel, dans une région ayant la portion de disposition, une portion de non-disposition du composé C1 et du composé C2 est en outre présente.

**3.** Article absorbant selon la revendication 1 ou 2, dans lequel une pluralité des portions de disposition existe dans la direction du plan de la feuille de dessus.

**4.** Article absorbant selon l'une quelconque des revendications 1 à 3, dans lequel, à la position d'épaisseur intermédiaire de la feuille de dessus, ou dans la surface faisant face au côté corps absorbant de la feuille de dessus, la région ayant la portion de disposition est une région complète de la feuille de dessus dans la direction du plan.

**5.** Article absorbant selon l'une quelconque des revendications 1 à 4, dans lequel la proportion en surface de la portion de disposition dans une zone de la feuille de dessus est de préférence ajustée pour être plus petite sur la surface faisant face au côté peau de l'utilisateur de la feuille de dessus qu'à la position d'épaisseur intermédiaire ou sur la surface faisant face au côté corps absorbant de la feuille de dessus, et il est davantage préférable qu'aucune portion de disposition n'existe sur la surface faisant face au côté peau de l'utilisateur de la feuille de dessus.

**6.** Article absorbant selon l'une quelconque des revendications 1 à 5, dans lequel la feuille de dessus contient un agent hydrophilisant, et la quantité de disposition de l'agent hydrophilisant est plus importante sur la surface faisant face au côté corps absorbant de la feuille de dessus que sur la surface faisant face au côté peau de l'utilisateur de la feuille de dessus.

**7.** Article absorbant selon l'une quelconque des revendications 1 à 6, dans lequel la viscosité du ou des composés choisis parmi le composé C1 et le composé C2 est de 0 cps ou plus ; et de préférence 6 000 cps ou moins, mieux encore 600 cps ou moins, et plus particulièrement 200 cps ou moins.

**8.** Utilisation d'un ou plusieurs composés choisis parmi le composé C1 qui suit et le composé C2 qui suit pour le clivage d'un film liquide dans un article absorbant,

dans laquelle l'article absorbant comprend une feuille de dessus, une feuille de dessous, et un corps absorbant interposé entre la feuille de dessus et la feuille de dessous, et
dans laquelle la feuille de dessus a une portion de disposition d'un ou plusieurs composés choisis parmi le composé C1 qui suit et le composé C2 qui suit à une position d'épaisseur intermédiaire ou sur un côté corps absorbant,
dans lequel
le composé C1 est un composé ayant un coefficient d'étalement de 15 mN/m ou plus vis-à-vis d'un liquide ayant une tension de surface de 50 mN/m, et une solubilité dans l'eau de 0 g ou plus et 0,025 g ou moins, et
le composé C2 est un composé ayant un coefficient d'étalement supérieur à 0 mN/m vis-à-vis d'un liquide ayant une tension de surface de 50 mN/m, une solubilité dans l'eau de 0 g ou plus et 0,025 g ou moins, et une tension à l'interface de 20 mN/m ou moins vis-à-vis du liquide ayant une tension de surface de 50 mN/m.

**9.** Méthode pour produire une étoffe non tissée, comprenant :

une étape de fourniture d'un ou plusieurs composés choisis parmi le composé C1 qui suit et le composé C2 qui suit sur une surface d'une première couche de fibres ayant des fibres thermofusibles ;
une étape de stratification d'une deuxième couche de fibres ayant des fibres thermofusibles sur la surface de la première couche de fibres, sur laquelle un ou plusieurs composés choisis parmi le composé C1 et le composé C2 sont disposés ; et
une étape de collage de la première couche de fibres et de la deuxième couche de fibres par fusion thermique par traitement thermique à une température supérieure ou égale au point de fusion des fibres thermofusibles, dans laquelle
le composé C1 est un composé ayant un coefficient d'étalement de 15 mN/m ou plus vis-à-vis d'un liquide ayant une tension de surface de 50 mN/m, et une solubilité dans l'eau de 0 g ou plus et 0,025 g ou moins, et
le composé C2 est un composé ayant un coefficient d'étalement supérieur à 0 mN/m vis-à-vis d'un liquide ayant une tension de surface de 50 mN/m, une solubilité dans l'eau de 0 g ou plus et 0,025 g ou moins, et une tension à l'interface de 20 mN/m ou moins vis-à-vis du liquide ayant une tension de surface de 50 mN/m.

**10.** Méthode pour produire une étoffe non tissée selon la revendication 9, dans lequel, dans l'étape de fourniture d'un ou plusieurs composés choisis parmi le composé C1 et le composé C2, un traitement de revêtement est effectué dans un état mélangé d'un agent hydrophilisant et du ou des composés choisis parmi le composé C1 et le composé C2.

**11.** Méthode pour produire une étoffe non tissée selon la revendication 9 ou 10,

dans laquelle le traitement thermique à une température supérieure ou égale au point de fusion des fibres thermofusibles est un traitement de gaufrage par compression, et
dans laquelle, après le traitement de gaufrage par compression, un traitement à l'air chaud est effectué à une température inférieure ou égale au point de fusion des fibres thermofusibles.

**12.** Méthode pour produire une étoffe non tissée selon l'une quelconque des revendications 9 à 11, dans laquelle l'étoffe non tissée est une feuille de dessus pour un article absorbant.

**13.** Méthode pour produire un article absorbant, dans laquelle l'étoffe non tissée produite par le procédé de production selon l'une quelconque des revendications 9 à 11 est utilisée en tant que feuille de dessus,
la méthode comprenant une étape de stratification et de collage d'un corps absorbant et d'une feuille de dessous sur un côté de surface de la feuille de dessus.

{FIG. 1}

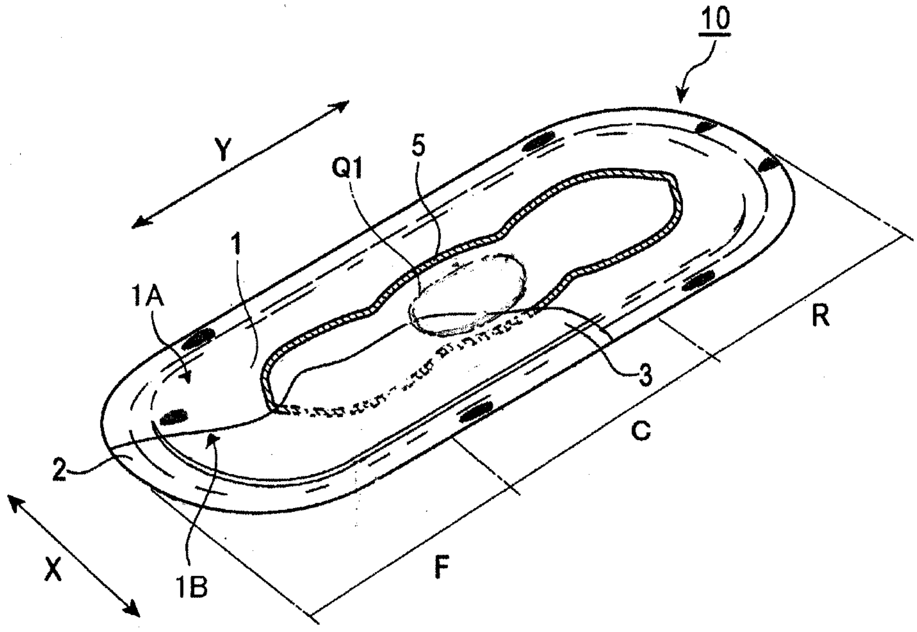


{FIG. 2}

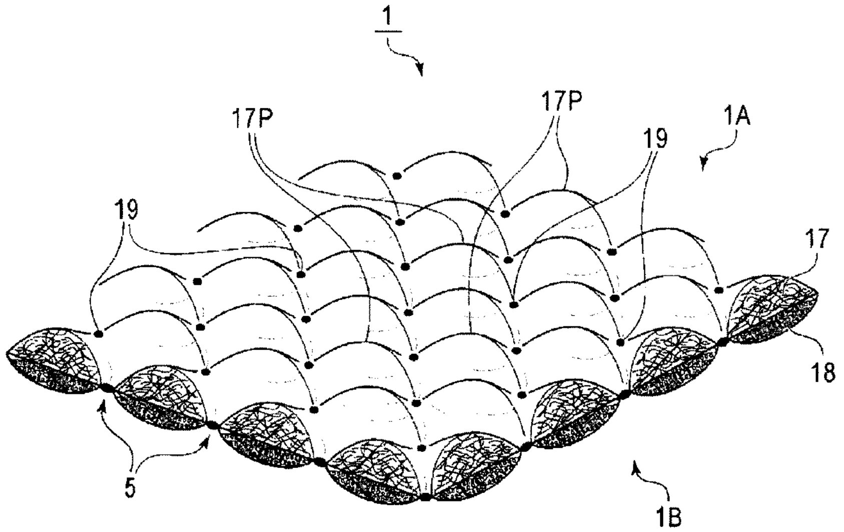

{FIG. 3}

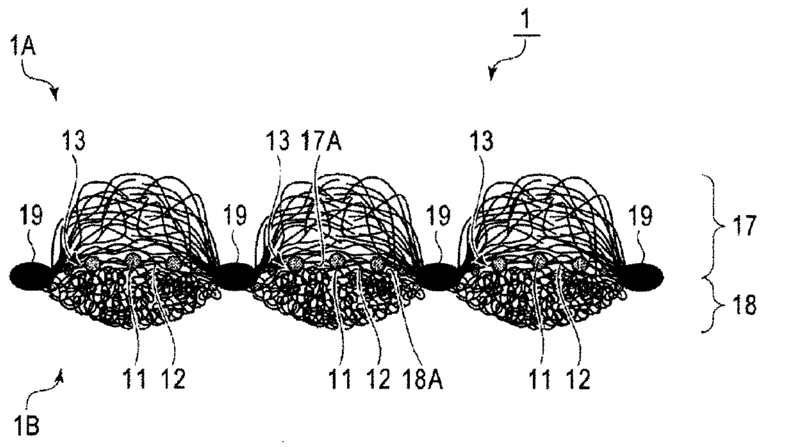
1
1A
13
13 17A
13
19
19
19
19
17
18
11 12
11 12 18A
11 12
1B

{FIG. 4}

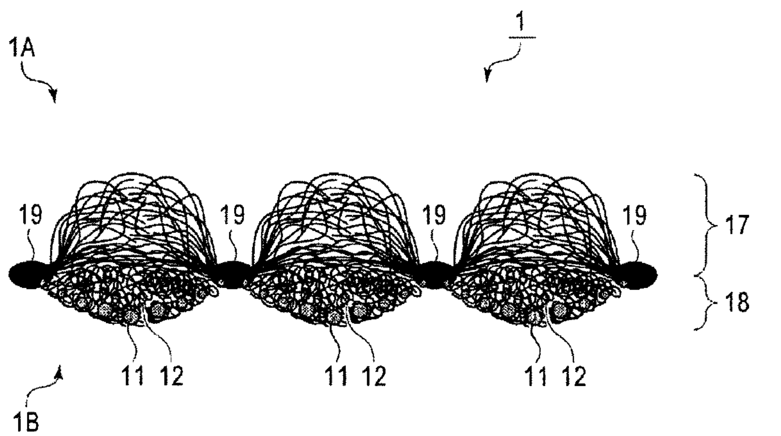
1
1A
19
19
19
19
17
18
11 12
11 12
11 12
1B

{FIG. 5}

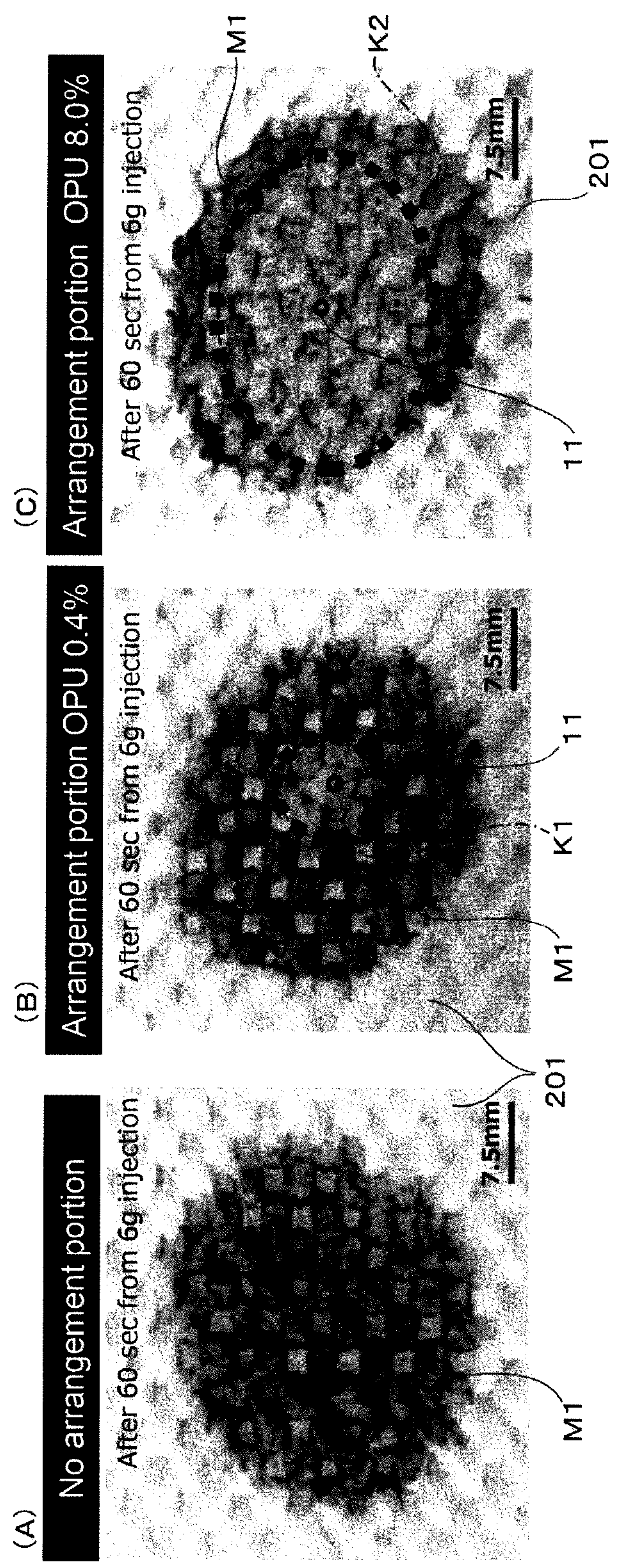
(A)
No arrangement portion
After 60 sec from 6g injection
7.5mm
M1
201
(B)
Arrangement portion OPU 0.4%
After 60 sec from 6g injection
7.5mm
M1
K1
11
(C)
Arrangement portion OPU 8.0%
After 60 sec from 6g injection
7.5mm
M1
K2
11
201

{FIG. 6}

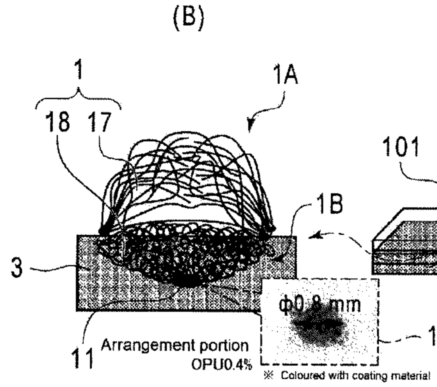


{FIG. 7}

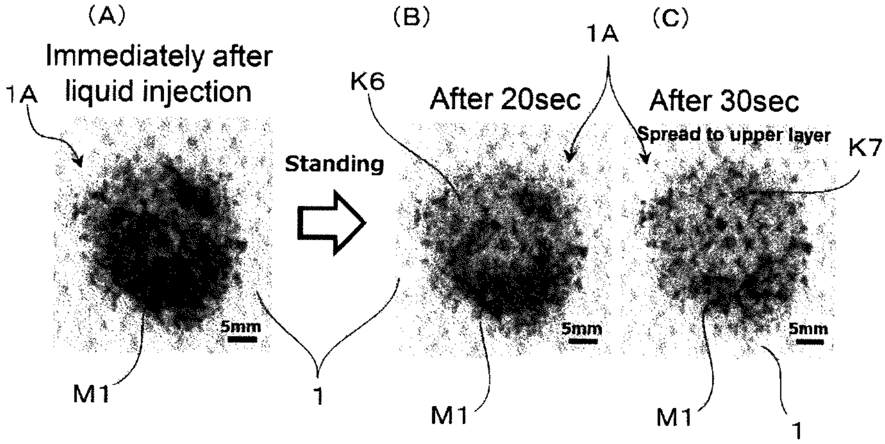


{FIG. 8}

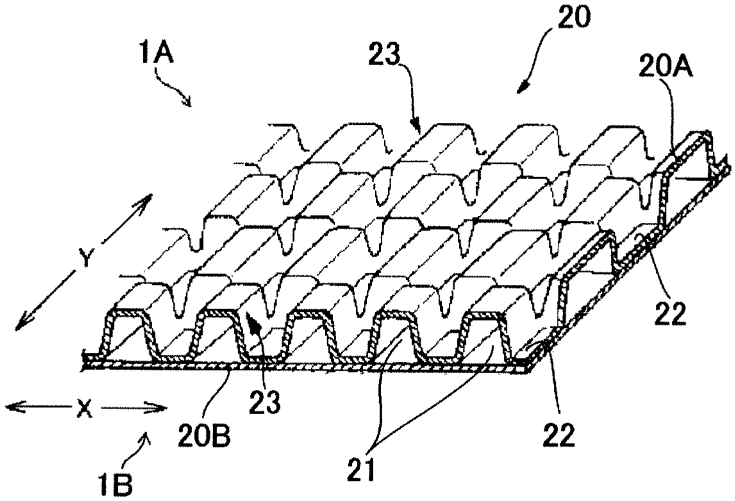

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016117981 A
- WO 2016098796 A
- JP 2015110846 A
- JP 2002161474 A
- JP 8246395 A
- JP 2004275225 A
- JP 2015186543 A
- JP 2004174234 A
- JP 2017119148 A


### Non-patent literature cited in the description


- Merries Sarasara Air Through M size, tape type. Kao Corporation, 2017